(19) Europäisches Patentamt
European Patent Office
Office européen des brevets

(11) Veröffentlichungsnummer: **0 463 444 B1**

# (12) EUROPÄISCHE PATENTSCHRIFT

(45) Veröffentlichungstag der Patentschrift: **30.08.95**

(21) Anmeldenummer: **91109440.7**

(22) Anmeldetag: **08.06.91**

(51) Int. Cl.6: **C07D 498/04**, C07D 513/04, A01N 43/76, A01N 43/78, A01N 43/80, //(C07D498/04, 261:00,207:00),(C07D498/04, 263:00,207:00),(C07D513/04, 275:00,207:00),(C07D513/04, 277:00,207:00)

## (54) Dicarbonsäureimide als Herbizide.

(30) Priorität: **23.06.90 DE 4020072**
**16.07.90 DE 4022566**

(43) Veröffentlichungstag der Anmeldung:
**02.01.92 Patentblatt 92/01**

(45) Bekanntmachung des Hinweises auf die Patenterteilung:
**30.08.95 Patentblatt 95/35**

(84) Benannte Vertragsstaaten:
**AT BE CH DE ES FR GB IT LI NL**

(56) Entgegenhaltungen:
**EP-A- 0 152 759**
**EP-A- 0 337 263**

(73) Patentinhaber: **BASF Aktiengesellschaft**
**Carl-Bosch-Strasse 38**
**D-67063 Ludwigshafen (DE)**

(72) Erfinder: **Maywald, Volker, Dr.**
**Berner Weg 24**
**W-6700 Ludwigshafen (DE)**
Erfinder: **Ditrich, Klaus, Dr.**
**Paray-le-Monial-Strasse 12**
**W-6702 Bad Duerkheim (DE)**
Erfinder: **Kuekenhoehner, Thomas, Dr.**
**Seidelstrasse 2**
**W-6710 Frankenthal (DE)**
Erfinder: **Hamprecht, Gerhard, Dr.**
**Rote-Turm-Strasse 28**
**W-6940 Weinheim (DE)**
Erfinder: **Freund, Wolfgang, Dr.**
**Johann-Gottlieb-Fichte-Strasse 71**
**W-6730 Neustadt (DE)**
Erfinder: **Westphalen, Karl-Otto, Dr.**
**Mausbergweg 58**
**W-6720 Speyer (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

Erfinder: **Gerber, Matthias, Dr.**
**Ritterstrasse 3**
**W-6704 Mutterstadt (DE)**
Erfinder: **Walter, Helmut, Dr.**
**Gruenstadter Strasse 82**
**W-6719 Obrigheim 3 (DE)**

## Beschreibung

Die vorliegende Erfindung betrifft Dicarbonsäureimide der Formeln Ia, Ib oder Ic

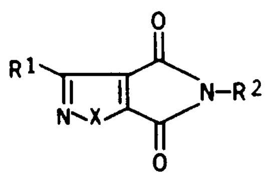

Ia

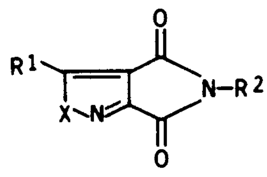

Ib

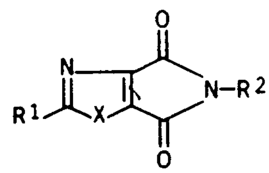

Ic

in der die Substituenten folgende Bedeutung haben:

X

Sauerstoff oder Schwefel;

$R^1$

Wasserstoff; Halogen; Cyano;

$C_1$-$C_6$-Alkyl, welches ein bis fünf Halogenatome und/oder einen oder zwei der folgenden Reste tragen kann: $C_3$-$C_6$-Cycloalkyl, $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Alkylsulfonyl, $C_1$-$C_4$-Halogenalkylsulfonyl oder Cyano;

$C_3$-$C_8$-Cycloalkyl, welches ein bis drei der folgenden Reste tragen kann: $C_1$-$C_4$-Alkyl oder Halogen;

$C_2$-$C_6$-Alkenyl, welches ein bis drei der folgenden Reste tragen kann: Halogen, $C_1$-$C_3$-Alkoxy und/oder ein Phenyl, das seinerseits eine bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Cyano oder Nitro;

$C_2$-$C_6$-Alkinyl, welches ein bis drei der folgenden Reste tragen kann: Halogen oder $C_1$-$C_3$-Alkoxy und/oder ein Phenyl, das seinerseits eine bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Cyano oder Nitro;

$C_1$-$C_4$-Alkoxy; $C_2$-$C_6$-Alkenyloxy; $C_2$-$C_6$-Alkinyloxy; $C_1$-$C_4$-Alkylthio; $C_1$-$C_4$-Halogenalkoxy; $C_1$-$C_4$-Halogenalkylthio; $C_1$-$C_4$-Alkylsulfonyl; $C_1$-$C_4$-Halogenalkylsulfonyl;

Phenoxy oder Phenylthio, welches ein bis drei der folgenden Reste tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Cyano oder Nitro;

ein 5- bis 6-gliedriger gesättigter oder aromatischer heterocyclischer Rest, enthaltend ein oder zwei Heteroatome, ausgewählt aus der Gruppe Sauerstoff, Schwefel und Stickstoff, wobei der Ring ein oder zwei der folgenden Reste tragen kann: $C_1$-$C_3$-Alkyl, Halogen, $C_1$-$C_3$-Alkoxy oder $C_1$-$C_3$-Alkoxycarbonyl;

ein kondensierter aromatischer Rest, ausgewählt aus der Gruppe Benzofuran-2-yl, Benzofuran-3-yl, Isobenzofuran-2-yl, Benzothiophen-2-yl, Benzothiophen-3-yl, Isobenzothiophen-2-yl, Indol-2-yl, Indol-3-yl, 1,2-Benzisoxal-3-yl, Benzoxazol-2-yl, 1,2-Benzisothiazol-3-yl, Benzothiazol-2-yl, Indazol-3-yl, (1H)-Benzimidazhol-2-yl, Chinolin-3-yl, Chinolin-5-yl, Chinolin-6-yl, Chinolin-8-yl, Isochinolin-1-yl und Isochinolin-5-yl, wobei dieser Rest ein oder zwei der folgenden Reste tragen kann: Alkyl wie oben genannt, insbesondere Methyl; Halogen wie oben genannt, insbesondere Fluor und Chlor; Alkoxy wie oben genannt, insbesondere Methoxy und Ethoxy oder Alkoxycarbonyl wie Methoxycarbonyl und Ethoxycarbonyl, insbesondere Methoxycarbonyl;

Phenyl oder Phenyl-$C_1$-$C_4$-alkyl, wobei der Phenylkern jeweils unsubstituiert oder durch ein bis drei der folgenden Gruppen substituiert ist: $C_1$-$C_6$-Alkyl, $C_1$-$C_6$-Halogenalkyl, $C_1$-$C_6$-Alkoxy, $C_1$-$C_6$-Halogenalkoxy, $C_1$-$C_6$-Alkylthio, $C_1$-$C_6$-Halogenalkylthio, Halogen, Nitro und Cyano;

$R^2$

Wasserstoff; Hydroxyl; $C_1$-$C_4$-Alkoxy;

$C_1$-$C_6$-Alkyl, das eine bis drei der folgenden Gruppen tragen kann: Cyano, $C_1$-$C_4$-Alkoxy-$C_1$-$C_4$-alkoxy, $C_1$-$C_4$-Alkoxy, $C_1$-$C_4$-Halogenalkoxy, $C_1$-$C_4$-Alkylthio, $C_1$-$C_4$-Halogenalkylthio, Di-$C_1$-$C_4$-alkylamino, Halogen, $C_3$-$C_8$-Cycloalkyl oder Phenyl, wobei der Phenylring seinerseits ein bis drei der folgenden Reste tragen kann: Halogen, Cyano, Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder $C_1$-$C_4$-Halogenalkylthio;

$C_3$-$C_8$-Cycloalkyl, das eine bis drei der folgenden Gruppen tragen kann: $C_1$-$C_6$-Alkyl, $C_1$-$C_6$-Halogenalkyl, $C_1$-$C_4$-Alkoxy, $C_1$-$C_4$-Halogenalkoxy, Halogen, Nitro oder Cyano;

$C_3$-$C_6$-Alkenyl, das ein- bis dreimal durch Halogen und/oder einmal durch Phenyl substituiert sein kann, wobei der Phenylrest seinerseits eine bis drei der folgenden Gruppen tragen kann: $C_1$-$C_4$-Alkyl, $C_1$-$C_4$-

Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Cyano oder Nitro;

$C_3$-$C_6$-Alkinyl, das ein- bis dreimal durch Halogen und/oder einmal durch Phenyl substituiert sein kann, wobei der Phenylrest seinerseits eine bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Cyano oder Nitro;

Di-$C_1$-$C_4$-alkylamino;

ein 5- bis 6-gliedriger heterocyclischer gesättigter oder aromatischer Rest mit einem oder zwei Heteroatomen, ausgewählt aus der Gruppe Sauerstoff, Schwefel oder Stickstoff, der ein- bis dreimal durch $C_1$-$C_4$-Alkyl oder Halogen substituiert sein kann;

ein kondensierter aromatischer Rest, ausgewählt aus der Gruppe Benzofuran-2-yl, Benzofuran-3-yl, Isobenzofuran-2-yl, Benzothiophen-2-yl, Benzothiophen-3-yl, Isobenzothiophen-2-yl, Indol-2-yl, Indol-3-yl, 1,2-Benzisoxal-3-yl, Benzoxazol-2-yl, 1,2-Benzisothiazol-3-yl, Benzothiazol-2-yl, Indazol-3-yl, (1H)-Benzimidazhol-2-yl, Chinolin-3-yl, Chinolin-5-yl, Chinolin-6-yl, Chinolin-8-yl, Isochinolin-1-yl und Isochinolin-5-yl, wobei dieser Rest ein- bis dreimal durch $C_1$-$C_4$-Alkyl oder Halogen substituiert sein kann;

Phenyl, das eine bis vier der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Nitro, Cyano, Formyl, Phenyl, $C_1$-$C_4$-Alkanoyl, $C_1$-$C_4$-Halogenalkanoyl oder $C_1$-$C_4$-Alkoxycarbonyl;

Naphthyl, das ein- bis dreimal durch $C_1$-$C_4$-Alkyl oder Halogen substituiert sein kann;

sowie pflanzenverträgliche Salze der Dicarbonsäureimide, ausgenommen 3-Methylisoxazol-4,5-dicarboximid und Thiazol-4,5-dicarbonsäureimide, in denen $R^2$ für Phenyl steht, wenn $R^1$ Methyl oder 2-Thiazolyl bedeutet.

Weiterhin betrifft die Erfindung Verfahren zur Herstellung dieser Verbindungen sowie herbizide Mittel, welche mindestens eine Verbindung Ia, Ib oder Ic enthalten, in der die Substituenten die vorstehend gegebene Bedeutung haben, einschließlich 3-Methylisoxazol-4,5-dicarboximid und Thiazol-4,5-dicarbonsäureimide, in denen $R^2$ für Phenyl steht, wenn $R^1$ Methyl oder 2-Thiazolyl bedeutet.

Aus Arch. Pharm. 320, 1281-83 (1987) sind Isoxazoldicarbonsäureimide mit einer Ethoxycarbonylmethylsubstitution in 3-Stellung bekannt. Ferner ist in J. Chem. Soc. Perkin Trans 1, 2391-4 (1982) das 3-Methyl-isoxazol-4,5-dicarbonsäureimid beschrieben. Synthesis 1988, 449-52 lehrt 5-Aminosubstituierte Isothiazoldicarbonsäureimide als Zwischenprodukte zur Herstellung von Farbstoffen.

Speziell substituierte Thiazol-4,5-dicarbonsäureimide sind bekannt aus J. Het. Chem. 26 (1989) 885; Synthesis 1988, 449; J. Het. Chem. 25 (1988) 901; J. Chem. Soc. Perkin Trans. 1, 1981, 2692). Dem zitierten Stand der Technik sind keine Hinweise auf herbizide Eigenschaften der Dicarbonsäureimide zu entnehmen.

Aufgabe der vorliegenden Erfindung war es, neue herbizid wirksame Verbindungen zur Verfügung zu stellen.

Demgemäß wurde gefunden, daß die eingangs definierten Dicarbonsäureimide Ia, Ib und Ic herbizide Eigenschaften besitzen.

Die erfindungsgemäßen Dicarbonsäureimide I sind auf verschiedenen Wegen herstellbar; man erhält sie beispielsweise nach den folgenden Verfahren:

Weg A

Durch Wasserentzug mit wasserabspaltenden Mitteln, beispielsweise Acetanhydrid, anorganischen Säurehalogeniden wie Thionylchlorid, Phosphor(III)- oder Phosphor(V)-halogeniden wie Phosphortrichlorid oder Phosphorpentachlorid, Phosgen, p-Toluolsulfonsäurechlorid oder Propan-phosphonsäureanhydrid, werden die Verbindungen IIa bis IIf in die Dicarbonsäureimide der Formel I umgewandelt.

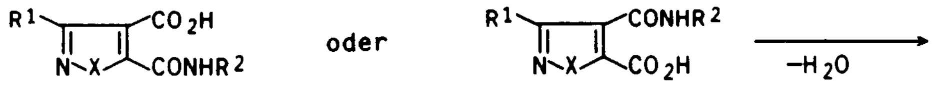


IIa oder IIb → Ia

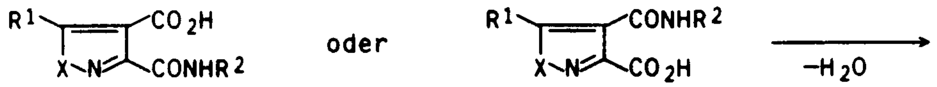


IIc oder IId → Ib

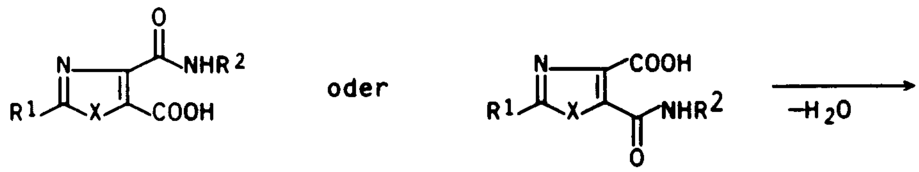


IIe oder IIf → Ic

Die Reaktion wird zweckmäßigerweise so durchgeführt, daß man die Dicarbonsäuremonoamide in einem inerten organischen Lösungsmittel vorlegt und etwa molare Mengen eines wasserabspaltenden Mittels, gegebenenfalls ebenfalls gelöst in einem inerten Lösungsmittel, zutropft. Das Gemisch kann wie üblich aufgearbeitet werden, beispielsweise durch Hydrolyse mit Wasser und Absaugen oder Extraktion des Produktes mit einem organischen Lösungsmittel und Einengen des organischen Lösungsmittels.

Als Lösungsmittel für diese Umsetzungen verwendet man zweckmäßigerweise Halogenkohlenwasserstoffe, z. B. Tetrachlorethan, Methylenchlorid, Chloroform, Dichlorethan, Chlorbenzol und 1,2-Dichlorbenzol; Ether, z.B. Diethylether, Methyl-tert.-butylether, Dimethoxiethan, Diethylenglykoldiethylether, Tetrahydrofuran und Dioxan; dipolare aprotische Lösungsmittel, z. B. Acetonitril, Dimethylformamid, Dimethylacetamid, Dimethylsulfoxid, N-Methylpyrrolidon, 1,3-Dimethyltetra-hydro-2(1H)-pyrimidinon und 1,3-Dimethylimidazolin-2-on; Aromaten, z. B. Benzol, Toluol, Xylol, Pyridin und Chinolin; Ketone, z. B. Aceton, Methylethylketon oder entsprechende Gemische.

Die Dehydratisierung kann bei Temperaturen von -10 °C bis zur Rückflußtemperatur des jeweiligen Lösungsmittels, vorzugsweise bei 0 bis 150 °C, durchgeführt werden.

Die molaren Verhältnisse, in denen die benötigten Ausgangsverbindungen miteinander umgesetzt werden, betragen im allgemeinen 0,9:1 bis 5:1 für das Verhältnis von wasserabspaltendem Mittel zu Säureamid.

Die Konzentration der Edukte im Lösungsmittel(gemisch) liegt im allgemeinen bei 0,1 bis 5 mol/l, bevorzugt 0,2 bis 2 mol/l.

Die Herstellung der Dicarbonsäuremonoamide IIa, die für dieses Verfahren als Ausgangsstoffe benötigt werden, ist in der DE-A-38 12 225 beschrieben.

Die Dicarbonsäuremonoamide IIc können wie in der DE-A 39 31 627 beschrieben erhalten werden. Ausgangsstoffe sind Isoxazol- und Isothiazol-3-carbonsäurehalogenide VII, die mit einem Amin VIII umgesetzt werden. Als Carbonsäurehalogenide VII sind die Chloride bevorzugt. Dabei geht man zweckmäßigerweise so vor, daß man das Carbonsäurehalogenid in einem inerten organischen Lösungsmittel wie Dichlormethan, oder einem Ether wie Diethylether oder Methyl-tert.butylether mit einem Amin VIII, ebenfalls gelöst in einem organischen Lösungsmittel, zur Reaktion bringt. Dabei setzt man das Amin VIII zweckmäßigerweise in 2- bis 5-fach molarer Menge, vorzugsweise 2- bis 3-fach molarer Menge, ein, um den entstehenden Halogenwasserstoff zu binden. Man kann auch in Gegenwart einer Hilfsbase wie z.B. einem tertiären Amin (Triethylamin) arbeiten. In diesem Fall genügen 1 bis 1,5 Moläquivalente Amin VIII. Die Reaktionstemperatur kann zwischen 0 und 50 °C, vorzugsweise zwischen 0 und 20 °C betragen. Die Reaktion ist im allgemeinen nach 1 bis 12 Stunden beendet. Das Gemisch kann wie üblich aufgearbeitet werden, beispielsweis durch Hydrolyse mit Wasser und Extraktion des Produktes IX mit einem organischen Lösungsmittel und Einengen des organischen Lösungsmittels.

Aus den Isoxazol- bzw. Isothiazolamiden IX erhält man die 3-Aminocarbonyl-isoxazol-4-carbonsäuren bzw. 3-Aminocarbonyl-isothiazol-4-carbonsäuren der Formel IIc, durch Umsetzung mit Alkyllithium wie n-Butyllithium, sec.-Butyllithium und Methyllithium, vorzugsweise unter Zugabe eines unter den Reaktionsbedingungen inerten Lösungsmittels, wie Diethylether oder Tetrahydrofuran. In der Regel wird diese Umsetzung unter einer Inertgasatmosphäre, z.B. Stickstoffatmosphäre, bei Temperaturen zwischen -70 und -80 °C vorgenommen. Die Alkyllithiumverbindung wird bei diesem Verfahren im allgemeinen in 2- 3-fach molarer Menge bezogen auf eingesetztes Amid der Formel IX verwendet. Nach vollständiger Umsetzung wird das Gemisch mit Kohlendioxid behandelt, vorzugsweise in einem inerten Lösungsmittel wie Diethylether oder z.B. Tetrahydrofuran, wobei man die gewünschten Produkte der Formel IIc erhält.

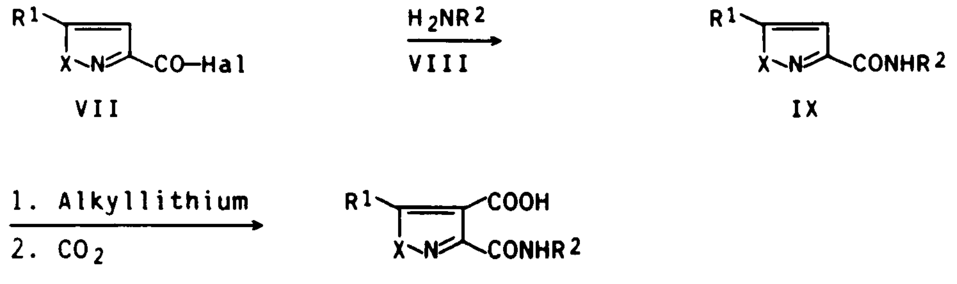

VII IX

IIc

Hal = Halogen wie Cl, Br

Die für dieses Verfahren als Ausgangsmaterial benötigten Isoxazol-und Isothiazol-3-carbonsäurehalogenide VII sind literaturbekannt oder können aus den entsprechenden Carbonsäuren auf übliche Art und Weise hergestellt werden.

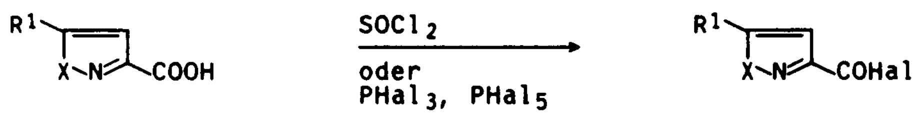


VII

Die für dieses Verfahren benötigten Carbonsäuren sind literaturbekannt (Beilstein, Hauptwerk sowie 1.-5. Ergänzungswerk, Band 27; R.W. Wiley, The Chemistry of Heterocyclic Compounds, Five- and Six-Membered Compounds with Nitrogen and Oxygen, Interscience Publishers, New York, London (1962)) oder können nach allgemein literaturbekannten Methoden, z.B. durch Oxidation aus den entsprechenden Alkoholen oder Aldehyden oder durch Hydrolyse aus den entsprechenden Nitrilen hergestellt werden.

Die als Ausgangsmaterialien verwendeten Dicarbonsäuremonoamide IIe und IIf können nach verschiedenen Verfahren erhalten werden:

a) durch Verseifung der entsprechenden Alkylester, z.B. Methyl- oder Ethylester gemäß den unter Reaktionsschritt A angegebenen Bedingungen.

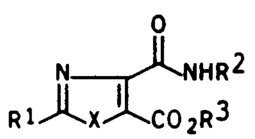

oder

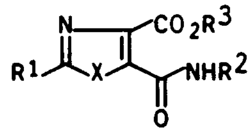

($R^3$ = $C_1$-$C_6$-Alkyl)

Man erhält diese Alkylester dadurch, daß man einen Diester der Formel III in an sich bekannter Weise mit einem Äquivalent einer wäßrigen Base zum Monoester IVa bzw. IVb hydrolysiert und IVa und IVb danach getrennt oder im Gemisch zunächst in das Halogenid oder eine andere aktivierte Form der Carbonsäure überführt und diese Derivate anschließend mit einem Amin VIII amidiert.

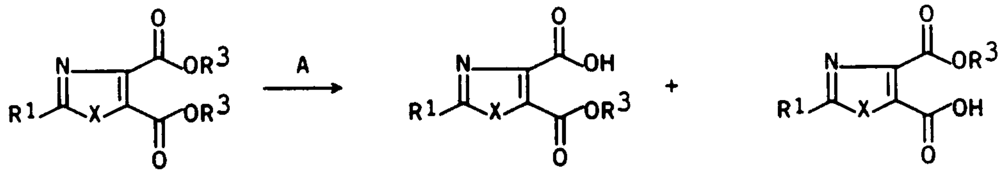


III IVa IVb

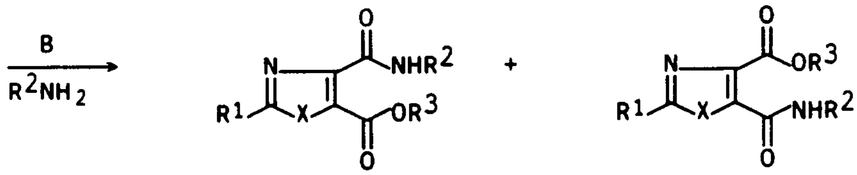


VIII

Die einzelnen Reaktionsschritte A und B dieser Synthesesequenz können wie folgt durchgeführt werden:

Reaktionsschritt A:

Die partielle Verseifung des Diesters III zum Monoester IVa und IVb wird üblicherweise bei Temperaturen von -20 bis 60 °C, vorzugsweise -10 bis 30 °C, in einem inerten, mit Wasser mischbaren organischen Lösungmittel in Gegenwart von 1,0 bis 1,2 mol-Äquivalenten einer Base durchgeführt.

Als Basen eignen sich insbesondere Hydroxyde von Alkalimetall-Kationen wie Natrium- oder Kaliumhydroxid. Die Base wird im allgemeinen als 5 bis 20 %ige wäßrige Lösung zugesetzt.

Bevorzugte Lösungsmittel für diese Umsetzung sind beispielsweise Dioxan oder der der Esterkomponente in der Formel III entsprechende Alkohol.

Zur Aufarbeitung wird das Reaktionsgemisch üblicherweise angesäuert, wobei sich das gewünschte Produkt als Feststoff oder als Öl abscheidet. Die Isolierung erfolgt in üblicher Weise durch Filtration bzw. Extraktion.

Das Gemisch der beiden Isomeren IVa und IVb kann durch fraktionierte Kristallisation oder auf chromatographischem Wege getrennt werden, oder es kann ohne Trennung weiter umgesetzt werden.

Reaktionsschritt B:

Die Monoester IVa und IVb werden zunächst in an sich bekannter Weise in das Halogenid oder eine andere aktivierte Form der Carbonsäurefunktion überführt und diese Derivate anschließend mit einem Amin VIII amidiert.

Aktivierte Formen der Carbonsäure sind neben Halogeniden wie insbesondere den Chloriden und den Bromiden beispielsweise auch Imidazolide. Im allgemeinen werden die Halogenide bevorzugt.

Man erhält sie durch Umsetzung der Carbonsäuren IVa und IVb mit einem Halogenierungsmittel wie Thionylchlorid, Thionylbromid, Phosphoroxychlorid bzw. -bromid, Phosphortri- und pentachlorid bzw. -bromid, Phosgen sowie elementarem Chlor und Brom.

Das Halogenierungsmittel wird in 1 bis 5, vorzugsweise 1 bis 2 mol-Äquivalenten, eingesetzt.

Die Umsetzung verläuft bei Temperaturen von 0 °C bis zum Siedepunkt des Halogenierungsmittels bzw. sofern man in Gegenwart eines inerten organischen Lösungsmittels arbeitet, auch dessen Siedepunkt, vorzugsweise 20 bis 120 °C.

Als Lösungsmittel eignen sich beispielsweise Kohlenwasserstoffe und Halogenkohlenwasserstoffe wie Tetrachlorethan, Methylenchlorid, Chloroform, Dichlorethan, Chlorbenzol, 1,2-Dichlorbenzol, Benzol, Toluol und Xylol.

Üblicherweise werden die aktivierten Carbonsäurederivate isoliert, beispielsweise durch Abdestillieren des Halogenierungsmittels und sofern vorhanden des Lösungsmittels und erst anschließend mit den Aminen umgesetzt.

In diesem Fall wird die Amidierung bei Temperaturen -20 bis 100 °C, vorzugsweise -10 bis 20 °C in einem inerten aprotisch polaren organischen Lösungsmittel durchgeführt.

Für diese Umsetzung eignen sich insbesondere Halogenkohlenwasserstoffe wie Dichlormethan und Ether wie Diethylether und tert.-Butylmethylether als Lösungsmittel.

Da bei der Amidierung von Säurehalogeniden Halogenwasserstoff gebildet wird, empfiehlt es sich, das Amin VIII in 2 bis 5 mol-Äquivalenten

überschuß, vorzugsweise 2 bis 3 mol-Äquivalenten zuzusetzen. Sofern das Amin in etwa äquimolaren Mengen (1 bis 1,2 mol-Äquivalenten) eingesetzt wird, sollte zum Binden des Halogenwasserstoffs eine Base, insbesondere ein tertiäres Amin wie Triethylamin oder Pyridin zugegeben werden.

Sofern man von einem Gemisch der Monoester IVa und IVb ausgeht, erhält man bei der Umsetzung ein Gemisch aus den isomeren Carbonsäureamiden. Dieses Gemisch kann auf herkömmliche Weise, beispielsweise durch fraktionierte Kristallisation oder Chromatographie in die Einzelkomponenten aufgetrennt werden.

Die für diese Synthesesequenz benötigten Edukte III sind bekannt (Bull. Soc. Chim. Fr. 1974, 2079) oder nach bekannten Methoden (Bull. Soc. Chim. Fr. 1969, 1762; J. Chem. Soc., 1953, 93) zugänglich.

b) Verfahren zur Herstellung von Thiazolcarbonsäureimiden IIe und IIf.

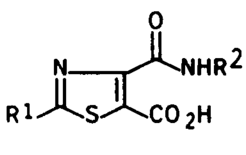

IIe

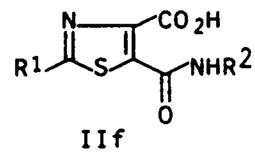

IIf

Man erhält diese Thiazolcarbonsäureamide IIe und IIf besonders vorteilhaft, indem man ein Dicarbonsäureanhydrid der Formel V in an sich bekannter Weise mit einem Amin der Formel VIII zu den Isomeren IIe und IIf umsetzt und anschließend das Gemisch in die Isomeren auftrennt.

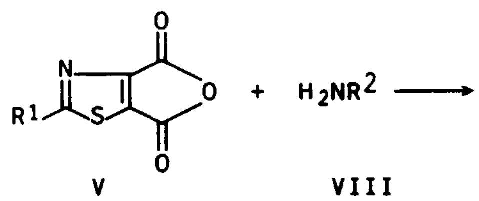

V VIII

Die Umsetzung wird üblicherweise bei Temperaturen von -10 bis 150 °C, vorzugsweise 20 bis 120 °C in einem inerten aprotisch polaren organischen Lösungsmittel durchgeführt.

Insbesondere kommen als Lösungsmittel Halogenkohlenwasserstoffe, z. B. Tetrachlorethan, Methylenchlorid, Chloroform, Dichlorethan, Chlorbenzol und 1,2-Dichlorbenzol; Ether z. B. Diethylether, Methyl-tert.-butylether, Dimethoxyethan, Diethylenglykoldimethylether, Tetrahydrofuran und Dioxan; dipolare aprotische Lösungsmittel, z. B. Acetonitril, Dimethylformamid, Dimethylacetamid, Dimethylsulfoxid, N-Methylpyrrolidon, 1,3-Dimethyltetrahydro-2(1H)-pyrimidinon und 1,3-Dimethylimidazolidin-2-on; Aromaten, z. B. Benzol, Toluol, Xylol, Pyridin und Chinolin; Ketone, z. B. Aceton, Methylethylketon oder entsprechende Gemische zur Anwendung.

Das Amin VIII wird im allgemeinen in äquimolaren Mengen oder im Überschuß, vorzugsweise in Mengen von 1,0 bis 5,0 mol-Äquivalenten, bezogen auf V eingesetzt.

Die für dieses Verfahren benötigten Dicarbonsäureanhydride sind bekannt oder können nach bekannten Methoden hergestellt werden (Bull. Soc. Chim. Fr. 1969, 1762; CS-A-195 369; CS-A-195 370).

c) Verfahren zur Herstellung der Verbindungen IIe und IIf in denen $R^1$ nicht Halogen oder Cyano bedeutet:

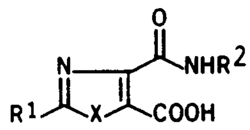

IIe

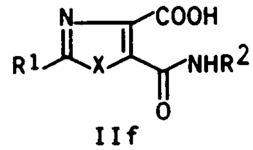

IIf

($R^1$ ≠ Halogen, Cyano)

Man erhält diese isomeren Oxazol- bzw. Thiazolcarbonsäureamide, indem man eine entsprechende Carbonsäure gemäß den unter B geschilderten Bedingungen aktiviert und amidiert und die so erhaltenen Amide VIa und VIb anschließend in an sich bekannter Weise in Gegenwart eines Carboxylierungsreagens umsetzt.

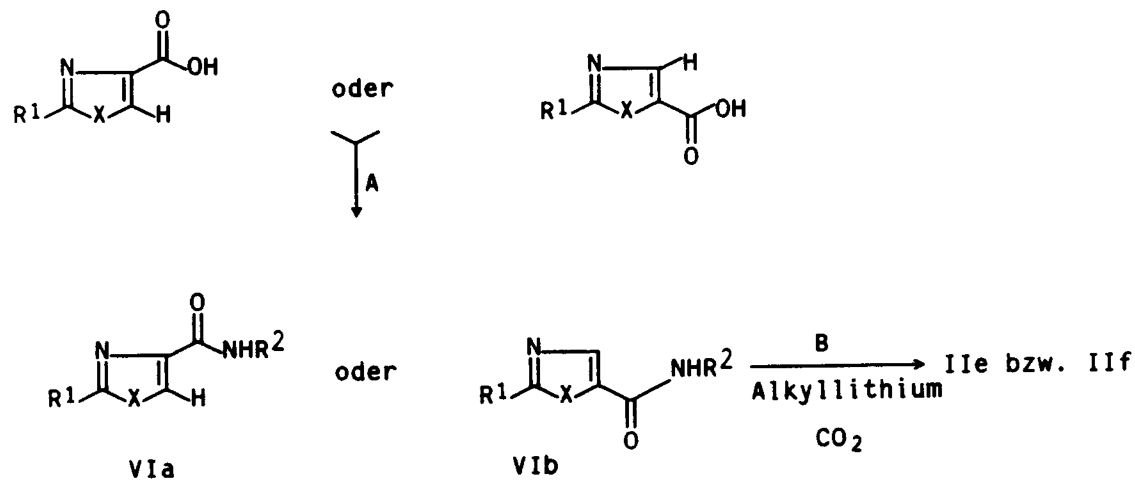


VIa VIb

Der Reaktionsschritt A dieser Synthesesequenz wird im allgemeinen und im besonderen entsprechend den im Verfahren a) unter Punkt B beschriebenen Bedingungen durchgeführt.

Reaktionsschritt B:

Die Carboxylierung der Oxazol- bzw. Thiazolcarbonsäureamide VIa bzw. VIb erfolgt in der Regel bei Temperaturen von 0 bis -100 °C, vorzugsweise -50 bis -80 °C in einem aprotisch polaren inerten organischen Lösungsmittel in Gegenwart einer Base unter Ausschluß von Feuchtigkeit.

Bevorzugtes Carboxylierungsreagens ist gasförmiges oder festes Kohlendioxid.

Geeignete Lösungsmittel sind insbesondere Ether, z. B. Diethylether, Methyl-tert.-butylether, Dimethoxyethan, Diethylenglykoldimethylether, Tetrahydrofuran und Dioxan.

Als Basen finden bevorzugt Organometallverbindungen wie Methyllithium, n-Butyllithium, s-Butyllithium, t-Butyllithium oder Phenyllithium Verwendung.

Die Umsetzung wird üblicherweise so durchgeführt, daß zunächst eine Lösung des Oxazol- bzw. Thiazolcarbonsäureamids VIa bzw. VIb mit bis zu 3 Mol-Äquivalenten der gelösten Base versetzt wird, wobei ein am Heterocyclus metalliertes Derivat entsteht, welches bei der anschließenden Zugabe des elektrophilen Carboxylierungsreagenzes zum gewünschten Produkt IIe bzw. IIf abreagiert.

Die für das vorstehende Verfahren benötigten Carbonsäuren sind literaturbekannt (Beilstein Band 27, 1.-5. Ergänzungswerke) oder sie können nach bekannten Methoden, beispielsweise durch Oxidation der entsprechenden Alkohole oder Aldehyde oder durch Hydrolyse der entsprechenden Nitrile hergestellt werden (J. V. Metzger in "The Chemistry of Heterocyclic Compounds, Vol. 34, Part 1, Thiazol and its Derivatives", Arnold Weissberger and E. D. Ward, C. Taylor (Editor), John Wiley and Sons, S. 519 ff, I. J. Turchi in "The Chemistry of Heterocyclic Compounds, Vol. 45, Oxazoles", Arnold Weissberger and E. D. Ward, C. Taylor (Editor), John Wiley and Sons).

d) Verbindungen der Formel IIf können auch gewonnen werden, indem man entsprechende Dicarbonsäureester in an sich bekannter Weise mit Aminen umsetzt und die resultierenden Amide verseift:

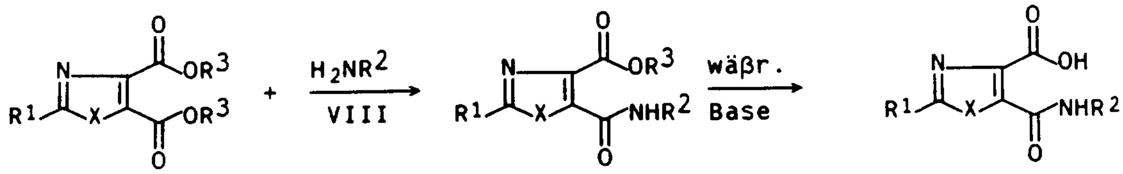


IIf

($R^3$ = $C_1$-$C_6$-Alkyl)

Zweckmäßigerweise geht man dabei so vor, daß man den Diester in einem inerten organischen Lösungsmittel löst und mit einem Amin VIII umsetzt.

Als Lösungsmittel verwendet man für diese Umsetzungen Ether, z.B. Diethylether, Methyl-tert.-butylether, Dimethoxyethan, Diethylenglykoldimethylether, Tetrahydroforan und Dioxan; Aromaten, z. B. Benzol, Toluol, Xylol oder Mesitylen; Alkohole, z. B. Methanol, Ethanol, iso-Propanol und tert.-Butanol oder entsprechende Gemische.

Die Umsetzung kann bei Temperaturen von -100 °C bis zur Rückflußtemperatur des jeweiligen Lösungsmittels bzw. -gemisches, vorzugsweise bei -60 °C bis 150 °C, durchgeführt werden.

Das molare Verhältnis, in dem Diester und Amin eingesetzt werden, beträgt im allgemeinen 1:1 bis 1:2, vorzugsweise 1:1 bis 1:1,2.

Die Konzentration der Edukte im Lösungsmittel beträgt im allgemeinen 0,1 bis 5 mol/l, bevorzugt 0,2 bis 2,0 mol/l.

Besonders bevorzugt arbeitet man in Alkoholen wie Ethanol in Gegenwart von einem Äquivalent Amin bei 50 bis 100 °C. Die für die Umsetzung benötigten Diester sind literaturbekannt oder können in Anlehnung an beschriebene Methoden hergestellt werden (Bull. Soc. Chim. Fr., 1969, 1762; J. Chem. Soc., 1953, 93).

Neben den vorstehend geschilderten Verfahren a-d zur Herstellung der Ausgangsverbindungen IIe und IIf gibt es weitere Synthesemöglichkeiten, die den folgenden Literaturstellen zu entnehmen sind: Beilstein, Hauptwerk sowie 1.-5. Ergänzungswerk, Band 27; R. W. Wiley, The Chemistry of Heterocyclic Compounds, Five- and Six-Membered Compounds with Nitrogen and Oxygen, Interscience Pubishers, New York, London (1962); Heterocyclic Chemistry, Vol. 6, Five-membered Rings with Two or More Oxygen, Sulfur or Nitrogen Atoms, Pergamon Press, 1984; J. March, Advanced Organic Chemistry, Third edition, John Wiley and Sons, 1985; Houben-Weyl, Methoden der organischen Chemie, 4. Auflage, Thieme Verlag, Bände IV, VI, VII, VIII, X; DE-A 39 32 052.

Weg B

Ein weiteres Verfahren zur Synthese von Verbindungen der Formel Ia besteht in der Umsetzung von Hydroxamsäurechloriden X mit Halogenmaleinsäureimiden XI. Dabei geht man zweckmäßigerweise so vor, daß man das Halogenmaleinsäureimid XI in einem inerten organischen Lösungsmittel vorlegt, etwa molare Mengen des Hydroxamsäurechlorids X zugibt und anschließend eine etwa doppelt molare Menge einer Base zutropft. Das Gemisch kann wie üblich aufgearbeitet werden, z.B. durch Hydrolyse mit Wasser und Absaugen oder Extraktion des Produktes mit einem organischen Lösungsmittel und Einengen des organischen Lösungsmittels.

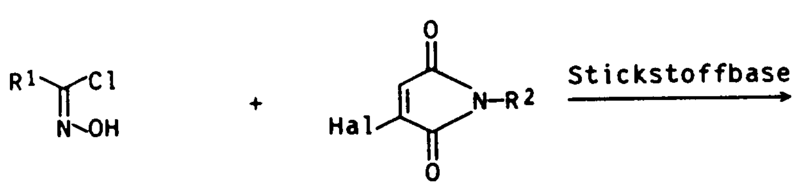


Ia

X XI

Hal = Cl, Br

Als Lösungsmittel für diese Umsetzungen verwendet man zweckmäßigerweise Halogenkohlenwasserstoffe

wie Dichlorethan, Chlorbenzol, 1,2-Dichlorbenzol, Tetrachlorethan, Dichlormethan und Chloroform; Ether wie Diethylether, Methyl-tert.-butylether, Dimethoxyethan, Diethylenglykoldimethylether, Tetrahydrofuran und Dioxan oder Aromaten wie Benzol, Toluol und Xylol oder Gemische dieser Lösungsmittel.

Die Umsetzungen können bei Temperaturen von -10 °C bis 50 °C, vorzugsweise bei 0 bis 30 °C, durchgeführt werden.

Als Basen kommen vorzugsweise Stickstoffbasen wie 2-, 3-, 4-Picolin, N,N-Dimethylanilin, N,N-Dimethylcyclohexylamin, Triethylamin (TEA), Pyridin und 1,8-Diazabicyclo(5.4.0)undec-7-en (DBU) in Betracht.

Die Reaktionspartner werden insbesondere im Molverhältnis 1:1 eingesetzt, die Stickstoffbase wird in der doppelt bis dreifach molaren Menge zugesetzt.

Die Konzentration der Edukte im Lösungsmittelgemisch beträgt im allgemeinen 0,1 bis 5 mol/l, bevorzugt 0,2 bis 2 mol/l.

Weg C:

Ein weiteres Verfahren zur Synthese der Verbindungen Ia besteht in der Oxidation von Isoxazolindicarbonsäureimiden XII, die z.B. hergestellt werden durch Umsetzung von Hydroxamsäurechloriden X mit Maleinimiden XIII. Dabei geht man zweckmäßigerweise so vor, daß man das Maleinimid XIII in einem inerten organischen Lösungsmittel vorlegt, etwa molare Mengen des Hydroxamsäurechlorids X zugibt und anschließend eine etwa molare Menge einer Base zutropft. Das Gemisch kann wie üblich aufgearbeitet werden, z.B. durch Hydrolyse mit Wasser und Absaugen oder Extraktion des Produktes mit einem organischen Lösungsmittel.

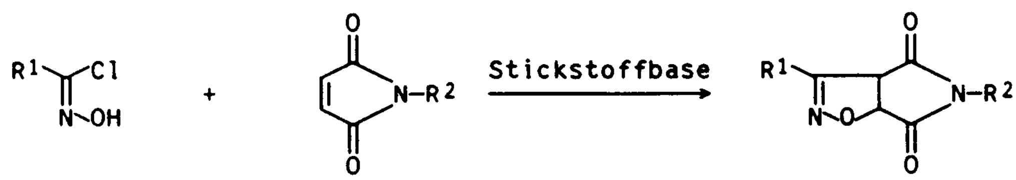


X XIII XII

Als Lösungsmittel für diese Umsetzungen verwendet man zweckmäßigerweise Halogenkohlenwasserstoffe wie Tetrachlorethan, Dichlormethan, Dichlorethan, Chlorbenzol, 1,2-Dichlorbenzol und Chloroform oder Aromaten wie Benzol, Toluol und Xylol oder Gemische dieser Lösungsmittel.

Die Umsetzungen können bei Temperaturen von -10 bis 50 °C, vorzugsweise bei 0 °C bis 30 °C, durchgeführt werden.

Als Basen kommen Stickstoffbasen wie 2-, 3-, 4-Picolin, N,N-Dimethylanilin, N,N-Dimethylcyclohexylamin, Triethylamin (TEA), Pyridin oder 1,8-Diazabicyclo(5.4.0)undec-7-en (DBU) in Betracht.

Die Reaktionspartner werden insbesondere in Molverhältnissen von 1 bis 1,1:1 (Maleinimid:Hydroxamsäurechlorid) eingesetzt, die Stickstoffbase wird in der einfach bis doppelt molaren Menge zugesetzt.

Die Konzentration der Edukte im Lösungsmittelgemisch beträgt im allgemeinen 0,1 bis 5 mol/l, bevorzugt 0,2 bis 2 mol/l.

Die Isoxazoldicarbonsäureimide Ia sind durch Dehydrierung der Isoxazolinimide XII zugänglich. Dabei geht man zweckmäßigerweise so vor, daß man das Isoxazolinimid XII in einem inerten organischen Lösungsmittel vorlegt und mit etwa molaren Mengen Oxidationsmittel versetzt.

Als Lösungsmittel für die Dehydrierung kommen insbesondere aromatische Kohlenwasserstoffe wie Benzol, Toluol oder Xylol in Betracht.

Als Oxidationsmittel verwendet man bevorzugt Natriumhypochlorit, Nitrobenzol oder Chinone wie 2,3,5,6-Tetrachlor-p-benzochinon oder 5,6-Dichlor-2,3-dicyano-p-benzochinon (DDQ).

Die Umsetzungen können bei Temperaturen von 50 °C bis zur Siedetemperatur des verwendeten Lösungsmittels durchgeführt werden.

Die Reaktionspartner können im Molverhältnis 1:1 bis 1:10 (Isoxazolinimid:Oxidationsmittel) eingesetzt werden.

Im Hinblick auf die bestimmungsgemäße Verwendung der Verbindungen I kommen als Substituenten bevorzugt folgende Reste in Betracht:

X

Sauerstoff oder Schwefel;

$R^1$

Halogen wie Fluor, Chlor, Brom und Iod, insbesondere Fluor und Chlor; Cyano;

$C_1$-$C_6$-Alkyl, wie Methyl, Ethyl, n-Propyl, iso-Propyl, n-Butyl, sek.-Butyl, iso-Butyl und tert.-Butyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, insbesondere Methyl, Ethyl, Propyl und iso-Propyl, welches ein bis fünf Halogenatome, insbesondere Fluor- und/oder Chloratome oder einen oder zwei der folgenden Reste tragen kann: $C_3$-$C_8$-Cycloalkyl wie Cyclopropyl, Cyclobutyl, Cyclopentyl und Cyclohexyl, insbesondere Cyclopropyl; $C_1$-$C_4$-Alkoxy wie Methoxy, Ethoxy, n-Propoxy, 2-Methylethoxy, n-Butoxy, 1-Methylpropoxy, 2-Methylpropoxy und 1,1-Dimethylethoxy, insbesondere Methoxy, Ethoxy, 1-Methylethoxy und 1,1-Dimethylethoxy; $C_1$-$C_4$-Halogenalkoxy wie Difluormethoxy, Trifluormethoxy, Chlordifluormethoxy, Dichlorfluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, insbesondere Trifluormethoxy und Pentafluorethoxy; $C_1$-$C_4$-Alkylthio wie Methylthio, Ethylthio, Propylthio, 1-Methylethylthio, Butylthio, 1-Methylpropylthio, 2-Methylpropylthio und 1,1-Dimethylethylthio, insbesondere Methylthio und Ethylthio, $C_1$-$C_4$-Halogenalkylthio wie Difluormethylthio, Trifluormethylthio, Chlordifluormethylthio, 1-Fluorethylthio, 2-Fluorethylthio, 2,2-Difluorethylthio, 2,2,2-Trifluorethylthio, 2-Chlor-2,2-difluorethylthio, 2,2-Dichlor-2-fluorethylthio, 2,2,2-Trichlorethylthio und Pentafluorethylthio, insbesondere Trifluormethylthio und Pentafluorethylthio, $C_1$-$C_4$-Alkylsulfonyl, wie Methylsulfonyl und Ethylsulfonyl, insbesondere Methylsulfonyl; $C_1$-$C_4$-Halogenalkylsulfonyl, wie Trifluormethylsulfonyl und Pentafluorethylsulfonyl, insbesondere Trifluormethylsulfonyl; oder Cyano;

$C_3$-$C_8$-Cycloalkyl wie Cyclopropyl, Cyclobutyl, Cyclopentyl, Cyclohexyl, Cycloheptyl und Cyclooctyl, insbesondere Cyclopropyl, Cyclopentyl und Cyclohexyl, welches ein bis drei der folgenden Reste tragen kann: Alkyl wie vorstehend genannt, insbesondere Methyl oder Halogen wie vorstehend genannt, insbesondere Chlor und Fluor;

$C_2$-$C_8$-Alkenyl wie Ethenyl, 1-Propenyl, 2-Propenyl, 1-Methylethenyl, 1-Butenyl, 2-Butenyl, 3-Butenyl, 1-Methyl-1-propenyl, 1-Methyl-2-propenyl, 2-Methyl-1-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-2-butenyl, 1-Methyl-2-butenyl, 2-Methyl-2-butenyl, 1-Methyl-3-butenyl, 2-Methyl-3-butenyl, 3-Methyl-3-butenyl, 1,1-Dimethyl-2-propenyl, 1,1-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 1,3-Dimethyl-1-butenyl, 2,3-Dimethyl-1-butenyl, 2,3-Dimethyl-2-butenyl, 2,3-Dimethyl-3-butenyl, 3,3-Dimethyl-1-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2,2-propenyl, 1-Ethyl-1-methyl-2-propenyl, 1-Ethyl-2-methyl-1-propenyl und 1-Ethyl-2-methyl-2-propenyl, insbesondere 2-Propenyl, welches ein bis drei der folgenden Reste tragen kann: Halogen wie oben genannt, insbesondere Fluor und Chlor; Alkoxy wie oben genannt, insbesondere Methoxy und Ethoxy, und/oder ein Phenyl, das seinerseits eine bis drei der folgenden Gruppen tragen kann: Alkyl wie vorstehend genannt, insbesondere Methyl, Ethyl und iso-Propyl; Halogenalkyl wie vorstehend genannt, insbesondere Trifluormethyl und Chlordifluormethyl; Alkoxy wie vorstehend genannt, insbesondere Methoxy und Ethoxy; Halogenalkoxy wie vorstehend genannt, insbesondere Trifluormethoxy, Pentafluorethoxy und Trichlormethoxy; Alkylthio wie vorstehend genannt, insbesondere Methylthio und Ethylthio; Halogenalkylthio wie vorstehend genannt, insbesondere Difluormethylthio, Pentafluorethylthio und Trifluormethylthio; Halogen wie vorstehend genannt, insbesondere Fluor und Chlor, Cyano oder Nitro;

$C_2$-$C_6$-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-3-butinyl, 2-Methyl-3-butinyl, 1-Methyl-2-butinyl, 3-Methyl-1-butinyl, 1,1-Dimethyl-2-propinyl, 1-Ethyl2-propinyl, 1-Hexinyl, 2-Hexinyl, 3-Hexinyl, 4-Alkinyl, 4-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-4-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl, insbesondere 2-Propinyl, welches einen bis drei der folgenden Reste tragen kann: Halogen wie oben genannt, insbesondere Iod; Alkoxy wie oben genannt, insbesondere Methoxy und Ethoxy, und/oder ein Phenyl, das seinerseits eine bis drei der

folgenden Gruppen tragen kann: Alkyl wie vorstehend genannt, insbesondere Methyl, Ethyl und iso-Propyl; Halogenalkyl wie vorstehend genannt, insbesondere Trifluormethyl und Chlordifluormethyl; Alkoxy wie vorstehend genannt, insbesondere Methoxy und Ethoxy; Halogenalkoxy wie vorstehend genannt, insbesondere Trifluormethoxy, Pentafluorethoxy und Trichlormethoxy, Alkylthio wie vorstehend genannt, insbesondere Methylthio und Ethylthio; Halogenalkylthio wie vorstehend genannt, insbesondere Difluormethylthio, Pentafluorethylthio und Trifluormethylthio; Halogen wie vorstehend genannt, insbesondere Fluor und Chlor, Cyano oder Nitro;

$C_2$-$C_6$-Alkenyloxy wie Vinyloxy, 2-Propenyloxy, 2-Methyl-2-propenyloxy, (E)-2-Butenyloxy, (Z)-2-Butenyloxy, 1,3-Dimethyl-2-butenyloxy, 2-Pentenyloxy, 2-Hexenyloxy, insbesondere 2-Propenyloxy und (E)-2-Butenyloxy;

$C_3$-$C_6$-Alkinyloxy wie 2-Propinyloxy, 2-Butinyloxy, 1-Ethyl-2-butinyloxy, 1-Methyl-2-propinyloxy, 2-Pentinyloxy, 2-Hexinyloxy, insbesondere 2-Propinyloxy;

$C_1$-$C_4$-Alkylthio wie obenstehend genannt, insbesondere Methylthio und Ethylthio;

$C_1$-$C_4$-Halogenalkylthio wie obenstehend genannt, insbesondere Difluormethylthio, Pentafluorethylthio und Trifluormethylthio;

$C_1$-$C_4$-Alkylsulfonyl wie vorstehend genannt, insbesondere Methylsulfonyl;

$C_1$-$C_4$-Halogenalkylsulfonyl wie vorstehend genannt, insbesondere Trifluormethylsulfonyl;

Phenoxy oder Phenylthio, wobei diese Reste ein bis drei der folgenden Gruppen tragen können: $C_1$-$C_4$-Alkyl wie vorstehend genannt, insbesondere Methyl, Ethyl und iso-Propyl; $C_1$-$C_4$-Halogenalkyl wie vorstehend genannt, insbesondere Trifluormethyl und Chlordifluormethyl; $C_1$-$C_4$-Alkoxy wie vorstehend genannt, insbesondere Methoxy und Ethoxy; $C_1$-$C_4$-Halogenalkoxy wie vorstehend genannt, insbesondere Trifluormethoxy, Pentafluorethoxy und Trichlormethoxy; $C_1$-$C_4$-Alkylthio wie vorstehend genannt, insbesondere Methylthio und Ethylthio; $C_1$-$C_4$-Halogenalkylthio wie vorstehend genannt, insbesondere Difluormethylthio, Pentafluorethylthio und Trifluormethylthio; Halogen wie vorstehend genannt, insbesondere Fluor und Chlor, Cyano oder Nitro;

ein 5- bis 6-gliedriger heterocyclischer Rest enthaltend ein oder zwei Heteroatome, ausgewählt aus der Gruppe Sauerstoff, Schwefel und Stickstoff, wie 2-Tetrahydrofuranyl, 3-Tetrahydrofuranyl, 4-Tetrahydropyranyl, 2-Tetrahydropyranyl, 3-Tetrahydropyranyl, 3-Furanyl, 2-Thienyl, 3-Thienyl, 2-Furanyl, 3-Tetrahydrothienyl, 3-Sulfolanyl, 2-Tetrahydropyranyl, 5-Isoxazolyl, 3-Isoxazolyl, 4-Isoxazolyl, 5-Isothiazolyl, 4-Isothiazolyl, 3-Isothiazolyl, 2-Oxazolyl, 4-Thiazolyl, 4-Oxazolyl, 2-Thiazolyl, 5-Oxazolyl, 5-Thiazolyl, 2-Imidazolyl, 4-Imidazolyl, 5-Imidazolyl, 3-Pyrrolyl, 2-Pyrrolyl, 3-Pyrazolyl, 4-Pyrazolyl, 5-Pyrazolyl, 4-Pyridyl, 3-Pyridyl und 2-Pyridyl, wobei diese Ringe ein oder zwei der folgenden Reste tragen können: Alkyl wie oben genannt, insbesondere Methyl; Halogen wie oben genannt, insbesondere Fluor und Chlor; Alkoxy wie oben genannt, insbesondere Methoxy und Ethoxy oder Alkoxycarbonyl wie Methoxycarbonyl und Ethoxycarbonyl, insbesondere Methoxycarbonyl;

ein kondensierter aromatischer Rest, ausgewählt aus der Gruppe Benzofuran-2-yl, Benzofuran-3-yl, Isobenzofuran-2-yl, Benzothiophen-2-yl, Benzothiophen-3-yl, Isobenzothiophen-2-yl, Indol-2-yl, Indol-3-yl, 1,2-Benzisoxal-3-yl, Benzoxazol-2-yl, 1,2-Benzisothiazol-3-yl, Benzothiazol-2-yl, Indazol-3-yl, (1H)-Benzimidazhol-2-yl, Chinolin-3-yl, Chinolin-5-yl, Chinolin-6-yl, Chinolin-8-yl, Isochinolin-1-yl und Isochinolin-5-yl, wobei dieser Rest ein oder zwei der folgenden Reste tragen kann: Alkyl wie oben genannt, insbesondere Methyl; Halogen wie oben genannt, insbesondere Fluor und Chlor; Alkoxy wie oben genannt, insbesondere Methoxy und Ethoxy oder Alkoxycarbonyl wie Methoxycarbonyl und Ethoxycarbonyl, insbesondere Methoxycarbonyl;

Phenyl oder Phenyl-$C_1$-$C_4$-alkyl, z.B. Benzyl oder Phenylethyl, wobei der Phenylrest jeweils eine bis drei der folgenden Gruppen tragen kann: Alkyl wie oben genannt, insbesondere Methyl, Ethyl und iso-Propyl; Halogenalkyl wie vorstehend genannt, insbesondere Trifluormethyl und Chlordifluormethyl; Alkoxy wie vorstehend genannt, insbesondere Methoxy und Ethoxy; Halogenalkoxy wie vorstehend genannt, insbesondere Trifluormethoxy, Pentafluorethoxy und Trichlormethoxy; Alkylthio wie vorstehend genannt, insbesondere Methylthio und Ethylthio; Halogenalkylthio wie vorstehend genannt, insbesondere Difluormethylthio, Pentafluorethylthio und Trifluormethylthio; Halogen wie vorstehend genannt, insbesondere Fluor und Chlor, Cyano oder Nitro;

$R^2$

Wasserstoff; Hydroxy;

$C_1$-$C_4$-Alkoxy wie unter $R^1$ genannt, insbesondere Methoxy und Ethoxy;

$C_1$-$C_6$-Alkyl wie unter $R^1$ genannt, insbesondere Methyl, Ethyl, Propyl, 1-Methylethyl, Butyl, 1-Methylpropyl, 2-Methylpropyl und 1,1-Dimethylethyl, 1,1-Dimethylpropyl, 1,1-Dimethylbutyl, 1,2-Dimethylpropyl, welches eine bis drei der folgenden Gruppen tragen kann: Cyano, Alkoxy-alkoxy wie Methoxy-methoxy, Ethoxy-ethoxy insbesondere Methoxy-methoxy, Alkoxy wie unter $R^1$ genannt, insbesondere Methoxy und Ethoxy; Halogenalkoxy wie unter $R^1$ genannt, insbesondere Trifluormethoxy; Alkylthio wie unter $R^1$ genannt,

insbesondere Methylthio und Ethylthio; Halogenalkylthio wie unter $R^1$ genannt, insbesondere Trifluormethylthio; Dialkylamino insbesondere Dimethylamino und Diethylamino; Halogen wie unter $R^1$ genannt, insbesondere Fluor und Chlor; Cycloalkyl wie bei $R^1$ genannt, insbesondere Cyclopropyl, Cyclopentyl und Cyclohexyl; oder einmal Phenyl, wobei der Phenylrest seinerseits eine bis drei der folgenden Gruppen tragen kann: Halogen; Cyano; Nitro; Alkyl wie unter $R^1$ genannt, insbesondere Methyl und Ethyl; Halogenalkyl wie unter $R^1$ genannt, insbesondere Trifluormethyl; Alkoxy wie unter $R^1$ genannt, insbesondere Methoxy und Ethoxy; Halogenalkoxy wie unter $R^1$ genannt, insbesondere Trifluormethoxy; Alkylthio wie unter $R^1$ genannt, insbesondere Methylthio und Ethylthio; oder Halogenalkylthio wie unter $R^1$ genannt, insbesondere Trifluormethylthio;

$C_3$-$C_8$-Cycloalkyl wie unter $R^1$ genannt, insbesondere Cyclopropyl, Cyclobutyl, Cyclopentyl und Cyclohexyl, das eine bis drei der folgenden Gruppen tragen kann: Alkyl wie unter $R^1$ genannt, insbesondere Methyl, Ethyl und 1-Methylethyl; Halogenalkyl wie unter $R^1$ genannt, insbesondere Trifluormethyl; Alkoxy wie unter $R^1$ genannt, insbesondere Methoxy und Ethoxy; Halogenalkoxy wie unter $R^1$ genannt, insbesondere Trifluormethoxy; Halogen wie unter $R^1$ genannt, insbesondere Fluor und Chlor; Nitro oder Cyano;

$C_3$-$C_6$-Alkenyl wie unter $R^1$ genannt, insbesondere 2-Propenyl, 1-Methyl-2-propenyl, 1,1-Dimethyl-2-propenyl und 2-Butenyl, welches ein-bis dreimal durch Halogen wie unter $R^1$ genannt, insbesondere Fluor und Chlor, und/oder einmal durch Phenyl substituiert sein kann, wobei der Phenylrest seinerseits eine bis drei der folgenden Gruppen tragen kann: Alkyl wie unter $R^1$ genannt, insbesondere Methyl und Ethyl; Halogenalkyl wie unter $R^1$ genannt, insbesondere Trifluormethyl; Alkoxy wie unter $R^1$ genannt, insbesondere Methoxy und Ethoxy; Halogenalkoxy wie unter $R^1$ genannt, insbesondere Trifluormethoxy; Alkylthio wie unter $R^1$ genannt, insbesondere Methylthio und Ethylthio; Halogenalkylthio wie unter $R^1$ genannt, insbesondere Trifluormethylthio; Halogen wie unter $R^1$ genannt, insbesondere Fluor und Chlor; Cyano oder Nitro;

$C_3$-$C_6$-Alkinyl wie unter $R^1$ genannt, insbesondere 2-Propinyl, 1-Methyl-2-propinyl und 1,1-Dimethyl-2-propinyl, welches ein- bis dreimal durch Halogen wie unter $R^1$ genannt, insbesondere Fluor und Chlor; und/oder einmal durch Phenyl substituiert sein kann, wobei der Phenylrest seinerseits eine bis drei der folgenden Gruppen tragen kann: Alkyl wie unter $R^1$ genannt, insbesondere Methyl und Ethyl; Halogenalkyl wie unter $R^1$ genannt, insbesondere Trifluormethyl, Alkoxy wie unter $R^1$ genannt, insbesondere Methyl und Ethyl; Halogenalkyl wie unter $R^1$ genannt, insbesondere Trifluormethyl, Alkoxy wie unter $R^1$ genannt, insbesondere Methoxy und Ethoxy; Halogenalkoxy wie unter $R^1$ genannt, insbesondere Methylthio und Ethylthio; Halogenalkylthio wie unter $R^1$ genannt, insbesondere Trifluormethylthio; Halogen wie unter $R^1$ genannt, insbesondere Fluor und Chlor; Cyano oder Nitro;

$C_1$-$C_4$-Dialkylamino, insbesondere Dimethylamino und Diethylamino;

ein 5- bis 6-gliedriger heterocyclischer Rest mit einem oder zwei Heteroatomen, ausgewählt aus der Gruppe Sauerstoff, Schwefel oder Stickstoff wie unter $R^1$ genannt, der ein- bis dreimal durch Alkyl wie unter $R^1$ genannt, insbesondere Methyl, Ethyl und 1-Methylethyl; oder Halogen wie unter $R^1$ genannt, insbesondere Fluor und Chlor substituiert sein kann;

ein kondensierter aromatischer Rest, ausgewählt aus der Gruppe Benzofuran-2-yl, Benzofuran-3-yl, Isobenzofuran-2-yl, Benzothiophen-2-yl, Benzothiophen-3-yl, Isobenzothiophen-2-yl, Indol-2-yl, Indol-3-yl, 1,2-Benzisoxal-3-yl, Benzoxazol-2-yl, 1,2-Benzisothiazol-3-yl, Benzothiazol-2-yl, Indazol-3-yl, (1H)-Benzimidazhol-2-yl, Chinolin-3-yl, Chinolin-5-yl, Chinolin-6-yl, Chinolin-8-yl, Isochinolin-1-yl und Isochinolin-5-yl, wobei dieser Rest ein- bis dreimal durch $C_1$-$C_4$-Alkyl oder Halogen substituiert sein kann;

Phenyl, das eine bis vier der folgenden Gruppen tragen kann: Alkyl wie unter $R^1$ genannt, insbesondere Methyl, Ethyl und 1-Methylethyl; Halogenalkyl wie unter $R^1$ genannt, insbesondere Trifluormethyl; Alkoxy wie unter $R^1$ genannt, insbesondere Methoxy und Ethoxy; Halogenalkoxy wie unter $R^1$ genannt, insbesondere Trifluormethoxy; Alkylthio wie unter $R^1$ genannt, insbesondere Methylthio und Ethylthio; Halogenalkylthio wie unter $R^1$ genannt, insbesondere Trifluormethylthio; Halogen wie unter $R^1$ genannt, insbesondere Fluor und Chlor; Nitro, Cyano, Formyl; $C_1$-$C_4$-Alkanoyl wie Acetyl, Propionyl, Butyryl, insbesondere Acetyl; Halogenalkanoyl wie Trifluoracetyl, Trichloracetyl, Pentafluorpropionyl, insbesondere Trifluoracetyl; oder Alkoxycarbonyl wie unter $R^1$ genannt, insbesondere Methoxycarbonyl;

Naphthyl, das ein- bis dreimal durch Alkyl wie unter $R^1$ genannt, insbesondere Methyl und Ethyl, oder Halogen wie unter $R^1$ genannt, insbesondere Fluor und Chlor substituiert sein kann.

Beispiele für herbizid wirksame Verbindungen der Formel Ia und Ib sind nachstehend im einzelnen aufgeführt:

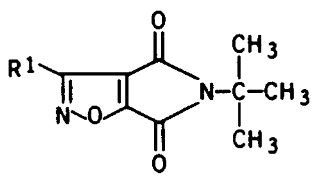

oder

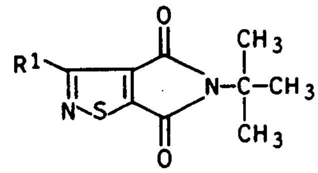

wobei $R^1$ jeweils die folgende Bedeutung hat:
Wasserstoff, Fluor, Chlor, Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, cyclo-Propyl, cyclo-Butyl, cyclo-Pentyl, cyclo-Hexyl, cyclo-Heptyl, cyclo-Octyl, 1-Methyl-cyclo-Propyl, cyclo-Propyl-methyl, 1-(cyclo-Propyl)-ethyl, Chlormethyl, Dichlormethyl, Trichlormethyl, Chlordifluormethyl, Trifluormethyl, Pentafluorethyl, Difluormethyl, 1-Chlorethyl, 2-Chlorethyl, 1-Methyl-1-chlorethyl, 1-Methyl-2-chlorethyl, Methoxymethyl, 1-Methylmethoxymethyl, 1-Methyl-2-methoxyethyl, 1-Methylethoxymethyl, Ethoxymethyl, Ethenyl, 1-Propenyl, 2-Propenyl, 1-Methylethenyl, 1-Ethylethenyl, 2-Phenylethenyl, 1-Butenyl, 2-Butenyl, 3-Butenyl, 1-Methyl-1-propenyl, 1-Methyl-2-propenyl, 2-Methyl-2-propenyl, 2-Propinyl, Methoxy, Ethoxy, Propoxy, 1-Methylethoxy, Butoxy, 1-Methylpropoxy, 1,1-Dimethylethoxy, Methylthio, Ethylthio, Chlordifluormethoxy, Trifluormethoxy, Trichlormethylthio, Phenyl, 2-Fluorphenyl, 3-Fluorphenyl, 4-Fluorphenyl, 2-Chlorphenyl, 3-Chlorphenyl, 4-Chlorphenyl, 2-Methylphenyl, 3-Methylphenyl, 4-Methylphenyl, 2-Trifluormethylphenyl, 3-Trifluormethylphenyl, 4-Trifluormethylphenyl, 2-Methoxyphenyl, 3-Methoxyphenyl, 4-Methoxyphenyl, 2,4-Dichlorphenyl, 2,4,6-Trimethylphenyl, Phenoxy, Phenylthio, 2-Chlorphenoxy, 3-Chlorphenoxy, 4-Chlorphenoxy, 2,4-Dichlorphenoxy, Benzyl, 2-Chlorbenzyl, 3-Chlorbenzyl, 4-Chlorbenzyl, 2-Fluorbenzyl, 3-Fluorbenzyl, 4-Fluorbenzyl, 2-Thienyl, 3-Thienyl, 2-Pyridyl, 3-Pyridyl, 4-Pyridyl;

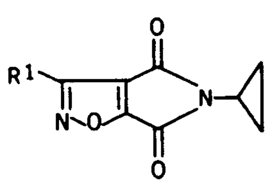

oder

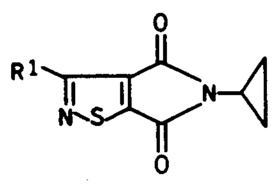

wobei $R^1$ jeweils die folgende Bedeutung hat:
Wasserstoff, Fluor, Chlor, Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, cyclo-Propyl, cyclo-Butyl, cyclo-Pentyl, cyclo-Hexyl, cyclo-Heptyl, cyclo-Octyl, 1-Methyl-cyclo-Propyl, cyclo-Propyl-methyl, 1-(cyclo-Propyl)-ethyl, Chlormethyl, Dichlormethyl, Trichlormethyl, Chlordifluormethyl, Trifluormethyl, Pentafluorethyl, Difluormethyl, 1-Chlorethyl, 2-Chlorethyl, 1-Methyl-1-chlorethyl, 1-Methyl-2-chlorethyl, Methoxymethyl, 1-Methylmethoxymethyl, 1-Methyl-2-methoxyethyl, 1-Methylethoxymethyl, Ethoxymethyl, Ethenyl, 1-Propenyl, 2-Propenyl, 1-Methylethenyl, 1-Ethylethenyl, 2-Phenylethenyl, 1-Butenyl, 2-Butenyl, 3-Butenyl, 1-Methyl-1-propenyl, 1-Methyl-2-propenyl, 2-Methyl-2-propenyl, 2-Propinyl, Methoxy, Ethoxy, Propoxy, 1-Methylethoxy, Butoxy, 1-Methylpropoxy, 1,1-Dimethylethoxy, Methylthio, Ethylthio, Chlordifluormethoxy, Trifluormethoxy, Trichlormethylthio, Phenyl, 2-Fluorphenyl, 3-Fluorphenyl, 4-Fluorphenyl, 2-Chlorphenyl, 3-Chlorphenyl, 4-Chlorphenyl, 2-Methylphenyl, 3-Methylphenyl, 4-Methylphenyl, 2-Trifluormethylphenyl, 3-Trifluormethylphenyl, 4-Trifluormethylphenyl, 2-Methoxyphenyl, 3-Methoxyphenyl, 4-Methoxyphenyl, 2,4-Dichlorphenyl, 2,4,6-Trimethylphenyl, Phenoxy, Phenylthio, 2-Chlorphenoxy, 3-Chlorphenoxy, 4-Chlorphenoxy, 2,4-Dichlorphenoxy, Benzyl, 2-Chlorbenzyl, 3-Chlorbenzyl, 4-Chlorbenzyl, 2-Fluorbenzyl, 3-Fluorbenzyl, 4-Fluorbenzyl, 2-Thienyl, 3-Thienyl, 2-Pyridyl, 3-Pyridyl, 4-Pyridyl;

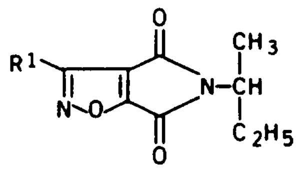

oder

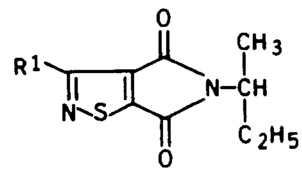

wobei $R^1$ jeweils die folgende Bedeutung hat:
Wasserstoff, Fluor, Chlor, Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, cyclo-Propyl, cyclo-Butyl, cyclo-Pentyl, cyclo-Hexyl, cyclo-Heptyl, cyclo-Octyl, 1-Methyl-cyclo-Propyl, cyclo-Propyl-methyl, 1-(cyclo-Propyl)-ethyl, Chlormethyl, Dichlormethyl, Trichlormethyl, Chlordifluormethyl, Trifluormethyl, Pentafluorethyl, Difluormethyl, 1-Chlorethyl, 2-Chlorethyl, 1-Methyl-1-chlorethyl, 1-Methyl-2-chlorethyl, Methoxymethyl, 1-Methylmethoxymethyl, 1-Methyl-2-methoxyethyl, 1-Methylethoxymethyl, Ethoxymethyl, Ethenyl, 1-Propenyl, 2-Propenyl, 1-Methylethenyl, 1-Ethylethenyl, 2-Phenylethenyl, 1-Butenyl, 2-Butenyl, 3-Butenyl, 1-Methyl-1-propenyl, 1-Methyl-2-propenyl, 2-Methyl-2-propenyl, 2-Propinyl, Methoxy, Ethoxy, Propoxy, 1-Methylethoxy, Butoxy, 1-Methylpropoxy, 1,1-Dimethylethoxy, Methylthio, Ethylthio, Chlordifluormethoxy, Trifluormethoxy, Trichlormethylthio, Phenyl, 2-Fluorphenyl, 3-Fluorphenyl, 4-Fluorphenyl, 2-Chlorphenyl, 3-Chlorphenyl, 4-Chlorphenyl, 2-Methylphenyl, 3-Methylphenyl, 4-Methylphenyl, 2-Trifluormethylphenyl, 3-Trifluormethylphenyl, 4-Trifluormethylphenyl, 2-Methoxyphenyl, 3-Methoxyphenyl, 4-Methoxyphenyl, 2,4-Dichlorphenyl, 2,4,6-Trimethylphenyl, Phenoxy, Phenylthio, 2-Chlorphenoxy, 3-Chlorphenoxy, 4-Chlorphenoxy, 2,4-Dichlorphenoxy, Benzyl, 2-Chlorbenzyl, 3-Chlorbenzyl, 4-Chlorbenzyl, 2-Fluorbenzyl, 3-Fluorbenzyl, 4-Fluorbenzyl, 2-Thienyl, 3-Thienyl, 2-Pyridyl, 3-Pyridyl, 4-Pyridyl;

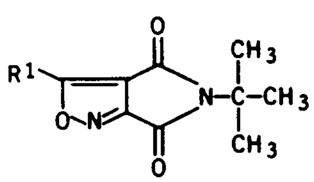

oder

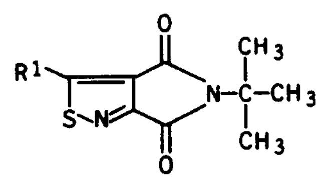

wobei $R^1$ jeweils die folgende Bedeutung hat:
Wasserstoff, Fluor, Chlor, Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, cyclo-Propyl, cyclo-Butyl, cyclo-Pentyl, cyclo-Hexyl, cyclo-Heptyl, cyclo-Octyl, 1-Methyl-cyclo-Propyl, cyclo-Propyl-methyl, 1-(cyclo-Propyl)-ethyl, Chlormethyl, Dichlormethyl, Trichlormethyl, Chlordifluormethyl, Trifluormethyl, Pentafluorethyl, Difluormethyl, 1-Chlorethyl, 2-Chlorethyl, 1-Methyl-1-chlorethyl, 1-Methyl-2-chlorethyl, Methoxymethyl, 1-Methylmethoxymethyl, 1-Methyl-2-methoxyethyl, 1-Methylethoxymethyl, Ethoxymethyl, Ethenyl, 1-Propenyl, 2-Propenyl, 1-Methylethenyl, 1-Ethylethenyl, 2-Phenylethenyl, 1-Butenyl, 2-Butenyl, 3-Butenyl, 1-Methyl-1-propenyl, 1-Methyl-2-propenyl, 2-Methyl-2-propenyl, 2-Propinyl, Methoxy, Ethoxy, Propoxy, 1-Methylethoxy, Butoxy, 1-Methylpropoxy, 1,1-Dimethylethoxy, Methylthio, Ethylthio, Chlordifluormethoxy, Trifluormethoxy, Trichlormethylthio, Phenyl, 2-Fluorphenyl, 3-Fluorphenyl, 4-Fluorphenyl, 2-Chlorphenyl, 3-Chlorphenyl, 4-Chlorphenyl, 2-Methylphenyl, 3-Methylphenyl, 4-Methylphenyl, 2-Trifluormethylphenyl, 3-Trifluormethylphenyl, 4-Trifluormethylphenyl, 2-Methoxyphenyl, 3-Methoxyphenyl, 4-Methoxyphenyl, 2,4-Dichlorphenyl, 2,4,6-Trimethylphenyl, Phenoxy, Phenylthio, 2-Chlorphenoxy, 3-Chlorphenoxy, 4-Chlorphenoxy, 2,4-Dichlorphenoxy, Benzyl, 2-Chlorbenzyl, 3-Chlorbenzyl, 4-Chlorbenzyl, 2-Fluorbenzyl, 3-Fluorbenzyl, 4-Fluorbenzyl, 2-Thienyl, 3-Thienyl, 2-Pyridyl, 3-Pyridyl, 4-Pyridyl;

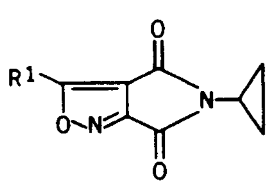

oder

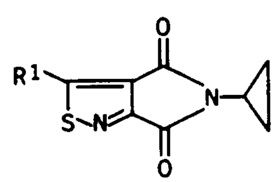

wobei $R^1$ jeweils die folgende Bedeutung hat:
Wasserstoff, Fluor, Chlor, Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, cyclo-Propyl, cyclo-Butyl, cyclo-Pentyl, cyclo-Hexyl, cyclo-Heptyl, cyclo-Octyl, 1-Methyl-cyclo-Propyl, cyclo-Propyl-methyl, 1-(cyclo-Propyl)-ethyl, Chlormethyl, Dichlormethyl, Trichlormethyl, Chlordifluormethyl, Trifluormethyl, Pentafluorethyl, Difluormethyl, 1-Chlorethyl, 2-Chlorethyl, 1-Methyl-1-chlorethyl, 1-Methyl-2-chlorethyl, Methoxymethyl, 1-Methylmethoxymethyl, 1-Methyl-2-methoxyethyl, 1-Methylethoxymethyl, Ethoxymethyl, Ethenyl, 1-Propenyl, 2-Propenyl, 1-Methylethenyl, 1-Ethylethenyl, 2-Phenylethenyl, 1-Butenyl, 2-Butenyl, 3-Butenyl, 1-Methyl-1-propenyl, 1-Methyl-2-propenyl, 2-Methyl-2-propenyl, 2-Propinyl, Methoxy, Ethoxy, Propoxy, 1-Methylethoxy, Butoxy, 1-Methylpropoxy, 1,1-Dimethylethoxy, Methylthio, Ethylthio, Chlordifluormethoxy, Trifluormethoxy, Trichlormethylthio, Phenyl, 2-Fluorphenyl, 3-Fluorphenyl, 4-Fluorphenyl, 2-Chlorphenyl, 3-Chlorphenyl, 4-Chlorphenyl, 2-Methylphenyl, 3-Methylphenyl, 4-Methylphenyl, 2-Trifluormethylphenyl, 3-Trifluormethylphenyl, 4-Trifluormethylphenyl, 2-Methoxyphenyl, 3-Methoxyphenyl, 4-Methoxyphenyl, 2,4-Dichlorphenyl, 2,4,6-Trimethylphenyl, Phenoxy, Phenylthio, 2-Chlorphenoxy, 3-Chlorphenoxy, 4-Chlorphenoxy, 2,4-Dichlorphenoxy, Benzyl, 2-Chlorbenzyl, 3-Chlorbenzyl, 4-Chlorbenzyl, 2-Fluorbenzyl, 3-Fluorbenzyl, 4-Fluorbenzyl, 2-Thienyl, 3-Thienyl, 2-Pyridyl, 3-Pyridyl, 4-Pyridyl;

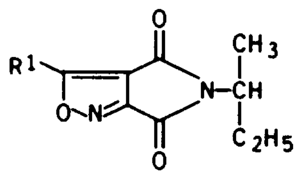

oder

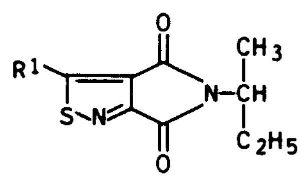

wobei $R^1$ jeweils die folgende Bedeutung hat:
Wasserstoff, Fluor, Chlor, Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, cyclo-Propyl, cyclo-Butyl, cyclo-Pentyl, cyclo-Hexyl, cyclo-Heptyl, cyclo-Octyl, 1-Methyl-cyclo-Propyl, cyclo-Propyl-methyl, 1-(cyclo-Propyl)-ethyl, Chlormethyl, Dichlormethyl, Trichlormethyl, Chlordifluormethyl, Trifluormethyl, Pentafluorethyl, Difluormethyl, 1-Chlorethyl, 2-Chlorethyl, 1-Methyl-1-chlorethyl, 1-Methyl-2-chlorethyl, Methoxymethyl, 1-Methylmethoxymethyl, 1-Methyl-2-methoxyethyl, 1-Methylethoxymethyl, Ethoxymethyl, Ethenyl, 1-Propenyl, 2-Propenyl, 1-Methylethenyl, 1-Ethylethenyl, 2-Phenylethenyl, 1-Butenyl, 2-Butenyl, 3-Butenyl, 1-Methyl-1-propenyl, 1-Methyl-2-propenyl, 2-Methyl-2-propenyl, 2-Propinyl, Methoxy, Ethoxy, Propoxy, 1-Methylethoxy, Butoxy, 1-Methylpropoxy, 1,1-Dimethylethoxy, Methylthio, Ethylthio, Chlordifluormethoxy, Trifluormethoxy, Trichlormethylthio, Phenyl, 2-Fluorphenyl, 3-Fluorphenyl, 4-Fluorphenyl, 2-Chlorphenyl, 3-Chlorphenyl, 4-Chlorphenyl, 2-Methylphenyl, 3-Methylphenyl, 4-Methylphenyl, 2-Trifluormethylphenyl, 3-Trifluormethylphenyl, 4-Trifluormethylphenyl, 2-Methoxyphenyl, 3-Methoxyphenyl, 4-Methoxyphenyl, 2,4-Dichlorphenyl, 2,4,6-Trimethylphenyl, Phenoxy, Phenylthio, 2-Chlorphenoxy, 3-Chlorphenoxy, 4-Chlorphenoxy, 2,4-Dichlorphenoxy, Benzyl, 2-Chlorbenzyl, 3-Chlorbenzyl, 4-Chlorbenzyl, 2-Fluorbenzyl, 3-Fluorbenzyl, 4-Fluorbenzyl, 2-Thienyl, 3-Thienyl, 2-Pyridyl, 3-Pyridyl, 4-Pyridyl.

Beispiele für herbizid wirksame Verbindungen der Formel Ic sind nachstehend im einzelnen aufgeführt:

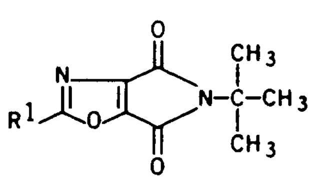

oder

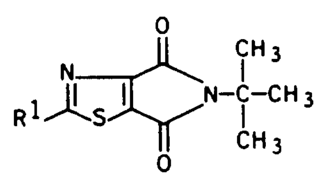

wobei $R^1$ jeweils die folgende Bedeutung hat:
Wasserstoff, Fluor, Chlor, Cyano, Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, cyclo-Propyl, cyclo-Butyl, cyclo-Pentyl, cyclo-Hexyl, cyclo-Heptyl, cyclo-Octyl, 1-Methyl-cyclo-Propyl, cyclo-Propyl-methyl, 1-(cyclo-Propyl)-ethyl, Chlormethyl, Dichlormethyl, Trichlormethyl, Chlordifluormethyl, Trifluormethyl, Pentafluorethyl, Difluormethyl, 1-Chlorethyl, 2-Chlorethyl, 1-Methyl-1-chlorethyl, 1-Methyl-2-chlorethyl, Methoxymethyl, 1-Methylmethoxymethyl, 1-Methyl-2-methoxyethyl, 1-Methylethoxymethyl, Ethoxymethyl, Ethenyl, 1-Propenyl, 2-Propenyl, 1-Methylethenyl, 1-Ethylethenyl, 2-Phenylethenyl, 1-Butenyl, 2-Butenyl, 3-Butenyl, 1-Methyl-1-propenyl, 1-Methyl-2-propenyl, 2-Methyl-2-propenyl, 2-Propinyl, Methoxy, Ethoxy, Propoxy, 1-Methylethoxy, Butoxy, 1-Methylpropoxy, 1,1-Dimethylethoxy, Methylthio, Ethylthio, Chlordifluormethoxy, Trifluormethoxy, Trichlormethylthio, Phenyl, 2-Fluorphenyl, 3-Fluorphenyl, 4-Fluorphenyl, 2-Chlorphenyl, 3-Chlorphenyl, 4-Chlorphenyl, 2-Methylphenyl, 3-Methylphenyl, 4-Methylphenyl, 2-Trifluormethylphenyl, 3-Trifluormethylphenyl, 4-Trifluormethylphenyl, 2-Methoxyphenyl, 3-Methoxyphenyl, 4-Methoxyphenyl, 2,4-Dichlorphenyl, 2,4,6-Trimethylphenyl, Phenoxy, Phenylthio, 2-Chlorphenoxy, 3-Chlorphenoxy, 4-Chlorphenoxy, 2,4-Dichlorphenoxy, Benzyl, 2-Chlorbenzyl, 3-Chlorbenzyl, 4-Chlorbenzyl, 2-Fluorbenzyl, 3-Fluorbenzyl, 4-Fluorbenzyl, 2-Thienyl, 3-Thienyl, 2-Pyridyl, 3-Pyridyl, 4-Pyridyl;

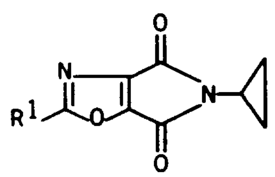

oder

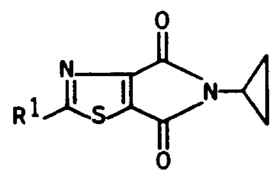

wobei $R^1$ jeweils die folgende Bedeutung hat:
Wasserstoff, Fluor, Chlor, Cyano, Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, cyclo-Propyl, cyclo-Butyl, cyclo-Pentyl, cyclo-Hexyl, cyclo-Heptyl, cyclo-Octyl, 1-Methyl-cyclo-Propyl, cyclo-Propyl-methyl, 1-(cyclo-Propyl)-ethyl, Chlormethyl, Dichlormethyl, Trichlormethyl, Chlordifluormethyl, Trifluormethyl, Pentafluorethyl, Difluormethyl, 1-Chlorethyl, 2-Chlorethyl, 1-Methyl-1-chlorethyl, 1-Methyl-2-chlorethyl, Methoxymethyl, 1-Methylmethoxymethyl, 1-Methyl-2-methoxyethyl, 1-Methylethoxymethyl, Ethoxymethyl, Ethenyl, 1-Propenyl, 2-Propenyl, 1-Methylethenyl, 1-Ethylethenyl, 2-Phenylethenyl, 1-Butenyl, 2-Butenyl, 3-Butenyl, 1-Methyl-1-propenyl, 1-Methyl-2-propenyl, 2-Methyl-2-propenyl, 2-Propinyl, Methoxy, Ethoxy, Propoxy, 1-Methylethoxy, Butoxy, 1-Methyl-propoxy, 1,1-Dimethylethoxy, Methylthio, Ethylthio, Chlordifluormethoxy, Trifluormethoxy, Trichlormethylthio, Phenyl, 2-Fluorphenyl, 3-Fluorphenyl, 4-Fluorphenyl, 2-Chlorphenyl, 3-Chlorphenyl, 4-Chlorphenyl, 2-Methylphenyl, 3-Methylphenyl, 4-Methylphenyl, 2-Trifluormethylphenyl, 3-Trifluormethylphenyl, 4-Trifluormethylphenyl, 2-Methoxyphenyl, 3-Methoxyphenyl, 4-Methoxyphenyl, 2,4-Dichlorphenyl, 2,4,6-Trimethylphenyl, Phenoxy, Phenylthio, 2-Chlorphenoxy, 3-Chlorphenoxy, 4-Chlorphenoxy, 2,4-Dichlorphenoxy, Benzyl, 2-Chlorbenzyl, 3-Chlorbenzyl, 4-Chlorbenzyl, 2-Fluorbenzyl, 3-Fluorbenzyl, 4-Fluorbenzyl, 2-Thienyl, 3-Thienyl, 2-Pyridyl, 3-Pyridyl, 4-Pyridyl;

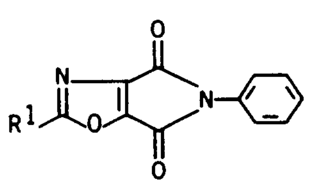

oder

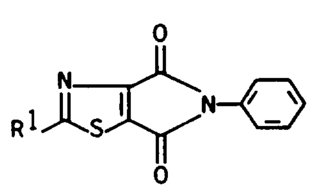

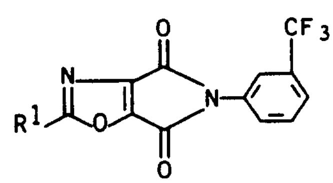

oder

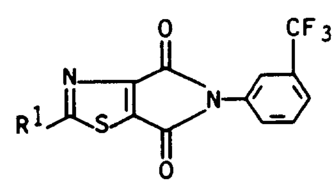

wobei $R^1$ jeweils die folgende Bedeutung hat:
Wasserstoff, Fluor, Chlor, Cyano, Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, cyclo-Propyl, cyclo-Butyl, cyclo-Pentyl, cyclo-Hexyl, cyclo-Heptyl, cyclo-Octyl, 1-Methyl-cyclo-Propyl, cyclo-Propyl-methyl, 1-(cyclo-Propyl)-ethyl, Chlormethyl, Dichlormethyl, Trichlormethyl, Chlordifluormethyl, Trifluormethyl, Pentafluorethyl, Difluormethyl, 1-Chlorethyl, 2-Chlorethyl, 1-Methyl-1-chlorethyl, 1-Methyl-2-chlorethyl, Methoxymethyl, 1-Methylmethoxymethyl, 1-Methyl-2-methoxyethyl, 1-Methylethoxymethyl, Ethoxymethyl, Ethenyl, 1-Propenyl, 2-Propenyl, 1-Methylethenyl, 1-Ethylethenyl, 2-Phenylethenyl, 1-Butenyl, 2-Butenyl, 3-Butenyl, 1-Methyl-1-propenyl, 1-Methyl-2-propenyl, 2-Methyl-2-propenyl, 2-Propinyl, Methoxy, Ethoxy, Propoxy, 1-Methylethoxy, Butoxy, 1-Methylpropoxy, 1,1-Dimethylethoxy, Methylthio, Ethylthio, Chlordifluormethoxy, Trifluor-methoxy, Trichlormethylthio, Phenyl, 2-Fluorphenyl, 3-Fluorphenyl, 4-Fluorphenyl, 2-Chlorphenyl, 3-Chlorphenyl, 4-Chlorphenyl, 2-Methylphenyl, 3-Methylphenyl, 4-Methylphenyl, 2-Trifluormethylphenyl, 3-Trifluormethylphenyl, 4-Trifluormethylphenyl, 2-Methoxyphenyl, 3-Methoxyphenyl, 4-Methoxyphenyl, 2,4-Dichlorphenyl, 2,4,6-Trimethylphenyl, Phenoxy, Phenylthio, 2-Chlorphenoxy, 3-Chlorphenoxy, 4-Chlorphenoxy, 2,4-Dichlorphenoxy, Benzyl, 2-Chlorbenzyl, 3-Chlorbenzyl, 4-Chlorbenzyl, 2-Fluorbenzyl, 3-Fluorbenzyl, 4-Fluorbenzyl, 2-Thienyl, 3-Thienyl, 2-Pyridyl, 3-Pyridyl, 4-Pyridyl;

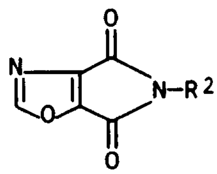 bzw. 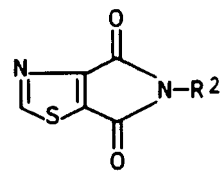

oder

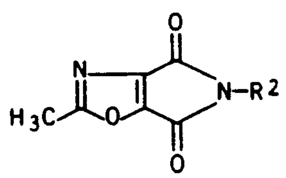 bzw. 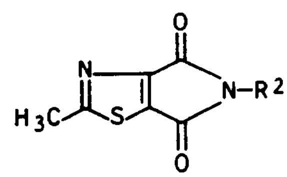

oder

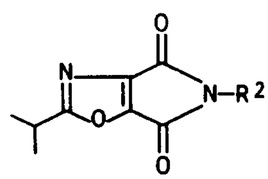 bzw. 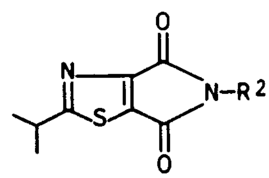

oder

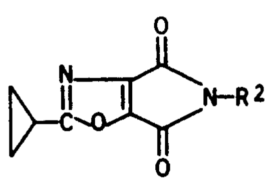 bzw. 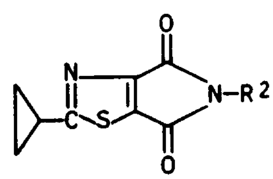

oder

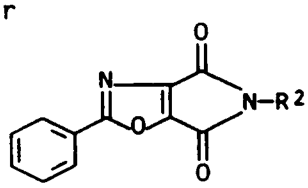 bzw. 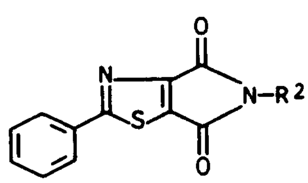

wobei $R^2$ jeweils die folgende Bedeutung hat:

Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, cyclo-Propyl, cyclo-Butyl, cyclo-Pentyl, cyclo-Hexyl, cyclo-Heptyl, cyclo-Octyl, 1-Methyl-cyclo-Propyl, cyclo-Propyl-methyl, 1-(cyclo-Propyl)-ethyl, 1-Methylcyclohexyl, 1-Ethylcyclohexyl, Cyclohexylmethyl, 2-Propenyl,

1-Methyl-2-propenyl, 1,1-Dimethyl-2-propenyl, 2-Propinyl, 1-Methyl-2-propinyl, 1,1-Dimethylpropinyl, Phenylmethyl, 1-Methylphenylmethyl, 1,1-Dimethylphenylmethyl, 2-Phenylethyl, 2-Methylthioethyl, 1-Methyl-2-methylthioethyl, 1,1-Dimethyl-2-methylthioethyl, 3-Methylthiopropyl, 2-Fluorethyl, 2-Fluor-1-methylethyl, 1,1-Dimethyl-2-fluorethyl, 2-Chlorethyl, 2-Chlor-1-methylethyl, 2-Chlor-1,1-Dimethylethyl, 2-Methoxyethyl, 2-Methoxy-1-methylethyl, 1,1-Dimethyl-2-methoxyethyl, 3-Methoxypropyl, 2-Cyanoethyl, 2-Cyano-1-methylethyl, 2-Cyano-1,1-dimethylethyl, Dimethylamino, Diethylamino, Morpholino, Piperidino, Phenyl, 2-Methylphenyl, 3-Methylphenyl, 4-Methylphenyl, 2-Ethylphenyl, 3-Ethylphenyl, 4-Ethylphenyl, 2,3-Dimethylphenyl, 2,4-Dimethylphenyl, 2,5-Dimethylphenyl, 2,6-Dimethylphenyl, 3,4-Dimethylphenyl, 3,5-Dimethylphenyl, 2,3,4-Trimethylphenyl, 2,3,5-Trimethylphenyl, 2,4,5-Trimethylphenyl, 2,4,6-Trimethylphenyl, 3,4,5-Trimethylphenyl, 2-Trifluormethylphenyl, 3-Trifluormethylphenyl, 4-Trifluormethylphenyl, 2-Fluorphenyl, 3-Fluorphenyl, 4-Fluorphenyl, 2-Chlorphenyl, 3-Chlorphenyl, 4-Chlorphenyl, 2,3-Difluorphenyl, 2,4-Difluorphenyl, 2,5-Difluorphenyl, 2,6-Difluorphenyl, 2,3-Dichlorphenyl, 2,4-Dichlorphenyl, 2,5-Dichlorphenyl, 2,6-Dichlorphenyl, 2,3,4-Trichlorphenyl, 2,3,5-Trichlorphenyl, 2,4,6-Trichlorphenyl, 3,4,5-Trichlorphenyl, 2-Cyanophenyl, 3-Cyanophenyl, 4-Cyanophenyl, 2-Methoxyphenyl, 3-Methoxyphenyl, 4-Methoxyphenyl, 2,3-Dimethoxyphenyl, 2,4-Dimethoxyphenyl, 2,5-Dimethoxyphenyl, 2,6-Dimethoxyphenyl, 3,4-Dimethoxyphenyl, 3,5-Dimethoxyphenyl, 3,4,5-Dimethoxyphenyl, 2-Trifluormethoxyphenyl, 3-Trifluormethoxyphenyl, 4-Trifluormethoxyphenyl, 2-Nitrophenyl, 3-Nitrophenyl, 4-Nitrophenyl, 2,3-Dinitrophenyl, 2,4-Dinitrophenyl, 2,5-Dinitrophenyl, 2,6-Dinitrophenyl, 3,4-Dinitrophenyl, 3,5-Dinitrophenyl, 1-Naphthyl, 2-Naphthyl, 3-Tetrahydrofuryl, 4-Tetrahydropyranyl, 2-Thiazolyl.

Die vorstehend genannten Restedefinitionen für $R^1$ und $R^2$ können darüberhinaus in den Oxazol- bzw. Thiazol-4,5-dicarbonsäureimiden zu anderen als den aufgeführten Kombinationen miteinander kombiniert werden.

Als Salze der Verbindungen der Formeln Ia, Ib und Ic kommen landwirtschaftlich brauchbare Salze, beispielsweise Alkalimetallsalze, wie das Kalium- oder Natriumsalz, Erdalkalimetallsalze, wie das Calcium-, Magnesium- oder Bariumsalz, Mangan-, Kupfer-, Zink- oder Eisensalze sowie Ammonium, Phosphonium-, Sulfonium- oder Sulfoxoniumsalze, beispielsweise Ammoniumsalze, Tetraalkylammoniumsalze, Benzyltrialkylammoniumsalze, Trialkylsulfoniumsalze oder Trialkylsulfoxoniumsalze in Betracht.

Die erfindungsgemäßen herbiziden Verbindungen bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Die Verbindungen eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.-%, vorzugsweise zwischen 0,5 und 90 Gew.-%, Wirkstoff. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen I können beispielsweise wie folgt formuliert werden:

I. Man vermischt 90 Gewichtsteile der Verbindung Nr. 1.001 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 4.003 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 1.001 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 4.003 werden in einer Mischung gelöst, die aus 25 Gewichtsteilen Cyclohexanon, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 1.001 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 1.001 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 4.003 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 4.003 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation der herbiziden und wachstumsregulierenden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff bei Anwendung als Herbizide betragen je nach Bekämpfungsziel, Jahreszeit, Zielpflanzen und Wachstumsstadium 0,001 bis 3, vorzugsweise 0,01 bis 2 kg/ha aktive Substanz (a.S.).

In Anbetracht des erfaßbaren Wirkungsspektrums zur Unkrautbekämpfung, der Verträglichkeit für Kulturpflanzen oder der erwünschten Beeinflussung des Wachstums derselben sowie angesichts der Vielfalt der Applikationsmethoden können die erfindungsgemäßen Verbindungen in einer großen Zahl von Kulturpflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Beta vulgaris spp. altissima | Zuckerrübe |
| Beta vulgaris spp. rapa | Futterrübe |
| Brassica napus var. napus | Raps |
| Brassica napus var. napobrassica | Kohlrübe |
| Brassica rapa var. silvestris | Rübsen |
| Camellia sinensis | Teestrauch |
| Carthamus tinctorius | Saflor - Färberdistel |
| Carya illinoinensis | Pekannußbaum |
| Citrus limon | Zitrone |
| Citrus sinensis | Apfelsine, Orange |

| Botanischer Name | Deutscher Name |
|---|---|
| Coffea arabica (Coffea canephora, Coffea liberica) | Kaffee |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Musa spp. | Obst- und Mehlbanane |
| Nicotiana tabacum (N. rustica) | Tabak |
| Olea europaea | Ölbaum |
| Oryza sativa | Reis |
| Phaseolus lunatus | Mondbohne |
| Phaseolus vulgaris | Buschbohne |
| Picea abies | Rotfichte |
| Pinus spp. | Kiefer |
| Pisum sativum | Gartenerbse |
| Prunus avium | Süßkirsche |
| Prunus dulcis | Mandelbaum |
| Prunus persica | Pfirsich |
| Pyrus communis | Birne |
| Ribes sylvestre | Rote Johannisbeere |
| Ricinus communis | Rizinus |
| Saccharum officinarum | Zuckerrohr |
| Secale cereale | Roggen |
| Solanum tuberosum | Kartoffel |
| Sorghum bicolor (s. vulgare) | Mohrenhirse |
| Theobroma cacao | Kakaobaum |

| Botanischer Name | Deutscher Name |
|---|---|
| Trifolium pratense | Rotklee |
| Triticum aestivum | Weizen |
| Triticum durum | Hartweizen |
| Vicia faba | Pferdebohnen |
| Vitis vinifera | Weinrebe |
| Zea mays | Mais |

Zur Verbreiterung des Wirkungsspektrums und zur Erzielung synergistischer Effekte können die Verbindungen der Formel I sowohl untereinander als auch mit Vertretern anderer herbizider oder wachstumsregulierender Wirkstoffgruppen gemischt und gemeinsam ausgebracht werden. Beispielsweise kommen als Mischungspartner Diazin, 4H-3,1-Benzoxazinderivate, Benzothiadiazinone, 2,6-Dinitroaniline, N-Phenylcarbamate, Thiocarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Sulfonylharnstoffderivate, (Hetero)-aryloxy-phenoxypropionsäuren, deren Salze, Ester und Amide und andere in Betracht.

Außerdem kann es von Nutzen sein, die Verbindungen I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Die folgenden Beispiele erläutern die Herstellung der Verbindungen I:

1. Herstellung der Verbindungen Ia und Ib

N-tert.-Butyl-3-isopropyl-isoxazol-4,5-dicarboximid

Zu 5,1 g (20 mmol) 5-tert.-Butylaminocarbonyl-3-isopropyl-isoxazol-4-carbonsäure in 200 ml Dichlormethan tropfte man bei -5 °C nacheinander 7,5 g (74,1 mmol) Methylmorpholin, 2,4 g (20 mmol) Dimethylaminopyridin und 17,4 g einer 50 %igen Lösung von Propanphosphonsäureanhydrid (27,3 mmol) in Dichlormethan und erhitzte anschließend 6 h unter Rückfluß. Man zog das Solvens im Vakuum ab, nahm den Rückstand in 300 ml Ethylacetat auf und extrahierte zweimal mit gesättigter Natriumhydrogencarbonatlösung sowie je einmal mit 5 %iger Zitronensäurelösung, gesättigter Natriumcarbonatlösung und gesättigter Natriumchloridlösung. Die organische Phase wurde getrocknet und das Solvens im Vakuum abgezogen. Man erhielt 4,1 g (87 %) N-tert.-Butyl-3-isopropyl-isoxazol-4,5-dicarboximid als Feststoff vom Fp. 60-62 °C (Beispiel Nr. 1.003)
8,5 g (45,3 mmol) Chlormaleinsäure-tert. butylimid in 100 ml trockenem Toluol versetzte man bei 0 °C mit 5,5 g (45,3 mmol) 2-Methyl-propyl-hydroxamylchlorid in 100 ml Toluol. Anschließend tropfte man bei 0 °C 9,6 g (94,9 mmol) Triethylamin zu und rührte 12 h bei Raumtemperatur. Man filtrierte, extrahierte das Filtrat mit 10 %iger HCl, trocknete die organische Phase über Magnesiumsulfat und zog das Solvens im Vakuum ab. Man erhielt 7,9 g (74 %) N-tert.-Butyl-3-isopropyl-isoxazol-4,5-dicarboximid (Physikalische Daten s.o.)

N-tert.-Butyl-3-isopropyl-isothiazol-4,5-dicarboximid

Zu 5,4 g (20 mmol) 5-tert.-Butylaminocarbonyl-3-isopropyl-isothiazol-4-carbonsäure in 200 ml Dichlormethan tropfte bei -5 °C nacheinander 7,5 g (74,1 mmol) Methylmorpholin, 2,4 g (20 mmol) Dimethylaminopyridin und 17,4 g einer 50 %igen Lösung von Propanphosphonsäureanhydrid (27,3 mmol) in Dichlormethan und erhitzte anschließend 6 h unter Rückfluß. Die Aufarbeitung erfolgte wie voranstehend für Beispiel Nr. 1.003 angegeben. Man erhielt 4,8 g (95 %) N-tert.-Butyl-3-isopropyl-isothiazol-4,5-dicarboximid als Feststoff vom Fp. 52-53 °C (Beispiel Nr. 3.001).

N-tert.-Butyl-5-methyl-isoxazol-3,4-dicarboximid

Zu 3,8 g (16,8 mmol) 3-tert.-Butylaminocarbonyl-5-methyl-isoxazol-4-carbonsäure in 150 ml Dichlormethan tropfte man bei -5 °C nacheinander 6,3 g (62,2 mmol) Methylmorpholin, 2,4 g (20 mmol) Dimethylaminopyridin und 14,6 g einer 50 %igen Lösung von Propanphosphonsäureanhydrid (23 mmol) in Dichlormethan und erhitzte anschließend 6 h unter Rückfluß. Die Aufarbeitung erfolgte wie voranstehend angeben. Man erhielt 3,2 g (91 %) N-tert.-Butyl-5-methyl-isoxazol-3,4-dicarboximid als Feststoff vom Fp. 48-50 °C (Beispiel Nr. 2.001).

Analog können darüber hinaus beispielsweise weitere Verbindungen hergestellt werden mit den allgemeinen Strukturen

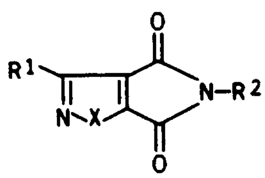

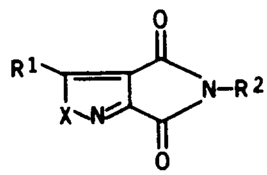

wobei X für Sauerstoff oder Schwefel, und beispielsweise $R^1$ für einen Rest aus der Gruppe $Q_1$ bis $Q_{104}$, $R^2$ für einen Rest aus der Gruppe $M_1$ bis $M_{145}$ stehen und die Reste X, Q und M beliebig kombiniert werden können.

$R^1$ und $R^2$ können beispielsweise die folgenden Reste bedeuten:

| Verb. Nr. | $R^1$ | Verb. Nr. | $R^1$ |
|---|---|---|---|
| $Q_1$ | H | $Q_{42}$ | 1-Methylethoxymethyl |
| $Q_2$ | F | $Q_{43}$ | Ethoxymethyl |
| $Q_3$ | Cl | $Q_{44}$ | Ethenyl |
| $Q_4$ | Methyl | $Q_{45}$ | 1-Propenyl |
| $Q_5$ | Ethyl | $Q_{46}$ | 2-Propenyl |
| $Q_6$ | Propyl | $Q_{47}$ | 1-Methylethenyl |
| $Q_7$ | 1-Methylethyl | $Q_{48}$ | 1-Ethylethenyl |
| $Q_8$ | Butyl | $Q_{49}$ | 2-Phenylethenyl |
| $Q_9$ | 1-Methylpropyl | $Q_{50}$ | 1-Butenyl |
| $Q_{10}$ | 2-Methylpropyl | $Q_{51}$ | 2-Butenyl |
| $Q_{11}$ | 1,1-Dimethylethyl | $Q_{52}$ | 3-Butenyl |
| $Q_{12}$ | Pentyl | $Q_{53}$ | 1-Methyl-1-propenyl |
| $Q_{13}$ | 1-Methylbutyl | $Q_{54}$ | 1-Methyl-2-propenyl |
| $Q_{14}$ | 2-Methylbutyl | $Q_{55}$ | 2-Methyl-2-propenyl |
| $Q_{15}$ | 3-Methylbutyl | $Q_{56}$ | 2-Propinyl |
| $Q_{16}$ | 1,1-Dimethylpropyl | $Q_{57}$ | Methoxy |
| $Q_{17}$ | 1,2-Dimethylpropyl | $Q_{58}$ | Ethoxy |
| $Q_{18}$ | 2,2-Dimethylpropyl | $Q_{59}$ | Propoxy |
| $Q_{19}$ | cyclo-Propyl | $Q_{60}$ | 1-Methylethoxy |
| $Q_{20}$ | cyclo-Butyl | $Q_{61}$ | Butoxy |
| $Q_{21}$ | cyclo-Pentyl | $Q_{62}$ | 1-Methylpropoxy |
| $Q_{22}$ | cyclo-Hexyl | $Q_{63}$ | 1,1-Dimethylethoxy |
| $Q_{23}$ | cyclo-Heptyl | $Q_{64}$ | Methylthio |
| $Q_{24}$ | cyclo-Octyl | $Q_{65}$ | Ethylthio |
| $Q_{25}$ | 1-Methyl-cyclo-Propyl | $Q_{66}$ | Chlordifluormethoxy |
| $Q_{26}$ | cyclo-Propyl-methyl | $Q_{67}$ | Trifluormethoxy |
| $Q_{27}$ | 1-(cyclo-Propyl)-ethyl | $Q_{68}$ | Trichlormethylthio |
| $Q_{28}$ | Chlormethyl | $Q_{69}$ | Phenyl |
| $Q_{29}$ | Dichlormethyl | $Q_{70}$ | 2-Fluorphenyl |
| $Q_{30}$ | Trichlormethyl | $Q_{71}$ | 3-Fluorphenyl |

| Verb. Nr. | $R^1$ | Verb. Nr. | $R^1$ |
|---|---|---|---|
| $Q_{31}$ | Chlordifluormethyl | $Q_{72}$ | 4-Fluorphenyl |
| $Q_{32}$ | Trifluormethyl | $Q_{73}$ | 2-Chlorphenyl |
| $Q_{33}$ | Pentafluorethyl | $Q_{74}$ | 3-Chlorphenyl |
| $Q_{34}$ | Difluormethyl | $Q_{75}$ | 4-Chlorphenyl |
| $Q_{35}$ | 1-Chlorethyl | $Q_{76}$ | 2-Methylphenyl |
| $Q_{36}$ | 2-Chlorethyl | $Q_{77}$ | 3-Methylphenyl |
| $Q_{37}$ | 1-Methyl-1-chlorethyl | $Q_{78}$ | 4-Methylphenyl |
| $Q_{38}$ | 1-Methyl-2-chlorethyl | $Q_{79}$ | 2-Trifluormethylphenyl |
| $Q_{39}$ | Methoxymethyl | $Q_{80}$ | 3-Trifluorphenyl |
| $Q_{40}$ | 1-Methylmethoxymethyl | $Q_{81}$ | 4-Trifluorphenyl |
| $Q_{41}$ | 1-Methyl-2-methoxyethyl | $Q_{82}$ | 2-Methoxyphenyl |

| Verb. Nr. | $R^1$ | Verb. Nr. | $R^2$ |
|---|---|---|---|
| $Q_{83}$ | 3-Methoxyphenyl | $M_1$ | Methyl |
| $Q_{84}$ | 4-Methoxyphenyl | $M_2$ | Ethyl |
| $Q_{85}$ | 2,4-Dichlorphenyl | $M_3$ | Propyl |
| $Q_{86}$ | 2,4,6-Trimethylphenyl | $M_4$ | 1-Methylethyl |
| $Q_{87}$ | Phenoxy | $M_5$ | Butyl |
| $Q_{88}$ | Phenylthio | $M_6$ | 1-Methylpropyl |
| $Q_{89}$ | 2-Chlorphenoxy | $M_7$ | 2-Methylpropyl |
| $Q_{90}$ | 3-Chlorphenoxy | $M_8$ | 1,1-Dimethylethyl |
| $Q_{91}$ | 4-Chlorphenoxy | $M_9$ | Pentyl |
| $Q_{92}$ | 2,4-Dichlorphenoxy | $M_{10}$ | 1-Methylbutyl |
| $Q_{93}$ | Benzyl | $M_{11}$ | 2-Methylbutyl |
| $Q_{94}$ | 2-Chlorbenzyl | $M_{12}$ | 3-Methylbutyl |
| $Q_{95}$ | 3-Chlorbenzyl | $M_{13}$ | 1,1-Dimethylpropyl |
| $Q_{96}$ | 4-Chlorbenzyl | $M_{14}$ | 1,2-Dimethylpropyl |
| $Q_{97}$ | 2-Fluorbenzyl | $M_{15}$ | 2,2-Dimethylpropyl |
| $Q_{98}$ | 3-Fluorbenzyl | $M_{16}$ | 1-Ethylpropyl |
| $Q_{99}$ | 4-Fluorbenzyl | $M_{17}$ | Hexyl |
| $Q_{100}$ | 2-Thienyl | $M_{18}$ | 1-Methylpentyl |
| $Q_{101}$ | 3-Thienyl | $M_{19}$ | 2-Methylpentyl |
| $Q_{102}$ | 2-Pyridyl | $M_{20}$ | 3-Methylpentyl |
| $Q_{103}$ | 3-Pyridyl | $M_{21}$ | 4-Methylpentyl |
| $Q_{104}$ | 4-Pyridyl | $M_{22}$ | 1,1-Dimethylbutyl |
| | | $M_{23}$ | 1,2-Dimethylbutyl |
| | | $M_{24}$ | 1,3-Dimethylbutyl |
| | | $M_{25}$ | 2,2-Dimethylbutyl |
| | | $M_{26}$ | 2,3-Dimethylbutyl |
| | | $M_{27}$ | 3,3-Dimethylbutyl |
| | | $M_{28}$ | 1-Ethylbutyl |
| | | $M_{29}$ | 2-Ethylbutyl |
| | | $M_{30}$ | 1,1,2-Trimethylpropyl |
| | | $M_{31}$ | 1,2,2-Trimethylpropyl |
| | | $M_{32}$ | 1-Ethyl-1-methylpropyl |
| | | $M_{33}$ | 1-Ethyl-2-methylpropyl |
| | | $M_{34}$ | cyclo-Propyl |
| | | $M_{35}$ | cyclo-Butyl |
| | | $M_{36}$ | cyclo-Pentyl |
| | | $M_{37}$ | cyclo-Hexyl |
| | | $M_{38}$ | cyclo-Heptyl |
| | | $M_{39}$ | cyclo-Octyl |
| | | $M_{40}$ | 1-Methyl-cyclo-Propyl |

| Verb. Nr. | $R^2$ | Verb. Nr. | $R^2$ |
|---|---|---|---|
| $M_{41}$ | cyclo-Propyl-methyl | $M_{80}$ | 4-Methylphenyl |
| $M_{42}$ | 1-(cyclo-Propyl)-ethyl | $M_{81}$ | 2-Ethylphenyl |
| $M_{43}$ | 1-Methylcyclohexyl | $M_{82}$ | 3-Ethylphenyl |
| $M_{44}$ | 1-Ethylcyclohexyl | $M_{83}$ | 4-Ethylphenyl |
| $M_{45}$ | Cyclohexylmethyl | $M_{84}$ | 2,3-Dimethylphenyl |
| $M_{46}$ | 2-Propenyl | $M_{85}$ | 2,4-Dimethylphenyl |
| $M_{47}$ | 1-Methyl-2-propenyl | $M_{86}$ | 2,5-Dimethylphenyl |
| $M_{48}$ | 1,1-Dimethyl-2-propenyl | $M_{87}$ | 2,6-Dimethylphenyl |
| $M_{49}$ | 2-Propinyl | $M_{88}$ | 3,4-Dimethylphenyl |
| $M_{50}$ | 1-Methyl-2-propinyl | $M_{89}$ | 3,5-Dimethylphenyl |
| $M_{51}$ | 1,1-Dimethylpropinyl | $M_{90}$ | 2,3,4-Trimethylphenyl |
| $M_{52}$ | Phenylmethyl | $M_{91}$ | 2,3,5-Trimethylphenyl |
| $M_{53}$ | 1-Methylphenylmethyl | $M_{92}$ | 2,4,5-Trimethylphenyl |
| $M_{54}$ | 1,1-Dimethylphenylmethyl | $M_{93}$ | 2,4,6-Trimethylphenyl |
| $M_{55}$ | 2-Phenylethyl | $M_{94}$ | 3,4,5-Trimethylphenyl |
| $M_{56}$ | 2-Methylthioethyl | $M_{95}$ | 2-Trifluormethylphenyl |
| $M_{57}$ | 1-Methyl-2-methylthioethyl | $M_{96}$ | 3-Trifluormethylphenyl |
| $M_{58}$ | 1,1-Dimethyl-2-methylthioethyl | $M_{97}$ | 4-Trifluormethylphenyl |
| $M_{59}$ | 3-Methylthiopropyl | $M_{98}$ | 2-Fluorphenyl |
| $M_{60}$ | 2-Fluorethyl | $M_{99}$ | 3-Fluorphenyl |
| $M_{61}$ | 2-Fluor-1-methylethyl | $M_{88}$ | 3,4-Dimethylphenyl |
| $M_{62}$ | 1,1-Dimethyl-2-fluorethyl | $M_{89}$ | 3,5-Dimethylphenyl |
| $M_{63}$ | 2-Chlorethyl | $M_{90}$ | 2,3,4-Trimethylphenyl |

| Verb. Nr. | $R^2$ | Verb. Nr. | $R^2$ |
|---|---|---|---|
| $M_{64}$ | 2-Chlor-1-methylethyl | $M_{91}$ | 2,3,5-Trimethylphenyl |
| $M_{65}$ | 2-Chlor-1,1-dimethylethyl | $M_{92}$ | 2,4,5-Trimethylphenyl |
| $M_{66}$ | 2-Methoxyethyl | $M_{93}$ | 2,4,6-Trimethylphenyl |
| $M_{67}$ | 2-Methoxy-1-methylethyl | $M_{94}$ | 3,4,5-Trimethylphenyl |
| $M_{68}$ | 1,1-Dimethyl-2-methoxyethyl | $M_{95}$ | 2-Trifluormethylphenyl |
| $M_{69}$ | 3-Methoxypropyl | $M_{96}$ | 3-Trifluormethylphenyl |
| $M_{70}$ | 2-Cyanoethyl | $M_{97}$ | 4-Trifluormethylphenyl |
| $M_{71}$ | 2-Cyano-1-methylethyl | $M_{98}$ | 2-Fluorphenyl |
| $M_{72}$ | 2-Cyano-1,1-dimethylethyl | $M_{99}$ | 3-Fluorphenyl |
| $M_{73}$ | Dimethylamino | $M_{100}$ | 4-Fluorphenyl |
| $M_{74}$ | Diethylamino | $M_{101}$ | 2-Chlorphenyl |
| $M_{75}$ | Morpholino | $M_{102}$ | 3-Chlorphenyl |
| $M_{76}$ | Piperidino | $M_{103}$ | 4-Chlorphenyl |
| $M_{77}$ | Phenyl | $M_{104}$ | 2,3-Difluorphenyl |
| $M_{78}$ | 2-Methylphenyl | $M_{105}$ | 2,4-Difluorphenyl |
| $M_{79}$ | 3-Methylphenyl | $M_{106}$ | 2,5-Difluorphenyl |

| Verb. Nr. | $R^2$ |
|---|---|
| $M_{107}$ | 2,6-Difluorphenyl |
| $M_{108}$ | 2,3-Dichlorphenyl |
| $M_{109}$ | 2,4-Dichlorphenyl |
| $M_{110}$ | 2,5-Dichlorphenyl |
| $M_{111}$ | 2,6-Dichlorphenyl |
| $M_{112}$ | 2,3,4-Trichlorphenyl |
| $M_{113}$ | 2,3,5-Trichlorphenyl |
| $M_{114}$ | 2,4,6-Trichlorphenyl |
| $M_{115}$ | 3,4,5-Trichlorphenyl |
| $M_{116}$ | 2-Cyanophenyl |
| $M_{117}$ | 3-Cyanophenyl |
| $M_{118}$ | 4-Cyanophenyl |
| $M_{119}$ | 2-Methoxyphenyl |
| $M_{120}$ | 3-Methoxyphenyl |
| $M_{121}$ | 4-Methoxyphenyl |
| $M_{122}$ | 2,3-Dimethoxyphenyl |
| $M_{123}$ | 2,4-Dimethoxyphenyl |
| $M_{124}$ | 2,5-Dimethoxyphenyl |
| $M_{125}$ | 2,6-Dimethoxyphenyl |
| $M_{126}$ | 3,4-Dimethoxyphenyl |
| $M_{127}$ | 3,5-Dimethoxyphenyl |
| $M_{128}$ | 3,4,5-Trimethoxyphenyl |
| $M_{129}$ | 2-Trifluormethoxyphenyl |
| $M_{130}$ | 3-Trifluormethoxyphenyl |
| $M_{131}$ | 4-Trifluormethoxyphenyl |
| $M_{132}$ | 2-Nitrophenyl |
| $M_{133}$ | 3-Nitrophenyl |
| $M_{134}$ | 4-Nitrophenyl |
| $M_{135}$ | 2,3-Dinitrophenyl |
| $M_{136}$ | 2,4-Dinitrophenyl |
| $M_{137}$ | 2,5-Dinitrophenyl |
| $M_{138}$ | 2,6-Dinitrophenyl |
| $M_{139}$ | 3,4-Dinitrophenyl |
| $M_{140}$ | 3,5-Dinitrophenyl |
| $M_{141}$ | 1-Naphthyl |
| $M_{142}$ | 2-Naphthyl |
| $M_{143}$ | 3-Tetrahydrofuryl |
| $M_{144}$ | 4-Tetrahydropyranyl |
| $M_{145}$ | 2-Thiazolyl |

Tabelle 1

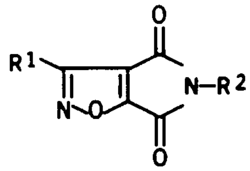

| Bsp. Nr. | $R^1$ | $R^2$ | physik. Daten Fp.[°C], $^1H$-NMR (250 MHz, $CDCl_3$) [δ in ppm] |
|---|---|---|---|
| 1.001 | Methyl | tert.-Butyl | 48-50 |
| 1.002 | Ethyl | cyclo-Propyl | 58-60 |
| 1.003 | iso-Propyl | tert.-Butyl | 60-62 |
| 1.004 | n-Propyl | tert.-Butyl | 1.00(t;3H), 1.63(s;9H), 1.84(m;2H), 2.76(t;2H) |
| 1.005 | cyclo-Propyl | tert.-Butyl | 35-38 |
| 1.006 | iso-Propyl | cyclo-Propyl | 0.82-1.10(m;4H), 3.24(sept; 1H) |

| Bsp. Nr. | $R^1$ | $R^2$ | physik. Daten Fp. [°C], $^1H$-NMR (250 MHz, $CDCl_3$) [δ in ppm] |
|---|---|---|---|
| 1.007 | $CH_2=C(C_2H_5)-$ | cyclo-Propyl | 95-96 |
| 1.008 | $CH_3-CH(Cl)-$ | cyclo-Propyl | 0.90-1.08(m;4H), 1.97(d;3H), 2.62(m;1H), 5.20(q;1H) |
| 1.009 | cyclo-Propyl | cyclo-Propyl | 89-90 |
| 1.010 | iso-Propyl | 4-Chlorphenyl | 80-82 |

Tabelle 2

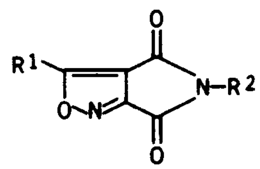

| Bsp. Nr. | $R^1$ | $R^2$ | physik. Daten Fp. [°C], $^1H$-NMR (250 MHz, $CDCl_3$) [δ in ppm] |
|---|---|---|---|
| 2.001 | Methyl | tert.-Butyl | 48-50 |
| 2.002 | n-Propyl | tert.-Butyl | 48-49 |
| 2.003 | iso-Propyl | tert.-Butyl | 35-36 |
| 2.004 | cyclo-Propyl | cyclo-Propyl-methyl | 42-46 |
| 2.005 | cyclo-Propyl | Benzyl | 72-77 |
| 2.006 | cyclo-Propyl | $-CH_2-CH_2-O-CH_3$ | 1.00-1.45 (m;4 H); 2.32 (m; 1H), 3,34 (s; 3H), 3,60 (t; 2H) 3,84 (t; 2H) |
| 2.007 | Methyl | cyclo-Butyl | 55-60 |
| 2.008 | Methyl | $-C(CH_3)_2-C_2H_5$ | 0,86 (t; 3H), 1,63 (s; 6H), 1,97 (q; 2H), 2,66 (s; 3H) |
| 2.009 | Methyl | $-CH_2-C(CH_3)_3$ | 84-91 |

| Bsp. Nr. | $R^1$ | $R^2$ | physik. Daten Fp.[°C], $^1H$-NMR (250 MHz, $CDCl_3$) [δ in ppm] |
|---|---|---|---|
| 2.010 | Methyl | sek.-Butyl | 0,88 (t; 3H),<br>1,34 (d; 3H)<br>1,65-2.10 (m; 2H)<br>2,68 (s; 3H)<br>4,20 (m; 1H) |
| 2.011 | Methyl | cyclo-Pentyl | 71-78 |
| 2.012 | iso-Propyl | cyclo-Propyl | 70-73 |

Tabelle 3

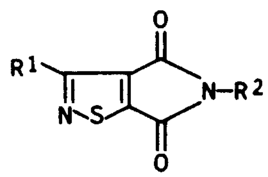

| Bsp. Nr. | $R^1$ | $R^2$ | physik. Daten Fp.[°C] |
|---|---|---|---|
| 3.001 | iso-Propyl | tert.-Butyl | 52- 53 |
| 3.002 | iso-Propyl | 4-Cl-Phenyl | 99-100 |
| 3.003 | Ethyl | 4-Cl-Phenyl | 121-123 |
| 3.004 | Phenyl | 4-Cl-Phenyl | 146-147 |

2. Herstellung von Vorprodukten für die Verbindungen Ic.

4(5)-t-Butylamino-carbonyl-2-methylthiazol-5(4)-carbonsäure
4(5)-Ethoxycarbonyl-2-methylthiazol-5(4)-carbonsäure

Eine Lösung von 51.8 g (0.213 mol) 2-Methylthiazol-4,5-dicarbonsäurediethylester in 300 ml Ethanol wurde bei 0 °C tropfenweise mit einer Lösung von 8.5 g (0.213 mol) Natriumhydroxid in 100 ml Wasser versetzt. Man ließ 12 Stunden bei Raumtemperatur rühren, entfernte dann das Lösungsmittel im Vakuum, nahm den Rückstand in 100 ml Ether und 200 ml Wasser auf, trennte die Phasen und säuerte die wäßrige Phase mit konz. HCl auf pH 1 an. Das ölig ausfallende Produkt wurde durch Extraktion mit $CH_2Cl_2$ isoliert. Ausbeute: 34.8 g (76 %) als 3:2-Isomerengemisch.

4-Ethoxycarbonyl-2-methylthiazol-5-carbonsäure (Hauptkomponente)

$^1H$-NMR ($CDCl_3$, 250 MHz) δ = 1.50 (t, J = 7 Hz; 3 H), 2.80 (s; 3 H), 4.62 (q, J = 7 Hz; 2 H), 13.50 (s, 1H).

5-Ethoxycarbonyl-2-methylthiazol-4-carbonsäure (Nebenkomponente)

$^1H$-NMR ($CDCl_3$, 250 MHz) δ = 1.52 (t, J = 7 Hz; 3 H), 2.82 (s; 3 H), 4.53 (q, 2 H), 13.50 (s, 1 H).

4(5)-t-Butylaminocarbonyl-2-methylthiazol-5(4)-carbonsäureethyl-ester

Eine Lösung von 8 g (37.2 mmol) 4(5)-Ethoxycarbonyl-2-methylthiazol-5(4)-carbonsäure in 50 ml Toluol wurde mit 3.20 g (40.9 mmol) Pyridin versetzt. Man rührte 30 Minuten nach und tropfte dann 4.70 g (39.8 mmol) Thionylchlorid zu. Die erhaltene Mischung wurde 2 h bei 60 °C gerührt, dann auf 0 °C gekühlt und mit 8 g (110 mmol) t-Butylamin versetzt. Man rührte 12 h bei Raumtemperatur nach, entfernte das Lösungsmittel im Vakuum und nahm den Rückstand mit 100 ml Ether und 50 ml Wasser auf. Man extrahierte nochmals mit 100 ml Ether, trocknete die vereinten Extrakte und entfernte das Lösungsmittel. Als Rückstand bleiben 9.70 g (97 %) Produkt als Kristallbrei. (3:2-Isomerengemisch).

5-t-Butylaminocarbonyl-2-methylthiazol-4-carbonsäureethylester (Hauptkomponente)

$^1$H-NMR ($CDCl_3$, 250 MHz) $\delta$ = 1.45 (t, 3 H), 1.45 (s; 3 H), 2.71 (s; 3 H), 4.50 (q, J = 7 Hz; 2 H), 9.95 (s; 1 H).

4-t-Butylaminocarbonyl-2-methylthiazol-5-carbonsäureethylester (Nebenkomponente)

$^1$H-NMR ($CDCl_3$, 250 MHz) $\delta$ = 1.37 (t, 3 H), 1.45 (s; 9 H), 2.71 (s, 3 H), 4.38 (q, 2H), 7.84 (s; 1H).

4(5)-t-Butylaminocarbonyl-2-methylthiazol-5(4)-carbonsäure

Eine Lösung von 9.70 g (35.9 mmol) 4(5)-t-Butylaminocarbonyl-2-methylthiazol-5(4)-carbonsäureethylester in 100 ml Ethanol wurde mit 1.70 g (43.1 mmol) Natriumhydroxid in 20 ml Wasser versetzt. Man erhitzte zwei Stunden zum Rückfluß, entfernte das Lösungsmittel dann im Vakuum und nahm den Rückstand mit 100 ml Wasser und 50 ml Ether auf. Die Phasen wurden getrennt, die wäßrige Phase mit 10%iger Salzsäure auf pH 1 gebracht und das ausgefallene Produkt (3:2-Isomerengemisch) abgesaugt und getrocknet. Ausbeute: 6.60 g (74 %) Fp.: 125-129 °C.

5-t-Butylaminocarbonyl-2-methylthiazol-4-carbonsäure (Hauptkomponente)

$^1$H-NMR ($CDCl_3$, 250 MHz) $\delta$ = 1.45 (s, 9 H), 2.68 (s; 3 H), 8.00 (s; 1 H), 16.75 (s, 1 H).

4-t-Butylaminocarbonyl-2-methylthiazol-5-carbonsäure (Nebenkomponente)

$^1$H-NMR ($CDCl_3$, 250 MHz) $\delta$ = 1.52 (s, 9 H), 2.73 (s; 3 H), 10.00 (s; 1 H), 16.75 (s, 1 H).

4-Cyclopropylaminocarbonyl-2-(1-methylethyl)-oxazol-5-carbonsäure

Zu einer Lösung von 10.4 g (0.054 mol) 2-(1-Methylethyl)-oxazol-4-carbonsäurecyclopropylamid in 250 ml Tetrahydrofuran tropfte man unter Stickstoffatmosphäre bei -70 °C 0.12 mol n-Butyllithium (80.0 ml einer 1.5 molaren Lösung in Hexan) und rührte 30 min bei dieser Temperatur. Anschließend goß man das Reaktionsgemisch auf 500 g festes $CO_2$ und ließ über Nacht stehen. Man engte ein, nahm den Rückstand in 200 ml Wasser und 30 ml 10 %iger NaOH auf, extrahierte zweimal mit Diethylether, säuerte die wäßrige Phase mit konz. Salzsäure auf pH 2 an und extrahierte mit Ethylacetat. Man trocknete über Magnesiumsulfat und zog das Solvens im Vakuum ab. Man erhielt 10.4 g (81 %) 4-Cyclopropylaminocarbonyl-2-(1-methylethyl)-oxazol-5-carbonsäure als weißes Pulver vom Fp. 109 bis 112 °C.

4-tert.-Butylaminocarbonyl-2-methoxythiazol-5-carbonsäure

Zu einer Lösung von 8.00 g (37 mmol) 2-Methoxythiazol-4-carbonsäure-tert.-butylamid in 150 ml Tetrahydrofuran tropfte man bei -70 °C 65 ml einer 1.5 m Lösung (97 mmol) von n-Butyllithium in n-Hexan und rührte 30 Minuten bei dieser Temperatur. Anschließend goß man das Reaktionsgemisch auf 500 g festes Kohlendioxid und ließ innerhalb von 14 Stunden auf Raumtemperatur erwärmen. Man entfernte das Lösungsmittel im Vakuum, nahm den Rückstand in einer Mischung aus 150 ml Wasser und 16 ml 2 m Natronlauge auf, filtrierte, säuerte das Filtrat mit konzentrierter Salzsäure an und saugte die ausgefallene Carbonsäure ab.

Man erhielt 7.8 g (82 %) 4-tert.-Butylaminocarbonyl-2-methoxy-thiazol-5-carbonsäure als weißes Pulver vom Fp.: 120 bis 122 °C.

3. Herstellung der Oxazol- bzw. Thiazol-4,5-dicarbon-säureimide Ic

N-t-Butyl-2-methylthiazol-4,5-dicarbonsäureimid

6.60 g (27.3 mmol) 4(5)-t-Butylaminocarbonyl-2-methylthiazol-5(4)-carbonsäure wurden in 20 ml Pyridin gelöst, mit 6.20 g (32.7 mmol) p-Toluolsulfonsäurechloridversetzt und 12 h bei Raumtemperatur gerührt. Man entfernte das Lösungsmittel im Vakuum, nahm den Rückstand mit $CH_2Cl_2$ und Wasser auf, trennte die Phasen, wusch die organische Phase nacheinander mit 10%iger HCl, gesättigter $NaHCO_3$-Lösung, Wasser und trocknete. Der nach dem Entfernen des Lösungsmittels verbliebene Rückstand wurde durch Filtration über Kieselgel (Elutionsmittel: $CH_2Cl_2$) gereinigt. Ausbeute: 5.20 g (85 %) Fp.: 106 °C (Wirkstoffbeispiel Nr. 4.013).
$^1$H-NMR (250 MHz, $CDCl_3$) $\delta$ = 1.65 (s; 9 H), 2.89 (s; 3 H).

N-(3-Trifluormethylphenyl)-2-(1-methylethyl)thiazol-4,5-dicarbonsäureimid

11.5 g (37 mmol) 4(5)-(3-Trifluormethylanilino)carbonyl-2-(1-methylethyl)thiazol-5(4)-carbonsäure wurde in 100 ml Pyridin gelöst und bei 0 °C mit 8.5 g (44.5 mmol) p-Toluolsulfonsäurechlorid versetzt. Man ließ auf Raumtemperatur erwärmen und erhitzte dann noch zwei Stunden auf 60 °C. Das Lösungsmittel wurde im Vakuum entfernt, der Rückstand in $CH_2Cl_2$ und Wasser aufgenommen, die Phasen getrennt, die wäßrige Phase noch einmal mit $CH_2Cl_2$ extrahiert und die vereinten Extrakte nacheinander mit 10%iger HCl, gesättigter $NaHCO_3$-Lösung und Wasser gewaschen. Man trocknete über $Na_2SO_4$, engte das Gemisch ein und reinigte den verbliebenen Rückstand durch Filtration über Kieselgel (Elutionsmittel: $CH_2Cl_2$). Ausbeute: 10.65 g (84 %) Produkt vom Fp.: 80 °C (Wirkstoffbeispiel Nr. 4.015).
$^1$H-NMR ($CDCl_3$, 250 MHz) $\delta$ = 1.52 (d, 6 H), 3.50 (sept.; 1 H), 7.55-7.75 (m; 3H).

N-t-Butyl-2-methoxythiazol-4,5-dicarbonsäureimid

In eine Lösung von 2.6 g (10 mmol) 4-t-Butylaminocarbonyl-2-methoxythiazol-5-carbonsäure (Beispiel 1.3) in 100 ml $CH_2Cl_2$ gab man bei -5 °C nacheinander 3.75 g (37.1 mmol) N-Methylmorpholin, 1.20 g (10 mmol) 4-N,N-Dimethylaminopyridin und 8.70 g einer 50%igen Lösung (13.7 mmol) von Propanphosphonsäureanhydrid in $CH_2Cl_2$. Man erhitzte 6 h zum Rückfluß, entfernte dann das Lösungsmittel und nahm den Rückstand mit Essigester auf. Man extrahierte zweimal mit gesättigter Natriumhydrogencarbonatlösung sowie einmal mit 5%iger Zitronensäurelösung; wusch mit Wasser und trocknete über $MgSO_4$. Nach Entfernen des Lösungsmittels reinigte man das Rohprodukt durch Chromatographie an Kieselgel (Elutionsmittel: Cyclohexan/Essigester = 1:1) und erhielt so 1.20 g (50 %) Produkt vom Schmelzpunkt 63-66 °C (Wirkstoffbeispiel Nr. 4.014).

Die in den nachfolgenden Tabellen 4 und 5 aufgeführten Verbindungen wurden analog zu den voranstehenden Beispielen hergestellt:

Tabelle 4

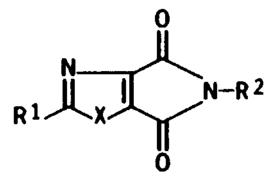

| Nr. | $R^1$ | $R^2$ | X | Fp ($^oC$) oder $^1H$-NMR ($CDCl_3$, 250 MHz) |
|---|---|---|---|---|
| 4.001 | Benzyl | 1,1-Dimethylethyl | S | 72-74 |
| 4.002 | 1-Phenylethyl | 1,1-Dimethylethyl | S | 1.61 (s; 9H), 1.82 (d; 3H), 4.54 (q; 1H), 7.24-7.42 (m; 5H) |
| 4.003 | cyclo-Propyl | 1,1-Dimethylethyl | S | 84 |
| 4.004 | 1-Methoxymethyl | 1,1-Dimethylethyl | S | 51-52 |
| 4.005 | 1,1-Dimethylethyl | 1,1-Dimethylethyl | S | 116 |
| 4.006 | 4-Fluorbenzyl | 1,1-Dimethylethyl | S | 92 |
| 4.007 | 3,4,5-Trimethoxybenzyl | 1,1-Dimethylethyl | S | 123 |
| 4.008 | 2-Phenylethyl | 1,1-Dimethylethyl | S | 83-84 |
| 4.009 | Phenyl | 1,1-Dimethylethyl | S | 115 |
| 4.010 | 1-Methylethyl | 1,1-Dimethylethyl | S | 86 |

Tabelle 4 (Fortsetzung)

| Nr. | $R^1$ | $R^2$ | X | Fp (°C) oder $^1H$-NMR ($CDCl_3$, 250 MHz) |
|---|---|---|---|---|
| 4.011 | H | 1,1-Dimethylethyl | S | 90-91 |
| 4.012 | 4-Phenoxyphenyl | 1,1-Dimethylethyl | S | 141-143 |
| 4.013 | Methyl | 1,1-Dimethylethyl | S | 105-107 |
| 4.014 | Methoxy | 1,1-Dimethylethyl | S | 63-66 |
| 4.015 | 1-Methylethyl | 3-Trifluormethylphenyl | S | 80 |
| 4.016 | Methyl | 3-Trifluormethylphenyl | S | 2.95 (s; 3H), 7.55-7.75 (m; 4H). |
| 4.017 | Methyl | 4-Phenylphenyl | S | >250 |
| 4.018 | 2-Methylpropyl | 1,1-Dimethylethyl | S | 1.00 (d; 6H), 2.20 (nonett; 1H), 1.66 (s; 9H), 3.00 (d; 2H). |
| 4.019 | 1-Methylethyl | Methoxy | S | 70 |
| 4.020 | Methylthiomethyl | 1,1-Dimethylethyl | S | 1.65 (s; 9H), 2.20 (s; 3H), 4.05 (s; 2H). |
| 4.021 | Butyl | 1,1-Dimethylethyl | S | 0.95 (t; 3H), 1.45 (m; 2H), 1.65 (s; 9H), 1.85 (sext; 2H), 3.15 (t; 2H). |

Tabelle 4 (Fortsetzung)

| Nr. | $R^1$ | $R^2$ | X | Fp ($^oC$) oder $^1H$-NMR ($CDCl_3$, 250 MHz) |
|---|---|---|---|---|
| 4.022 | Methyl | Methyl | S | 97-99 |
| 4.023 | Methyl | Cyclopropyl | S | 111 |
| 4.024 | Methyl | 1-Methylethyl | S | 80-81 |
| 4.025 | Propyl | 1,1-Dimethylethyl | S | 42-44 |
| 4.026 | 2-Pyridyl | 1,1-Dimethylethyl | S | 129 |
| 4.027 | Methylsulfonylmethyl | 1,1-Dimethylethyl | S | 113-115 |
| 4.028 | Methyl | 1-Methyl-2-thiomethylpropyl | S | 1.75 (s; 6H), 210 (s; 3H), 2.90 (s; 3H), 3.15 (s; 2H) |
| 4.029 | Methyl | 2,2,2-Trifluorethyl | S | 147 |
| 4.030 | Methyl | 2-Methoxy-1-methylethyl | S | 1.40 (d; 3H), 2,90 (s; 3H), 3.30 (s; 3H) 3.50 u. 3.90 (ABX-System; 2H), 4.40-4.60 (m; 1H) |
| 4.031 | Methyl | 4-Chlor-phenyl | S | 182 |
| 4.032 | Methyl | Dimethylamino | S | 48 |
| 4.033 | Methyl | 1,1-Dimethyl-2-propinyl | S | 46 |
| 4.034 | Methyl | Cyclopropyl | S | 43-44 |
| 4.035 | Methyl | Benzyl | S | 113 |
| 4.036 | Methyl | Cyclo-Propylmethyl | S | 56 |
| 4.037 | Methyl | 2-Propenyl | S | 86 |

Tabelle 4 (Fortsetzung)

| Nr. | $R^1$ | $R^2$ | X | Fp (°C) oder $^1H$-NMR ($CDCl_3$, 250 MHz) |
|---|---|---|---|---|
| 4.038 | Methyl | 1-Phenylethyl | S | 91 |
| 4.039 | Methyl | 4-Chlor-3-(-2-chlor-4-trifluormethyl-phenoxy)-phenyl | S | 2.95 (s; 3H), 6.95-7.80 (m, 6H) |
| 4.040 | Methyl | 4-Chlor-2-methyl-phenyl | S | 2.20 (s; 3H), 2.90 (s; 3H), 7.00-7.40 (m; 3H) |
| 4.041 | Methyl | 2-Methyl-1-(2-propyl)-propyl | S | 0.95 (d; 6H), 1.00 (d; 6H), 2.40-2.60 (m; 2H) 2.90 (s; 3H), 3,70 (t; 1H) |
| 4.042 | Methyl | 4-Phenyl-cyclohexyl | S | 179 |
| 4.043 | Cyclopropyl | Cyclopropyl | S | 100-101 |
| 4.044 | Methyl | 2-(iso-Propyl)-phenyl | S | 131-133 |
| 4.045 | Methyl | 4-Methoxy-phenyl | S | 210-212 |
| 4.046 | Methyl | 2,5-Dimethoxy-phenyl | S | 147-150 |
| 4.047 | Methyl | 2,4,6-Trimethylphenyl | S | 133-134 |
| 4.048 | Methyl | 3-Methoxyphenyl | S | 117-119 |
| 4.049 | Methyl | 2,4-Difluorphenyl | S | 152-154 |
| 4.050 | Methyl | 4-Trifluormethylphenyl | S | 221-223 |
| 4.051 | Methyl | 3-Trifluormethoxyphenyl | S | 107-109 |
| 4.052 | Methyl | 2-Benzothiazolyl | S | >220 |
| 4.053 | Methyl | Cyclohexylmethyl | S | 94 |
| 4.054 | Methyl | Cyclohexyl | S | 173 |

Tabelle 4 (Fortsetzung)

| Nr. | $R^1$ | $R^2$ | X | Fp (°C) oder $^1$H-NMR ($CDCl_3$, 250 MHz) |
|---|---|---|---|---|
| 4.055 | Methyl | 2-(1,3,4-Thiadiazolyl) | S | 177-178 |
| 4.056 | Methyl | 1-Indanyl | S | 106-108 |
| 4.057 | Methyl | 3-Sulfolanyl | S | 197-198 |
| 4.058 | Methyl | 1,1,3,3-Tetramethyl-butyl | S | 1.00 (s; 9H), 1.85 (s; 6H), 2.00 (s; 2H). 2.90 (s; 3H) |
| 4.059 | Methyl | Adamantyl | S | 132-134 |
| 4.060 | Methyl | 4-Thiomethylphenyl | S | >250 |
| 4.061 | Methyl | 8-Chinolinyl | S | 207 |
| 4.062 | Methyl | 2-Cyano-1,1-dimethylethyl | S | 1.85 (s; 6H), 2.90 (s; 3H), 3.10 (s; 2H). |
| 4.063 | Methyl | 3-Tetrahydrofuranyl | S | 73-75 |
| 4.64 | Methyl | 1-Cyano-1-methylethyl | S | 82 |
| 4.065 | Methyl | 2-(1,4-Benzochinonyl) | S | 127 |
| 4.066 | Methyl | 3-Ethyl-pentin-3-yl | S | 1.05 (t; 6H), 1.95 (m; 4H), 2.50 (m; 4H), 2.60 (s; 1H), 2.95 (s; 3H) |
| 4.067 | Methyl | 1-tert.-Butoxy-propan-2-yl | S | 1.10 (s; 9H), 1.45 (d; 3H), 2.90 (s; 3H), 3.65 (m; 1H), 3.80 (dd; 1H), 4.40 (m; 1H). |
| 4.068 | Methyl | 1-Cyclopropyl-ethyl | S | 0.15-0.85 (m; 4H), 1.55 (d; 3H), 1.55-1.65 (m; 1H), 2.95 (s; 3H), 3.25-3.50 (m; 1H). |

Tabelle 4 (Fortsetzung)

| Nr. | $R^1$ | $R^2$ | X | Fp ($^oC$) oder $^1H$-NMR ($CDCl_3$, 250 MHz) |
|---|---|---|---|---|
| 4.069 | Methyl | 2,6-Dimethyl-undeca-2,6-dien-10-yl | S | 1.30-2.30 (m; 20H), 2.90 (s; 3H) 4.25 (m; 1H), 5.05 (m; 2H). |
| 4.070 | Methyl | 2-Methyl-4-phenyl-butan-2-yl | S | 1.70 (s; 6H), 2.20-2.40 (m; 2H) 2.50-2.70 (m; 2H), 2.90 (s; 3H), 6.95-7.25 (m; 5H) |
| 4.071 | Methyl | Cyclododecanyl | S | 90-91 |
| 4.072 | Methyl | 2-Methyl-pentan-2-yl | S | 0.95 (t; 3H), 1.65 (s; 6H), 2.00 (g; 2H), 2.85 (s; 3H). |
| 4.073 | Methyl | 2-Heptanyl | S | 0.85 (t; 3H), 1.15-1.35 (m; 6H), 1.40 (d; 3H), 1.55-1.75 (m; 1H), 1.95-2.10 (m; 1H), 2.90 (s; 3H), 4.15-4.45 (m; 1H) |
| 4.074 | Methyl | Diphenylmethyl | S | 152 |
| 4.075 | Methyl | 4-Chlor-3-trifluormethyl-phenyl | S | 141 |
| 4.076 | Methyl | Bicyclo[3.3.0]octan-1-yl | S | 61 |
| 4.077 | Methyl | 3,4-Dichlorphenyl | S | 174-176 |
| 4.078 | Methyl | 4-Methyl-tetrahydropyran-4-yl | S | 125 |
| 4.079 | Methyl | 2-Methyl-4-Oxo-pentan-2-yl | S | 1.75 (s; 6H), 2.15 (s; 3H), 2.90 (s; 3H), 3.20 (s; 2H). |

Tabelle 4 (Fortsetzung)

| Nr. | $R^1$ | $R^2$ | X | Fp (°C) oder $^1H$-NMR ($CDCl_3$, 250 MHz) |
|---|---|---|---|---|
| 4.080 | Methyl | 1-Chlor-butan-2-yl | S | 0.95 (t; 3H), 1.70-2.15 (m; 2H), 2.95 (s; 3H) 3.75 und 4.10 (ABX-System; 2H), 4.50 (m; 1H). |
| 4.081 | Methyl | Bicyclo[2.2.1]heptan-2-yl | S | 100 |
| 4.082 | Methyl | 6-Methyl-heptan-2-yl | S | 0.80 (d; 3H), 0.85 (d,3H), 1.10-2.10 (m; 7H), 1.45 (d; 3H), 2.40 (s; 3H), 4.25 (m; 1H). |
| 4.083 | Methyl | 3-Methyl-butyn-2-yl | S | 78 |
| 4.084 | Methyl | 6-Methyl-hept-5-en-2-yl | S | 1.45 (d, 3H), 1.55 (s; 3H), 1.60 (s; 3H), 1.65-180 (m; 1H), 1.90-2.15 (m; 3H), 2.90 (s; 3H), 4.20-4.35 (m; 1H), 5.00-5.15 (m; 1H). |
| 4.085 | Methyl | 1,2-Diphenylethyl | S | 134 |
| 4.086 | Methyl | Phenoxy-propan-2-yl | S | 71 |
| 4.087 | Phenyl | 1,1-Dimethylethyl | O | 130-138 |

Anwendungsbeispiele

Die herbizide Wirkung der Dicarbonsäureimide der Formeln Ia, Ib und Ic ließ sich durch Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zwecke der Nachauflaufbehandlung wurden die Testpflanzen je nach Wuchsform erst bei einer Wuchshöhe von 3 bis 15 cm mit den in Wasser suspendierten oder emulgierten Wirkstoffen behandelt. Die Aufwandmenge für die Nachauflaufbehandlung betrug 1 und 2 kg/ha aktive Substanz (a.S.).

Die Pflanzen wurden artenspezifisch bei Temperaturen von 10-25 °C bzw. 20-35 °C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen im Fall der Beispielverbindung 4.003 verwendeten Pflanzen setzten sich aus folgenden Arten zusammen:

| Lateinischer Name | Deutscher Name | Englischer Name |
|---|---|---|
| Chenopodium album | Weißer Gänsefuß | lambsquarters (goosefoot) |
| Stellaria media | Vogelsternmiere | chickweed |
| Veronica spp. | Ehrenpreisarten | speedwell |

Mit 1,0 kg/ha a.S. im Nachauflaufverfahren eingesetzt, lassen sich mit der Beispielsverbindung Nr. 4.003 breitblättrige unerwünschte Pflanzen sehr gut bekämpfen.

Die in den Gewächshausversuchen im Fall der Beispielverbindung 1.001 verwendeten Pflanzen setzten sich aus folgenden Arten zusammen:

| Lateinischer Name | Deutscher Name |
|---|---|
| Chenopodium album | Weißer Gänsefuß |
| Chysanthemum coronarium | Kronenwucherblume |
| Setaria italica | Kolbenhirse |
| Solanum nigrum | Schwarzer Nachtschatten |

Mit 2,0 kg/ha a.S. im Nachauflaufverfahren eingesetzt, lassen sich mit dem Beispiel 1.001 breitblättrige unerwünschte Pflanzen sehr gut bekämpfen.

**Patentansprüche**

**Patentansprüche für folgende Vertragsstaaten : AT, BE, CH, DE, FR, GB, IT, LI, NL**

**1.** Dicarbonsäureimide der Formeln Ia, Ib und Ic

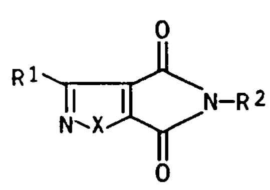

Ia

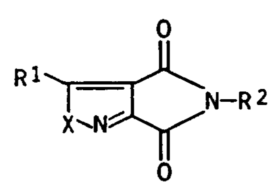

Ib

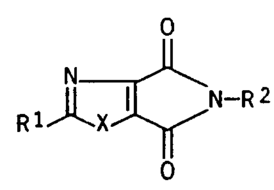

Ic

in der die Substituenten folgende Bedeutung haben:

X

Sauerstoff oder Schwefel;

$R^1$

Wasserstoff; Halogen; Cyano;

$C_1$-$C_6$-Alkyl, welches ein bis fünf Halogenatome und/oder einen oder zwei der folgenden Reste tragen kann: $C_3$-$C_6$-Cycloalkyl, $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Alkylsulfonyl, $C_1$-$C_4$-Halogenalkylsulfonyl oder Cyano;

$C_3$-$C_8$-Cycloalkyl, welches ein bis drei der folgenden Reste tragen kann: $C_1$-$C_4$-Alkyl oder Halogen;

$C_2$-$C_6$-Alkenyl, welches ein bis drei der folgenden Reste tragen kann: Halogen, $C_1$-$C_3$-Alkoxy und/oder ein Phenyl, das seinerseits eine bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Cyano oder Nitro;

$C_2$-$C_6$-Alkinyl, welches ein bis drei der folgenden Reste tragen kann: Halogen oder $C_1$-$C_3$-Alkoxy und/oder ein Phenyl, das seinerseits eine bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Cyano oder Nitro;

$C_1$-$C_4$-Alkoxy; $C_2$-$C_6$-Alkenyloxy; $C_2$-$C_6$-Alkinyloxy; $C_1$-$C_4$-Alkylthio; $C_1$-$C_4$-Halogenalkoxy; $C_1$-$C_4$-Halogenalkylthio, $C_1$-$C_4$-Alkylsulfonyl; $C_1$-$C_4$-Halogenalkylsulfonyl;

Phenoxy oder Phenylthio, welches ein bis drei der folgenden Reste tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Cyano oder Nitro;

ein 5- bis 6-gliedriger gesättigter oder aromatischer heterocyclischer Rest, enthaltend ein oder zwei Heteroatome, ausgewählt aus der Gruppe Sauerstoff, Schwefel und Stickstoff, wobei der Ring ein oder zwei der folgenden Reste tragen kann: $C_1$-$C_3$-Alkyl, Halogen, $C_1$-$C_3$-Alkoxy oder $C_1$-$C_3$-Alkoxycarbonyl;

ein kondensierter aromatischer Rest, ausgewählt aus der Gruppe Benzofuran-2-yl, Benzofuran-3-yl, Isobenzofuran-2-yl, Benzothiophen-2-yl, Benzothiophen-3-yl, Isobenzothiophen-2-yl, Indol-2-yl, Indol-3-yl, 1,2-Benzisoxal-3-yl, Benzoxazol-2-yl, 1,2-Benzisothiazol-3-yl, Benzothiazol-2-yl, Indazol-3-yl, (1H)-Benzimidazhol-2-yl, Chinolin-3-yl, Chinolin-5-yl, Chinolin-6-yl, Chinolin-8-yl, Isochinolin-1-yl und Isochinolin-5-yl, wobei dieser Rest ein oder zwei der folgenden Reste tragen kann: Alkyl wie oben genannt, insbesondere Methyl; Halogen wie oben genannt, insbesondere Fluor und Chlor; Alkoxy wie oben genannt, insbesondere Methoxy und Ethoxy oder Alkoxycarbonyl wie Methoxycarbonyl und Ethoxycarbonyl, insbesondere Methoxycarbonyl;

Phenyl oder Phenyl-$C_1$-$C_4$-alkyl, wobei der Phenylkern jeweils unsubstituiert oder durch ein bis drei der folgenden Gruppen substituiert ist: $C_1$-$C_6$-Alkyl, $C_1$-$C_6$-Halogenalkyl, $C_1$-$C_6$-Alkoxy, $C_1$-$C_6$-Halogenalkoxy, $C_1$-$C_6$-Alkylthio, $C_1$-$C_6$-Halogenalkylthio, Halogen, Nitro und Cyano;

$R^2$

Wasserstoff; Hydroxyl; $C_1$-$C_4$-Alkoxy;

$C_1$-$C_6$-Alkyl, das eine bis drei der folgenden Gruppen tragen kann: Cyano, $C_1$-$C_4$-Alkoxy-$C_1$-$C_4$-alkoxy, $C_1$-$C_4$-Alkoxy, $C_1$-$C_4$-Halogenalkoxy, $C_1$-$C_4$-Alkylthio, $C_1$-$C_4$-Halogenalkylthio, Di-$C_1$-$C_4$-alkylamino, Halogen, $C_3$-$C_8$-Cycloalkyl oder Phenyl, wobei der Phenylring seinerseits ein bis drei der folgenden Reste tragen kann: Halogen, Cyano, Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder $C_1$-$C_4$-Halogenalkylthio;

$C_3$-$C_8$-Cycloalkyl, das eine bis drei der folgenden Gruppen tragen kann: $C_1$-$C_6$-Alkyl, $C_1$-$C_6$-Halogenalkyl, $C_1$-$C_4$-Alkoxy, $C_1$-$C_4$-Halogenalkoxy, Halogen, Nitro oder Cyano;

$C_3$-$C_6$-Alkenyl, das ein- bis dreimal durch Halogen und/oder einmal durch Phenyl substituiert sein kann, wobei der Phenylrest seinerseits eine bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Cyano oder Nitro;

$C_3$-$C_6$-Alkinyl, das ein- bis dreimal durch Halogen und/oder einmal durch Phenyl substituiert sein kann, wobei der Phenylrest seinerseits eine bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Cyano oder Nitro;

Di-$C_1$-$C_4$-alkylamino;

ein 5- bis 6-gliedriger heterocyclischer gesättigter oder aromatischer Rest mit einem oder zwei Heteroatomen, ausgewählt aus der Gruppe Sauerstoff, Schwefel oder Stickstoff, der ein- bis dreimal durch $C_1$-$C_4$-Alkyl oder Halogen substituiert sein kann;

ein kondensierter aromatischer Rest, ausgewählt aus der Gruppe Benzofuran-2-yl, Benzofuran-3-yl, Isobenzofuran-2-yl, Benzothiophen-2-yl, Benzothiophen-3-yl, Isobenzothiophen-2-yl, Indol-2-yl, Indol-3-yl, 1,2-Benzisoxal-3-yl, Benzoxazol-2-yl, 1,2-Benzisothiazol-3-yl, Benzothiazol-2-yl, Indazol-3-yl, (1H)-Benzimidazhol-2-yl, Chinolin-3-yl, Chinolin-5-yl, Chinolin-6-yl, Chinolin-8-yl, Isochinolin-1-yl und Isochinolin-5-yl, wobei dieser Rest ein- bis dreimal durch $C_1$-$C_4$-Alkyl oder Halogen substituiert sein kann;
Phenyl, das eine bis vier der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Nitro, Cyano, Formyl, Phenyl, $C_1$-$C_4$-Alkanoyl, $C_1$-$C_4$-Halogenalkanoyl oder $C_1$-$C_4$-Alkoxycarbonyl;
Naphthyl, das ein- bis dreimal durch $C_1$-$C_4$-Alkyl oder Halogen substituiert sein kann;
sowie pflanzenverträgliche Salze der Dicarbonsäureimide I, ausgenommen 3-Methylisoxazol-4,5-dicarboximid und Thiazol-4,5-dicarbonsäureimide, in denen $R^2$ für Phenyl steht, wenn $R^1$ Methyl oder 2-Thiazolyl bedeutet.

**2.** Verfahren zur Herstellung von Verbindungen der Formel I, dadurch gekennzeichnet, daß man die entsprechenden Dicarbonsäuremonoamide II

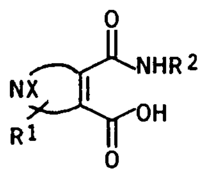

II

in an sich bekannter Weise mit wasserentziehenden Mitteln behandelt.

**3.** Verfahren zur Herstellung der Verbindungen Ia, dadurch gekennzeichnet, daß man ein Hydroxamsäurechlorid der Formel X

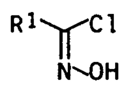

X,

in Gegenwart einer Base mit Halogenmaleinsäureimiden der Formel XI

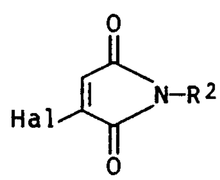

XI,

wobei $R^1$ und $R^2$ die o.g. Bedeutung haben und Hal für Chlor oder Brom steht, umsetzt.

**4.** Verfahren zur Herstellung der Verbindungen Ia, dadurch gekennzeichnet, daß man ein Hydroxamsäurechlorid X gemäß Anspruch 3 in an sich bekannter Weise mit Maleinimiden XIII

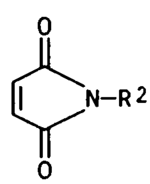

XIII

zu den Isoxazolinimiden XII umsetzt

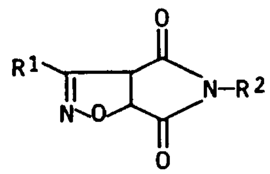

XII

und diese dann mit Oxidationsmitteln dehydriert.

**5.** Herbizides Mittel, enthaltend neben inerten Zusatzstoffen mindestens ein Dicarbonsäureimid der Formeln Ia, Ib oder Ic

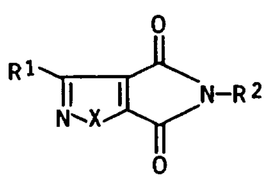

Ia

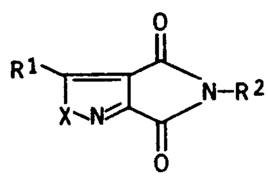

Ib

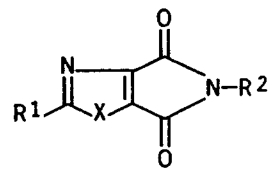

Ic

in der die Substituenten die in Anspruch 1 angegebene Bedeutung haben, einschließlich 3-Methylisoxazol-4,5-dicarboximid und Thiazol-4,5-dicarbonsäureimide, in denen $R^2$ für Phenyl steht, wenn $R^1$ Methyl oder 2-Thiazolyl bedeutet.

**6.** Verfahren zur Bekämpfung unerwünschten Pflanzenwuchses, dadurch gekennzeichnet, daß man die unerwünschten Pflanzen und/oder die von unerwünschten Pflanzenwuchs freizuhaltende Fläche mit einer herbizid wirksamen Menge eines Dicarbonsäureimids der Formeln Ia, Ib oder Ic gemäß Anspruch 5 behandelt.

**Patentansprüche für folgenden Vertragsstaat : ES**

**1.** Verfahren zur Herstellung von Dicarbonsäureimiden der Formeln Ia, Ib und Ic,

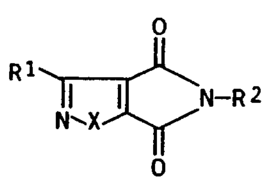

Ia

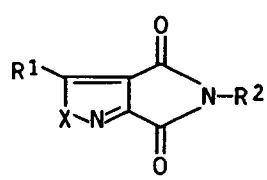

Ib

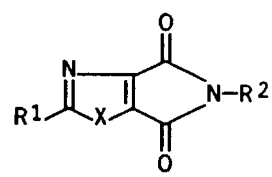

Ic

in der die Substituenten folgende Bedeutung haben:

X

Sauerstoff oder Schwefel;

$R^1$

Wasserstoff; Halogen; Cyano;

$C_1$-$C_6$-Alkyl, welches ein bis fünf Halogenatome und/oder einen oder zwei der folgenden Reste tragen kann: $C_3$-$C_6$-Cycloalkyl, $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Alkylsulfonyl, $C_1$-$C_4$-Halogenalkylsulfonyl oder Cyano;

$C_3$-$C_8$-Cycloalkyl, welches ein bis drei der folgenden Reste tragen kann: $C_1$-$C_4$-Alkyl oder Halogen;

$C_2$-$C_6$-Alkenyl, welches ein bis drei der folgenden Reste tragen kann: Halogen, $C_1$-$C_3$-Alkoxy und/oder ein Phenyl, das seinerseits eine bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Cyano oder Nitro;

$C_2$-$C_6$-Alkinyl, welches ein bis drei der folgenden Reste tragen kann: Halogen oder $C_1$-$C_3$-Alkoxy

und/oder ein Phenyl, das seinerseits eine bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Cyano oder Nitro;

$C_1$-$C_4$-Alkoxy; $C_2$-$C_6$-Alkenyloxy; $C_2$-$C_6$-Alkinyloxy; $C_1$-$C_4$-Alkylthio; $C_1$-$C_4$-Halogenalkoxy; $C_1$-$C_4$-Halogenalkylthio, $C_1$-$C_4$-Alkylsulfonyl; $C_1$-$C_4$-Halogenalkylsulfonyl;

Phenoxy oder Phenylthio, welches ein bis drei der folgenden Reste tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Cyano oder Nitro;

ein 5- bis 6-gliedriger gesättigter oder aromatischer heterocyclischer Rest, enthaltend ein oder zwei Heteroatome, ausgewählt aus der Gruppe Sauerstoff, Schwefel und Stickstoff, wobei der Ring ein oder zwei der folgenden Reste tragen kann: $C_1$-$C_3$-Alkyl, Halogen, $C_1$-$C_3$-Alkoxy oder $C_1$-$C_3$-Alkoxycarbonyl;

ein kondensierter aromatischer Rest, ausgewählt aus der Gruppe Benzofuran-2-yl, Benzofuran-3-yl, Isobenzofuran-2-yl, Benzothiophen-2-yl, Benzothiophen-3-yl, Isobenzothiophen-2-yl, Indol-2-yl, Indol-3-yl, 1,2-Benzisoxal-3-yl, Benzoxazol-2-yl, 1,2-Benzisothiazol-3-yl, Benzothiazol-2-yl, Indazol-3-yl, (1H)-Benzimidazhol-2-yl, Chinolin-3-yl, Chinolin-5-yl, Chinolin-6-yl, Chinolin-8-yl, Isochinolin-1-yl und Isochinolin-5-yl, wobei dieser Rest ein oder zwei der folgenden Reste tragen kann: Alkyl wie oben genannt, insbesondere Methyl; Halogen wie oben genannt, insbesondere Fluor und Chlor; Alkoxy wie oben genannt, insbesondere Methoxy und Ethoxy oder Alkoxycarbonyl wie Methoxycarbonyl und Ethoxycarbonyl, insbesondere Methoxycarbonyl;

Phenyl oder Phenyl-$C_1$-$C_4$-alkyl, wobei der Phenylkern jeweils unsubstituiert oder durch ein bis drei der folgenden Gruppen substituiert ist: $C_1$-$C_6$-Alkyl, $C_1$-$C_6$-Halogenalkyl, $C_1$-$C_6$-Alkoxy, $C_1$-$C_6$-Halogenalkoxy, $C_1$-$C_6$-Alkylthio, $C_1$-$C_6$-Halogenalkylthio, Halogen, Nitro und Cyano;

$R^2$

Wasserstoff; Hydroxyl; $C_1$-$C_4$-Alkoxy;

$C_1$-$C_6$-Alkyl, das eine bis drei der folgenden Gruppen tragen kann: Cyano, $C_1$-$C_4$-Alkoxy-$C_1$-$C_4$-alkoxy, $C_1$-$C_4$-Alkoxy, $C_1$-$C_4$-Halogenalkoxy, $C_1$-$C_4$-Alkylthio, $C_1$-$C_4$-Halogenalkylthio, Di-$C_1$-$C_4$-alkylamino, Halogen, $C_3$-$C_8$-Cycloalkyl oder Phenyl, wobei der Phenylring seinerseits ein bis drei der folgenden Reste tragen kann: Halogen, Cyano, Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder $C_1$-$C_4$-Halogenalkylthio;

$C_3$-$C_8$-Cycloalkyl, das eine bis drei der folgenden Gruppen tragen kann: $C_1$-$C_6$-Alkyl, $C_1$-$C_6$-Halogenalkyl, $C_1$-$C_4$-Alkoxy, $C_1$-$C_4$-Halogenalkoxy, Halogen, Nitro oder Cyano;

$C_3$-$C_6$-Alkenyl, das ein- bis dreimal durch Halogen und/oder einmal durch Phenyl substituiert sein kann, wobei der Phenylrest seinerseits eine bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Cyano oder Nitro;

$C_3$-$C_6$-Alkinyl, das ein- bis dreimal durch Halogen und/oder einmal durch Phenyl substituiert sein kann, wobei der Phenylrest seinerseits eine bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Cyano oder Nitro;

Di-$C_1$-$C_4$-alkylamino;

ein 5- bis 6-gliedriger heterocyclischer gesättigter oder aromatischer Rest mit einem oder zwei Heteroatomen, ausgewählt aus der Gruppe Sauerstoff, Schwefel oder Stickstoff, der ein- bis dreimal durch $C_1$-$C_4$-Alkyl oder Halogen substituiert sein kann;

ein kondensierter aromatischer Rest, ausgewählt aus der Gruppe Benzofuran-2-yl, Benzofuran-3-yl, Isobenzofuran-2-yl, Benzothiophen-2-yl, Benzothiophen-3-yl, Isobenzothiophen-2-yl, Indol-2-yl, Indol-3-yl, 1,2-Benzisoxal-3-yl, Benzoxazol-2-yl, 1,2-Benzisothiazol-3-yl, Benzothiazol-2-yl, Indazol-3-yl, (1H)-Benzimidazhol-2-yl, Chinolin-3-yl, Chinolin-5-yl, Chinolin-6-yl, Chinolin-8-yl, Isochinolin-1-yl und Isochinolin-5-yl, wobei dieser Rest ein- bis dreimal durch $C_1$-$C_4$-Alkyl oder Halogen substituiert sein kann;

Phenyl, das eine bis vier der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Nitro, Cyano, Formyl, Phenyl, $C_1$-$C_4$-Alkanoyl, $C_1$-$C_4$-Halogenalkanoyl oder $C_1$-$C_4$-Alkoxycarbonyl;

Naphthyl, das ein- bis dreimal durch $C_1$-$C_4$-Alkyl oder Halogen substituiert sein kann;

ausgenommen 3-Methylisoxazol-4,5-dicarboximid und Thiazol-4,5-dicarbonsäureimide, in denen $R^2$ für Phenyl steht, wenn $R^1$ Methyl oder 2-Thiazolyl bedeutet, dadurch gekennzeichnet, daß man die entsprechenden Dicarbonsäuremonoamide II

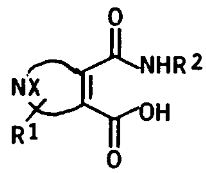

II

in an sich bekannter Weise mit wasserentziehenden Mitteln behandelt.

2. Verfahren zur Herstellung der Verbindungen Ia, dadurch gekennzeichnet, daß man ein Hydroxamsäurechlorid der Formel X

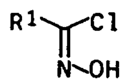

X,

in Gegenwart einer Base mit Halogenmaleinsäureimiden der Formel XI

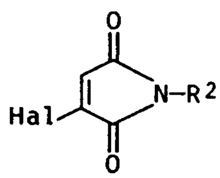

XI,

wobei $R^1$ und $R^2$ die o.g. Bedeutung haben und Hal für Chlor oder Brom steht, umsetzt.

3. Verfahren zur Herstellung der Verbindungen Ia, dadurch gekennzeichnet, daß man ein Hydroxamsäurechlorid X gemäß Anspruch 3 in an sich bekannter Weise mit Maleinimiden XIII zu den Isoxazolinimiden XII umsetzt

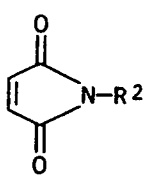

XIII

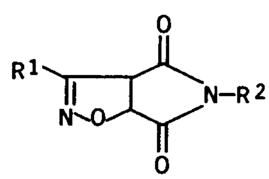

XII

und diese dann mit Oxidationsmitteln dehydriert.

4. Herbizides Mittel, enthaltend neben inerten Zusatzstoffen mindestens ein Dicarbonsäureimid der Formeln Ia, Ib oder Ic

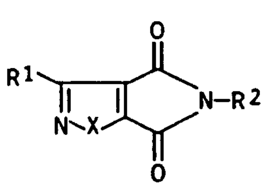

Ia

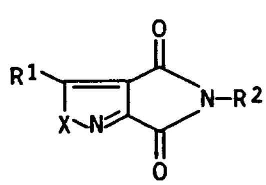

Ib

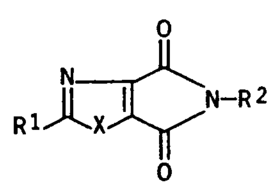

Ic

in der die Substituenten die in Anspruch 1 angegebene Bedeutung haben, einschließlich 3-Methylisoxazol-4,5-dicarboximid und Thiazol-4,5-dicarbonsäureimide, in denen $R^2$ für Phenyl steht, wenn $R^1$ Methyl oder 2-Thiazolyl bedeutet.

**5.** Verfahren zur Bekämpfung unerwünschten Pflanzenwuchses, dadurch gekennzeichnet, daß man die unerwünschten Pflanzen und/oder die von unerwünschten Pflanzenwuchs freizuhaltende Fläche mit einer herbizid wirksamen Menge eines Dicarbonsäureimids der Formeln Ia, Ib oder Ic gemäß Anspruch 4 behandelt.

**Claims**

**Claims for the following Contracting States : AT, BE, CH, DE, FR, GB, IT, LI, NL**

**1.** A dicarboximide of the formula Ia, Ib or Ic

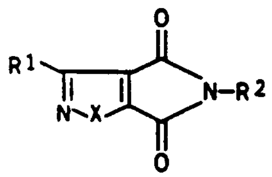

Ia

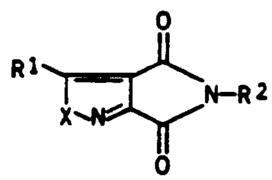

Ib

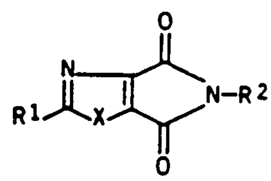

Ic

where
X is oxygen or sulfur,
$R^1$ is hydrogen, halogen, cyano,
$C_1$-$C_6$-alkyl which may carry from one to five halogen atoms and/or one or two of the following radicals: $C_3$-$C_6$-cycloalkyl, $C_1$-$C_4$-alkoxy, $C_1$-$C_4$-haloalkoxy, $C_1$-$C_4$-alkylthio, $C_1$-$C_4$-haloalkylthio, $C_1$-$C_4$-alkylsulfonyl, $C_1$-$C_4$-haloalkylsulfonyl or cyano,
$C_3$-$C_8$-cycloalkyl which may carry from one to three of the following radicals: $C_1$-$C_4$-alkyl or halogen,
$C_2$-$C_6$-alkenyl which may carry from one to three of the following radicals: halogen, $C_1$-$C_3$-alkoxy and/or a phenyl radical which in turn may carry from one to three of the following groups: $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, halogen, cyano or nitro,
$C_2$-$C_6$-alkynyl which may carry from one to three of the following radicals: halogen, $C_1$-$C_3$-alkoxy and/or a phenyl radical which in turn may carry from one to three of the following groups: $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, halogen, cyano or nitro,
$C_1$-$C_4$-alkoxy, $C_2$-$C_6$-alkenyloxy, $C_2$-$C_6$-alkynyloxy, $C_1$-$C_4$-alkylthio, $C_1$-$C_4$-haloalkoxy, $C_1$-$C_4$-haloalkylthio, $C_1$-$C_4$-alkylsulfonyl or $C_1$-$C_4$-haloalkylsulfonyl,
phenoxy or phenylthio which may carry from one to three of the following radicals: $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, halogen, cyano or nitro,
a 5-membered or 6-membered saturated or aromatic heterocyclic radical containing one or two hetero atoms selected from the group consisting of oxygen, sulfur and nitrogen, where the ring may carry one or two of the following radicals: $C_1$-$C_3$-alkyl, halogen, $C_1$-$C_3$-alkoxy or $C_1$-$C_3$-alkoxycarbonyl,
a fused aromatic radical selected from the group consisting of benzofuran-2-yl, benzofuran-3-yl, isobenzofuran-2-yl, benzothiophen-2-yl, benzothiophen-3-yl, isobenzothiophen-2-yl, indol-2-yl, indol-3-yl, 1,2-benzoisoxal-3-yl, benzoxazol-2-yl, 1,2-benzoisothiazol-3-yl, benzothiazol-2-yl, indazol-3-yl, (1H)-benzimidazol-2-yl, quinol-3-yl, quinol-5-yl, quinol-6-yl, quinol-8-yl, isoquinol-1-yl and isoquinol-5-yl, where this radical may carry one or two of the following radicals: alkyl as stated above, in particular methyl, halogen as stated above, in particular fluorine or chlorine, alkoxy as stated above, in particular methoxy or ethoxy, or alkoxycarbonyl, such as methoxycarbonyl or ethoxycarbonyl, in particular methoxycarbonyl, or
phenyl or phenyl-$C_1$-$C_4$-alkyl where the phenyl nucleus in each case is unsubstituted or substituted by from one to three of the following groups: $C_1$-$C_6$-alkyl, $C_1$-$C_6$-haloalkyl, $C_1$-$C_6$-alkoxy, $C_1$-$C_6$-haloalkoxy, $C_1$-$C_6$-alkylthio, $C_1$-$C_6$-haloalkylthio, halogen, nitro or cyano,
$R^2$ is hydrogen, hydroxyl, $C_1$-$C_4$-alkoxy, $C_1$-$C_6$-alkyl which may carry from one to three of the following groups: cyano, $C_1$-$C_4$-alkoxy-$C_1$-$C_4$-alkoxy, $C_1$-$C_4$-alkoxy, $C_1$-$C_4$-haloalkoxy, $C_1$-$C_4$-alkylthio, $C_1$-$C_4$-haloalkylthio, di-$C_1$-$C_4$-alkylamino, halogen, $C_3$-$C_8$-cycloalkyl or phenyl, where the phenyl ring in turn may carry from one to three of the following radicals: halogen, cyano, nitro, $C_1$-$C_4$-alkyl,$C_1$-$C_4$-haloalkyl, $C_1$-$C_4$-alkoxy, $C_1$-$C_4$-haloalkoxy, $C_1$-$C_4$-alkylthio or $C_1$-$C_4$-haloalkylthio,

$C_3$-$C_8$-cycloalkyl which may carry from one to three of the following groups: $C_1$-$C_6$-alkyl, $C_1$-$C_6$-haloalkyl, $C_1$-$C_4$-alkoxy, $C_1$-$C_4$-haloalkoxy, halogen, nitro or cyano,
$C_3$-$C_6$-alkenyl which may be monosubstituted to trisubstituted by halogen and/or monosubstituted by phenyl, where the phenyl radical in turn may carry from one to three of the following groups: $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, halogen, cyano or nitro,
$C_3$-$C_6$-alkynyl which may be monosubstituted to trisubstituted by halogen and/or monosubstituted by phenyl, where the phenyl radical in turn may carry from one to three of the following groups: $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, halogen, cyano or nitro,
di-$C_1$-$C_4$-alkylamino,
a 5-membered or 6-membered heterocyclic saturated or aromatic radical which has one or two hetero atoms selected from the group consisting of oxygen, sulfur and nitrogen and which may be monosubstituted to trisubstituted by $C_1$-$C_4$-alkyl or halogen,
a fused aromatic radical selected from the group consisting of benzofuran-2-yl, benzofuran-3-yl, isobenzofuran-2-yl, benzothiophen-2-yl, benzothiophen-3-yl, isobenzothiophen-2-yl, indol-2-yl, indol-3-yl, 1,2-benzoisoxal-3-yl, benzoxazol-2-yl, 1,2-benzoisothiazol-3-yl, benzothiazol-2-yl, indazol-3-yl, (1H)-benzimidazol-2-yl, quinol-3-yl, quinol-5-yl, quinol-6-yl, quinol-8-yl, isoquinol-1-yl and isoquinol-5-yl, where this radical may be monosubstituted to trisubstituted by $C_1$-$C_4$-alkyl or halogen,
phenyl which may carry from one to four of the following groups: $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, halogen, nitro, cyano, formyl, phenyl, $C_1$-$C_4$-alkanoyl, $C_1$-$C_4$-haloalkanoyl or $C_1$-$C_4$-alkoxycarbonyl,
naphthyl which may be monosubstituted to trisubstituted by $C_1$-$C_4$-alkyl or halogen,
and plant-tolerated salts of the dicarboximides I, except for 3-methylisoxazole-4,5-dicarboximide and thiazole-4,5-dicarboximides in which $R^2$ is phenyl when $R^1$ is methyl or 2-thiazolyl.

2. A process for the preparation of a compound of the formula I, wherein the corresponding dicarboxylic acid monoamide II

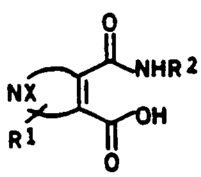

II

is treated with a water-eliminating agent in a conventional manner.

3. A process for the preparation of a compound Ia, wherein a hydroxamyl chloride of the formula X

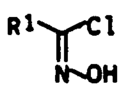

X,

is reacted with a halomaleimide of the formula XI

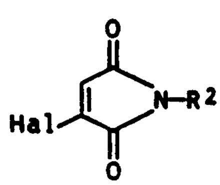

XI,

where $R^1$ and $R^2$ have the abovementioned meanings and Hal is chlorine or bromine, in the presence of

a base.

**4.** A process for the preparation of a compound Ia, wherein a hydroxamyl chloride X as claimed in claim 3 is converted in a conventional manner with a maleimide XIII

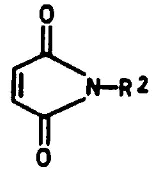

XIII

into an isoxasolinimide XII

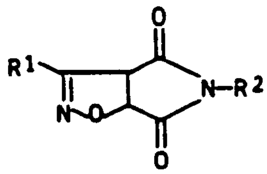

XII

and the latter is then dehydrogenated using an oxidizing agent.

**5.** A herbicide containing, in addition to inert additives, one or more dicarboximides of the formula Ia, Ib or Ic

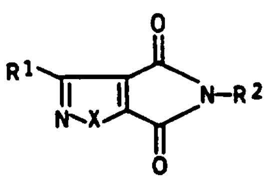

Ia

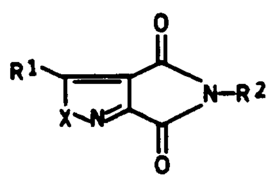

Ib

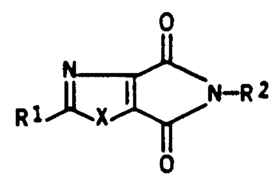

Ic

where the substituents have the meanings stated in claim 1, including 3-methylisoxazole-4,5-dicarboximide and thiazole-4,5-dicarboximides in which $R^2$ is phenyl when $R^1$ is methyl or 2-thiazolyl.

**6.** A method for controlling undesirable plant growth, wherein the undesirable plants and/or the area to be kept free of undesirable plant growth are or is treated with a herbicidal amount of a dicarboximide of the formula Ia, Ib or Ic as claimed in claim 5.

**Claims for the following Contracting State : ES**

**1.** A process for the preparation of a dicarboximide of the formula Ia, Ib or Ic

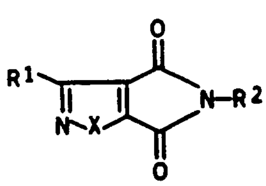

Ia

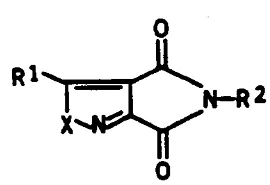

Ib

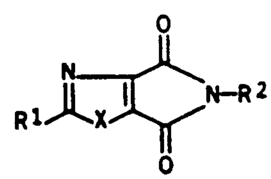

Ic

where
X is oxygen or sulfur,
$R^1$ is hydrogen, halogen, cyano,

$C_1$-$C_6$-alkyl which may carry from one to five halogen atoms and/or one or two of the following radicals: $C_3$-$C_6$-cycloalkyl, $C_1$-$C_4$-alkoxy, $C_1$-$C_4$-haloalkoxy, $C_1$-$C_4$-alkylthio, $C_1$-$C_4$-haloalkylthio, $C_1$-$C_4$-alkylsulfonyl, $C_1$-$C_4$-haloalkylsulfonyl or cyano,
$C_3$-$C_8$-cycloalkyl which may carry from one to three of the following radicals: $C_1$-$C_4$-alkyl or halogen,
$C_2$-$C_6$-alkenyl which may carry from one to three of the following radicals: halogen, $C_1$-$C_3$-alkoxy and/or a phenyl radical which in turn may carry from one to three of the following groups: $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, halogen, cyano or nitro,
$C_2$-$C_6$-alkynyl which may carry from one to three of the following radicals: halogen, $C_1$-$C_3$-alkoxy and/or a phenyl radical which in turn may carry from one to three of the following groups: $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, halogen, cyano or nitro,
$C_1$-$C_4$-alkoxy, $C_2$-$C_6$-alkenyloxy, $C_2$-$C_6$-alkynyloxy, $C_1$-$C_4$-alkylthio, $C_1$-$C_4$-haloalkoxy, $C_1$-$C_4$-haloalkylthio, $C_1$-$C_4$-alkylsulfonyl or $C_1$-$C_4$-haloalkylsulfonyl,
phenoxy or phenylthio which may carry from one to three of the following radicals: $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, halogen, cyano or nitro,
a 5-membered or 6-membered saturated or aromatic heterocyclic radical containing one or two hetero atoms selected from the group consisting of oxygen, sulfur and nitrogen, where the ring may carry one or two of the following radicals: $C_1$-$C_3$-alkyl, halogen, $C_1$-$C_3$-alkoxy or $C_1$-$C_3$-alkoxycarbonyl,
a fused aromatic radical selected from the group consisting of benzofuran-2-yl, benzofuran-3-yl, isobenzofuran-2-yl, benzothiophen-2-yl, benzothiophen-3-yl, isobenzothiophen-2-yl, indol-2-yl, indol-3-yl, 1,2-benzoisoxal-3-yl, benzoxazol-2-yl, 1,2-benzoisothiazol-3-yl, benzothiazol-2-yl, indazol-3-yl, (1H)-benzimidazol-2-yl, quinol-3-yl, quinol-5-yl, quinol-6-yl, quinol-8-yl, isoquinol-1-yl and isoquinol-5-yl, where this radical may carry one or two of the following radicals: alkyl as stated above, in particular methyl, halogen as stated above, in particular fluorine or chlorine, alkoxy as stated above, in particular methoxy or ethoxy, or alkoxycarbonyl, such as methoxycarbonyl or ethoxycarbonyl, in particular methoxycarbonyl, or
phenyl or phenyl-$C_1$-$C_4$-alkyl where the phenyl nucleus in each case is unsubstituted or substituted by from one to three of the following groups: $C_1$-$C_6$-alkyl, $C_1$-$C_6$-haloalkyl, $C_1$-$C_6$-alkoxy, $C_1$-$C_6$-haloalkoxy, $C_1$-$C_6$-alkylthio, $C_1$-$C_6$-haloalkylthio, halogen, nitro or cyano,
$R^2$ is hydrogen, hydroxyl, $C_1$-$C_4$-alkoxy, $C_1$-$C_6$-alkyl which may carry from one to three of the following groups: cyano, $C_1$-$C_4$-alkoxy-$C_1$-$C_4$-alkoxy, $C_1$-$C_4$-alkoxy, $C_1$-$C_4$-haloalkoxy, $C_1$-$C_4$-alkylthio, $C_1$-$C_4$-haloalkylthio, di-$C_1$-$C_4$-alkylamino, halogen, $C_3$-$C_8$-cycloalkyl or phenyl, where the phenyl ring in turn may carry from one to three of the following radicals: halogen, cyano, nitro, $C_1$-$C_4$-alkyl,$C_1$-$C_4$-haloalkyl, $C_1$-$C_4$-alkoxy, $C_1$-$C_4$-haloalkoxy, $C_1$-$C_4$-alkylthio or $C_1$-$C_4$-haloalkylthio,
$C_3$-$C_8$-cycloalkyl which may carry from one to three of the following groups: $C_1$-$C_6$-alkyl, $C_1$-$C_6$-haloalkyl, $C_1$-$C_4$-alkoxy, $C_1$-$C_4$-haloalkoxy, halogen, nitro or cyano,
$C_3$-$C_6$-alkenyl which may be monosubstituted to trisubstituted by halogen and/or monosubstituted by phenyl, where the phenyl radical in turn may carry from one to three of the following groups: $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, halogen, cyano or nitro,
$C_3$-$C_6$-alkynyl which may be monosubstituted to trisubstituted by halogen and/or monosubstituted by phenyl, where the phenyl radical in turn may carry from one to three of the following groups: $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, halogen, cyano or nitro,
di-$C_1$-$C_4$-alkylamino,
a 5-membered or 6-membered heterocyclic saturated or aromatic radical which has one or two hetero atoms selected from the group consisting of oxygen, sulfur and nitrogen and which may be monosubstituted to trisubstituted by $C_1$-$C_4$-alkyl or halogen,
a fused aromatic radical selected from the group consisting of benzofuran-2-yl, benzofuran-3-yl, isobenzofuran-2-yl, benzothiophen-2-yl, benzothiophen-3-yl, isobenzothiophen-2-yl, indol-2-yl, indol-3-yl, 1,2-benzoisoxal-3-yl, benzoxazol-2-yl, 1,2-benzoisothiazol-3-yl, benzothiazol-2-yl, indazol-3-yl, (1H)-benzimidazol-2-yl, quinol-3-yl, quinol-5-yl, quinol-6-yl, quinol-8-yl, isoquinol-1-yl and isoquinol-5-yl, where this radical may be monosubstituted to trisubstituted by $C_1$-$C_4$-alkyl or halogen,
phenyl which may carry from one to four of the following groups: $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, halogen, nitro, cyano, formyl, phenyl, $C_1$-$C_4$-alkanoyl, $C_1$-$C_4$-haloalkanoyl or $C_1$-$C_4$-alkoxycarbonyl,
naphthyl which may be monosubstituted to trisubstituted by $C_1$-$C_4$-alkyl or halogen,
except for 3-methylisoxazole-4,5-dicarboximide and thiazole-4,5-dicarboximides in which $R^2$ is phenyl when $R^1$ is methyl or 2-thiazolyl, wherein the corresponding dicarboxylic acid monoamide II

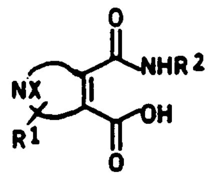

II

is treated with a water-eliminating agent in a conventional manner.

2. A process for the preparation of a compound Ia, wherein a hydroxamyl chloride of the formula X

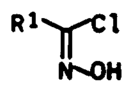

X,

is reacted with a halomaleimide of the formula XI

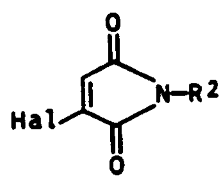

XI,

where $R^1$ and $R^2$ have the abovementioned meanings and Hal is chlorine or bromine, in the presence of a base.

3. A process for the preparation of a compound Ia, wherein a hydroxamyl chloride X as claimed in claim 2 is converted in a conventional manner with a maleimide XIII into an isoxazolinimide XII

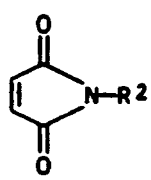

XIII

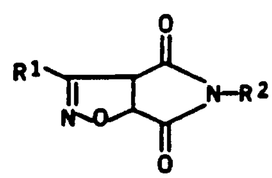

XII

and the latter is then dehydrogenated using an oxidizing agent.

4. A herbicide containing, in addition to inert additives, one or more dicarboximides of the formula Ia, Ib or Ic

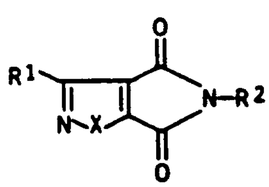

Ia

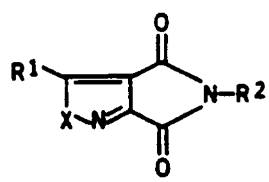

Ib

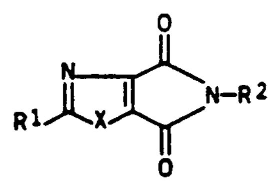

Ic

where the substituents have the meanings stated in claim 1, including 3-methylisoxazole-4,5-dicarboximide and thiazole-4,5-dicarboximides in which $R^2$ is phenyl when $R^1$ is methyl or 2-thiazolyl.

5. A method for controlling undesirable plant growth, wherein the undesirable plants and/or the area to be kept free of undesirable plant growth are or is treated with a herbicidal amount of a dicarboximide of the formula Ia, Ib or Ic as claimed in claim 4.

**Revendications**
**Revendications pour les Etats contractants suivants : AT, BE, CH, DE, FR, GB, IT, LI, NL**

1. Dicarboximides de formules Ia, Ib et Ic

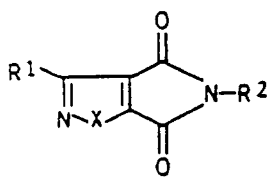
Ia

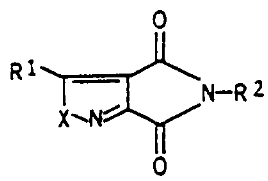
Ib

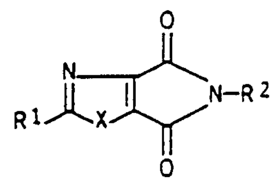
Ic

dans lesquelles les symboles ont les significations suivantes :
X représente
l'oxygène ou le soufre ;
$R^1$ représente
l'hydrogène ; un halogène ; un groupe cyano ;
un groupe alkyle en C1-C6 qui peut porter un à cinq atomes d'halogènes et/ou un ou deux des substituants suivants : cycloalkyle en C3-C6, alcoxy en C1-C4, halogénoalcoxy en C1-C4, alkylthio en C1-C4, halogénoalkylthio en C1-C4, alkylsulfonyle en C1-C4, halogénoalkylsulfonyle en C1-C4 ou cyano ;
un groupe cycloalkyle en C3-C8 qui peut porter un à trois des substituants suivants : alkyle en C1-C4 ou halogènes ;
un groupe alcényle en C2-C6 qui peut porter un à trois des substituants suivants : les halogènes, les groupes alcoxy en C1-C3 et/ou un groupe phényle qui peut lui-même porter un à trois des substituants suivants : alkyle en C1-C4, halogénoalkyle en C1-C4, alcoxy en C1-C4, halogénoalcoxy en C1-C4, alkylthio en C1-C4, halogénoalkylthio en C1-C4, les halogènes, les groupes cyano ou nitro ;
un groupe alcynyle en C2-C6 qui peut porter un à trois des substituants suivants : les halogènes ou les groupes alcoxy en C1-C3 et/ou un groupe phényle qui peut lui-même porter un à trois des substituants suivants : alkyle en C1-C4, halogénoalkyle en C1-C4, alcoxy en C1-C4, halogénoalcoxy en C1-C4, alkylthio en C1-C4, halogénoalkylthio en C1-C4, les halogènes, les groupes cyano ou nitro ;
un groupe alcoxy en C1-C4 ; alcényloxy en C2-C6, alcynyloxy en C2-C6, alkylthio en C1-C4, halogénoalcoxy en C1-C4, halogénoalkylthio en C1-C4 ; alkylsulfonyle en C1-C4 ; halogénoalkylsulfonyle en C1-C4 ;
un groupe phénoxy ou phénylthio qui peut porter un à trois des substituants suivants : alkyle en C1-C4, halogénoalkyle en C1-C4, alcoxy en C1-C4, halogénoalcoxy en C1-C4, alkylthio en C1-C4, halogénoalkylthio en C1-C4, les halogènes, les groupes cyano ou nitro ;
un radical hétérocyclique saturé ou aromatique de 5 à 6 chaînons contenant un ou deux hétéroatomes choisis parmi l'oxygène, le soufre et l'azote, le cycle pouvant porter un ou deux des substituants suivants : alkyle en C1-C3, halogènes, alcoxy en C1-C3 ou (alcoxy en C1-C3)-carbonyle ;
un radical aromatique condensé choisi parmi les radicaux benzofuranne-2-yle, benzofuranne-3-yle, isobenzofuranne-2-yle, benzothiophène-2-yle, benzothiophène-3-yle, isobenzothiophène-2-yle, indole-2-yle, indole-3-yle, 1,2-benzoisoxal-3-yle, benzoxazole-2-yle, 1,2-benzoisothiazole-3-yle, benzothiazole-2-yle, indazole-3-yle, (1H)-benzimidazole-2-yle, quinoléine-3-yle, quinoléine-5-yle, quinoléine-6-yle, quinoléine-8-yle, isoquinoléine-1-yle et isoquinoléine-5-yle, ce radical pouvant porter porter un ou deux des substituants suivants : alkyle tel que mentionné ci-dessus, en particulier méthyle ; halogènes, tels que mentionnés ci-dessus, en particulier fluor et chlore ; alcoxy tel que mentionné ci-dessus, en particulier méthoxy et éthoxy, ou alcoxycarbonyle tel que méthoxycarbonyle et éthoxycarbonyle, plus spécialement méthoxycarbonyle ;
un groupe phényle ou phényl-alkyle en C1-C4 dont le noyau phényle est non substitué ou porte un à trois des substituants suivants : alkyle en C1-C6, halogénoalkyle en C1-C6, alcoxy en C1-C6,

halogénoalcoxy en C1-C6, alkylthio en C1-C6, halogénoalkylthio en C1-C6, halogènes, nitro et cyano ;
$R^2$ représente
l'hydrogène, un groupe hydroxy ; alcoxy en C1-C4 ;
un groupe alkyle en C1-C6 qui peut porter un à trois des substituants suivants : cyano, (alcoxy en C1-C4)-alcoxy en C1-C4, alcoxy en C1-C4, halogénoalcoxy en C1-C4, alkylthio en C1-C4, halogénoalkylthio en C1-C4, di-(alkyle en C1-C4)-amino, halogènes, cycloalkyle en C3-C8 ou phényle dont le noyau phényle peut lui-même porter un à trois des substituants suivants : halogènes, cyano, nitro, alkyle en C1-C4, halogénoalkyle en C1-C4, alcoxy en C1-C4, halogénoalcoxy en C1-C4, alkylthio en C1-C4 ou halogénoalkylthio en C1-C4 ;
un groupe cycloalkyle en C3-C8 qui peut porter un à trois des substituants suivants : alkyle en C1-C6, halogénoalkyle en C1-C6, alcoxy en C1-C4, halogénoalcoxy en C1-C4, halogènes, nitro ou cyano ;
un groupe alcényle en C3-C6 qui peut porter un à trois substituants halogéno et/ou un substituant phényle qui peut lui-même porter un à trois des substituants suivants : alkyle en C1-C4, halogénoalkyle en C1-C4, alcoxy en C1-C4, halogénoalcoxy en C1-C4, alkylthio en C1-C4, halogénoalkylthio en C1-C4, halogènes, cyano ou nitro ;
un groupe alcényle en C3-C6 qui peut porter un à trois substituants halogéno et/ou un substituant phényle qui peut lui-même porter un à trois des substituants suivants : alkyle en C1-C4, halogénoalkyle en C1-C4, alcoxy en C1-C4, halogénoalcoxy en C1-C4, alkylthio en C1-C4, halogénoalkylthio en C1-C4, halogènes, cyano ou nitro ;
un groupe di-(alkyle en C1-C4)-amino ;
un radical hétérocyclique saturé ou aromatique de 5 à 6 chaînons contenant un ou deux hétéroatomes choisis parmi l'oxygène, le soufre et l'azote et qui peut porter un à trois substituants alkyle en C1-C4 ou halogéno ;
un radical aromatique condensé choisi parmi les radicaux benzofuranne-2-yle, benzofuranne-3-yle, isobenzofuranne-2-yle, benzothiophène-2-yle, benzothiophène-3-yle, isobenzothiophène-2-yle, indole-2-yle, indole-3-yle, 1,2-benzoisoxal-3-yle, benzoxazole-2-yle, 1,2-benzoisothiazole-3-yle, benzothiazole-2-yle, indazole-3-yle, (1H)-benzimidazole-2-yle, quinoléine-3-yle, quinoléine-5-yle, quinoléine-6-yle, quinoléine-8-yle, isoquinoléine-1-yle et isoquinoléine-5-yle, ce radical pouvant porter un à trois substituants alkyle en C1-C4 ou halogéno ;
un groupe phényle qui peut porter un à quatre des substituants suivants : alkyl en C1-C4, halogénoalkyle en C1-C4, alcoxy en C1-C4, halogénoalcoxy en C1-C4, alkylthio en C1-C4, halogénoalkylthio en C1-C4, halogéno, nitro, cyano, formyle, phényle, alcanoyle en C1-C4, halogénoalcanoyle ou (alcoxy en C1-C4)-carbonyle ;
un groupe naphtyle qui peut porter un à trois substituants alkyle en C1-C4 ou halogéno ;
et les sels dicarboximides I tolérés par les végétaux, à l'exception du 3-méthyl-isoxazole-4,5-dicarboximide et des thiazole-4,5-dicarboximides pour lesquels $R^2$ représente un groupe phényle lorsque $R^1$ représente un groupe méthyle ou 2-thiazolyle.

2. Procédé de préparation des composés de formule I, caractérisé en ce que l'on traite les monoamides d'acides dicarboxyliques correspondants II

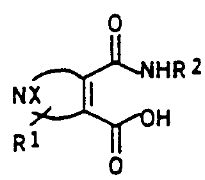

II

de manière connue en soi, par des agents déshydratants.

3. Procédé de préparation des composés Ia, caractérisé en ce que l'on fait réagir un chlorure d'acide hydroxamique de formule X

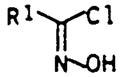

X,

en présence d'une base, avec des halogénomaléimides de formule XI

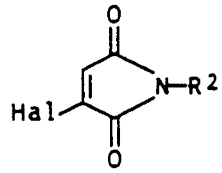

XI,

dans laquelle $R^1$ et $R^2$ ont les significations indiquées ci-dessus et Hal représente le chlore ou le brome.

**4.** Procédé de préparation des composés Ia, caractérisé en ce que l'on fait réagir un chlorure d'acide hydroxamique X selon la revendication 3, de manière connue en soi, avec des maléimides XIII

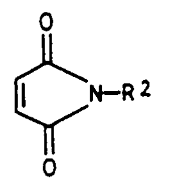

XIII

ce qui donne les isoxazolinimides XII

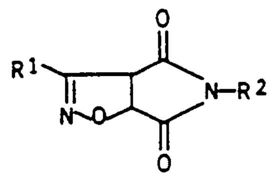

XII

qu'on déshydrogène ensuite à l'aide d'agents oxydants.

**5.** Produit herbicide contenant, avec des additifs inertes, au moins un dicarboximide de formule Ia, Ib ou Ic

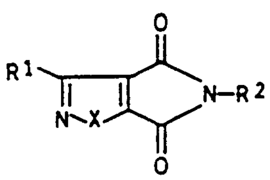

Ia

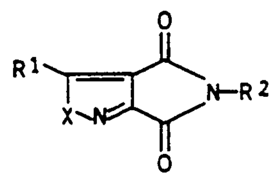

Ib

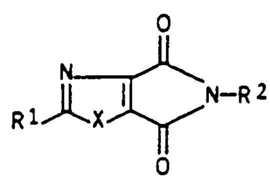

Ic

dans lesquelles les symboles ont les significations indiquées dans la revendication 1, y compris le 3-méthylisoxazole-4,5-dicarboximide et les thiazole-4,5-dicarboximides pour lesquels $R^2$ représente un groupe phényle lorsque $R^1$ représente un groupe méthyle ou 2-thiazolyle.

**6.** Procédé pour combattre les croissances de végétaux indésirables, caractérisé en ce que l'on traite les végétaux indésirables et/ou l'aire à protéger contre les croissances de végétaux indésirables par une quantité herbicide efficace d'un dicarboximide de formule Ia, Ib ou Ic selon la revendication 5.

**Revendications pour l'Etat contractant suivant : ES**

**1.** Procédé de préparation des dicarboximides de formules Ia, Ib et Ic

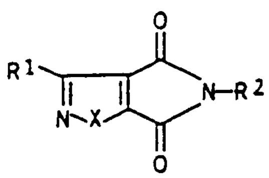

Ia

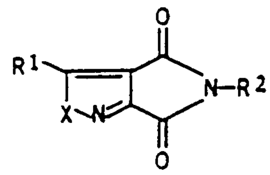

Ib

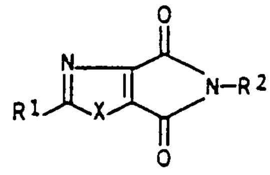

Ic

dans lesquelles les symboles ont les significations suivantes :
X représente
l'oxygène ou le soufre ;
$R^1$ représente
l'hydrogène ; un halogène ; un groupe cyano ;
un groupe alkyle en C1-C6 qui peut porter un à cinq atomes d'halogènes et/ou un ou deux des substituants suivants : cycloalkyle en C3-C6, alcoxy en C1-C4, halogénoalcoxy en C1-C4, alkylthio en C1-C4, halogénoalkylthio en C1-C4, alkylsulfonyle en C1-C4, halogénoalkylsulfonyle en C1-C4 ou cyano ;
un groupe cycloalkyle en C3-C8 qui peut porter un à trois des substituants suivants :
alkyle en C1-C4 ou halogènes ;
un groupe alcényle en C2-C6 qui peut porter un à trois des substituants suivants : les halogènes, les groupes alcoxy en C1-C3 et/ou un groupe phényle qui peut lui-même porter un à trois des substituants suivants : alkyle en C1-C4, halogénoalkyle en C1-C4, alcoxy en C1-C4, halogénoalcoxy en C1-C4, alkylthio en C1-C4, halogénoalkylthio en C1-C4, les halogènes, les groupes cyano ou nitro ;
un groupe alcynyle en C2-C6 qui peut porter un à trois des substituants suivants : les halogènes ou les groupes alcoxy en C1-C3 et/ou un groupe phényle qui peut lui-même porter un à trois des substituants suivants : alkyle en C1-C4, halogénoalkyle en C1-C4, alcoxy en C1-C4, halogénoalcoxy en C1-C4, alkylthio en C1-C4, halogénoalkylthio en C1-C4, les halogènes, les groupes cyano ou nitro ;
un groupe alcoxy en C1-C4 ; alcényloxy en C2-C6, alcynyloxy en C2-C6, alkylthio en C1-C4, halogénoalcoxy en C1-C4, halogénoalkylthio en C1-C4 ; alkylsulfonyle en C1-C4 ; halogénoalkylsulfonyle en C1-C4 ;
un groupe phénoxy ou phénylthio qui peut porter un à trois des substituants suivants : alkyle en C1-C4, halogénoalkyle en C1-C4, alcoxy en C1-C4, halogénoalcoxy en C1-C4, alkylthio en C1-C4, halogénoalkylthio en C1-C4, les halogènes, les groupes cyano ou nitro ;
un radical hétérocyclique saturé ou aromatique de 5 à 6 chaînons contenant un ou deux hétéroatomes choisis parmi l'oxygène, le soufre et l'azote, le cycle pouvant porter un ou deux des substituants suivants : alkyle en C1-C3, halogènes, alcoxy en C1-C3 ou (alcoxy en C1-C3)-carbonyle ;
un radical aromatique condensé choisi parmi les radicaux benzofuranne-2-yle, benzofuranne-3-yle, isobenzofuranne-2-yle, benzothiophène-2-yle, benzothiophène-3-yle, isobenzothiophène-2-yle, indole-2-yle, indole-3-yle, 1,2-benzoisoxal-3-yle, benzoxazole-2-yle, 1,2-benzoisothiazole-3-yle, benzothiazole-2-yle, indazole-3-yle, (1H)-benzimidazole-2-yle, quinoléine-3-yle, quinoléine-5-yle, quinoléine-6-yle, quinoléine-8-yle, isoquinoléine-1-yle et isoquinoléine-5-yle, ce radical pouvant porter porter un ou deux des substituants suivants : alkyle tel que mentionné ci-dessus, en particulier méthyle ; halogènes, tels que mentionnés ci-dessus, en particulier fluor et chlore ; alcoxy tel que mentionné ci-dessus, en particulier méthoxy et éthoxy, ou alcoxycarbonyle tel que méthoxycarbonyle et éthoxycarbonyle, plus spécialement méthoxycarbonyle ;
un groupe phényle ou phényl-alkyle en C1-C4 dont le noyau phényle est non substitué ou porte un à trois des substituants suivants : alkyle en C1-C6, halogénoalkyle en C1-C6, alcoxy en C1-C6, halogénoalcoxy en C1-C6, alkylthio en C1-C6, halogénoalkylthio en C1-C6, halogènes, nitro et cyano ;
$R^2$ représente
l'hydrogène, un groupe hydroxy ; alcoxy en C1-C4 ;
un groupe alkyle en C1-C6 qui peut porter un à trois des substituants suivants : cyano, (alcoxy en C1-C4)-alcoxy en C1-C4, alcoxy en C1-C4, halogénoalcoxy en C1-C4, alkylthio en C1-C4, halogénoalkylthio en C1-C4, di-(alkyle en C1-C4)-amino, halogènes, cycloalkyle en C3-C8 ou phényle dont le noyau phényle peut lui-même porter un à trois des substituants suivants : halogènes, cyano, nitro, alkyle en C1-C4, halogénoalkyle en C1-C4, alcoxy en C1-C4, halogénoalcoxy en C1-C4, alkylthio en C1-C4 ou halogénoalkylthio en C1-C4 ;
un groupe cycloalkyle en C3-C8 qui peut porter un à trois des substituants suivants : alkyle en C1-C6, halogénoalkyle en C1-C6, alcoxy en C1-C4, halogénoalcoxy en C1-C4, halogènes, nitro ou cyano ;
un groupe alcényle en C3-C6 qui peut porter un à trois substituants halogéno et/ou un substituant phényle qui peut lui-même porter un à trois des substituants suivants : alkyle en C1-C4, halogénoalkyle en C1-C4, alcoxy en C1-C4, halogénoalcoxy en C1-C4, alkylthio en C1-C4, halogénoalkylthio en C1-C4, halogènes, cyano ou nitro ;
un groupe alcényle en C3-C6 qui peut porter un à trois substituants halogéno et/ou un substituant phényle qui peut lui-même porter un à trois des substituants suivants : alkyle en C1-C4, halogénoalkyle en C1-C4, alcoxy en C1-C4, halogénoalcoxy en C1-C4, alkylthio en C1-C4, halogénoalkylthio en C1-C4, halogènes, cyano ou nitro ;
un groupe di-(alkyle en C1-C4)-amino ;
un radical hétérocyclique saturé ou aromatique de 5 à 6 chaînons contenant un ou deux hétéroatomes choisis parmi l'oxygène, le soufre et l'azote et qui peut porter un à trois substituants alkyle en C1-C4 ou halogéno ;
un radical aromatique condensé choisi parmi les radicaux benzofuranne-2-yle, benzofuranne-3-yle, isobenzofuranne-2-yle, benzothiophène-2-yle, benzothiophène-3-yle, isobenzothiophène-2-yle, indole-2-yle, indole-3-yle, 1,2-benzoisoxal-3-yle, benzoxazole-2-yle, 1,2-benzoisothiazole-3-yle, benzothiazole-2-yle, indazole-3-yle, (1H)-benzimidazole-2-yle, quinoléine-3-yle, quinoléine-5-yle, quinoléine-6-yle, quinoléine-8-yle, isoquinoléine-1-yle et isoquinoléine-5-yle, ce radical pouvant porter un à trois substituants alkyle en C1-C4 ou halogéno ;
un groupe phényle qui peut porter un à quatre des substituants suivants : alkyl en C1-C4, halogénoalkyle en C1-C4, alcoxy en C1-C4, halogénoalcoxy en C1-C4, alkylthio en C1-C4, halogénoalkylthio en C1-C4, halogéno, nitro, cyano, formyle, phényle, alcanoyle en C1-C4, halogénoalcanoyle ou (alcoxy en C1-C4)-carbonyle ;
un groupe naphtyle qui peut porter un à trois substituants alkyle en C1-C4 ou halogéno, à l'exception du 3-méthylisoxazole-4,5-dicarboximide et des thiazole-4,5-dicarboximides pour lesquels $R^2$ représente un groupe phényle, lorsque $R^1$ représente un groupe méthyle ou 2-thiazolyle, caractérisé en ce que l'on traite les monoamides d'acides dicarboxyliques correspondants II

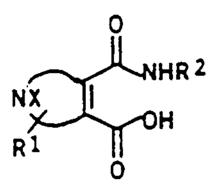

II

de manière connue en soi, par des agents déshydratants.

**2.** Procédé de préparation des composés Ia, caractérisé en ce que l'on fait réagir un chlorure d'acide hydroxamique de formule X

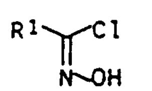

X,

en présence d'une base, avec des halogénomaléimides de formule XI

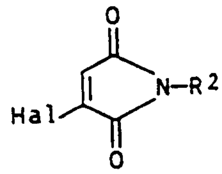 XI,

dans laquelle $R^1$ et $R^2$ ont les significations indiquées ci-dessus et Hal représente le chlore ou le brome.

**3.** Procédé de préparation des composés Ia, caractérisé en ce que l'on fait réagir un chlorure d'acide hydroxamique X selon la revendication 3, de manière connue en soi, avec des maléimides XIII

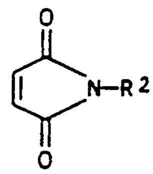 XIII

ce qui donne les isoxazolinimides XII

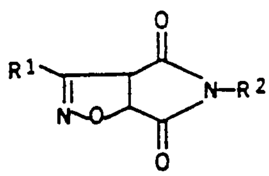 XII

qu'on déshydrogène ensuite à l'aide d'agents oxydants.

**4.** Produit herbicide contenant, avec des additifs inertes, au moins un dicarboximide de formule Ia, Ib ou Ic

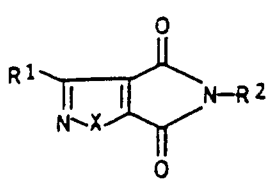
Ia

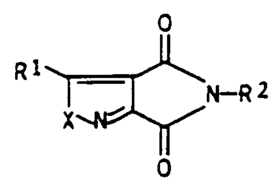
Ib

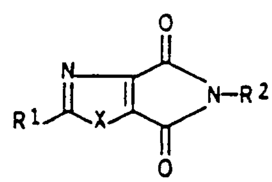
Ic

dans lesquelles les symboles ont les significations indiquées dans la revendication 1, y compris le 3-méthylisoxazole-4,5-dicarboximide et les thiazole-4,5-dicarboximides pour lesquels $R^2$ représente un groupe phényle lorsque $R^1$ représente un groupe méthyle ou 2-thiazolyle.

**5.** Procédé pour combattre les croissances de végétaux indésirables, caractérisé en ce que l'on traite les végétaux indésirables et/ou l'aire à protéger contre les croissances de végétaux indésirables par une quantité herbicide efficace d'un dicarboximide de formule Ia, Ib ou Ic selon la revendication 4.